# EUROPEAN PATENT APPLICATION

(11) **EP 4 714 980 A1**
(43) Date of publication of application: **25.03.2026**
(21) Application number: 24810337.6
(22) Date of filing: 17.05.2024
(51) Int. Cl.: C07K 19/00, C07K 14/47, C07K 14/705, A61K 38/17, A61P 19/02, A61P 13/12, C12N 15/861

(54) **AAV VECTOR FOR TREATING ALTERNATIVE COMPLEMENT PATHWAY-RELATED DISEASES**

(30) Priority: 19.05.2023 CN 202310588495
(71) Applicant: Starrygene Therapeutics Co., Ltd., Hefei, Anhui 230088 (CN)
(72) Inventor: ZHANG, Jie, Hefei, Anhui 230088 (CN); CAI, Yuan, Hefei, Anhui 230088 (CN); ZHU, Ying, Hefei, Anhui 230088 (CN); MA, Zhen, Hefei, Anhui 230088 (CN); ZHANG, Huiyun, Hefei, Anhui 230088 (CN); ZHOU, Qianqian, Hefei, Anhui 230088 (CN)
(74) Representative: J A Kemp LLP
(86) International application number: PCT/CN2024/094059
(87) International publication number: WO 2024/240095

(57) **Abstract**

The present disclosure provides a CR2-FH fusion protein, a polynucleotide encoding it, a vector, a pharmaceutical composition and use thereof, which can be used to treat an alternative complement pathway-related disease such as dry AMD and the like.

## Description

### PRIORITY

The present application claims the benefit of and priority to Chinese application No. 2023105884953, filed on May 19, 2023, the entire content of which is incorporated herein by reference in its entirety for all purposes.

### TECHNICAL FIELD

The present application relates to a fusion protein or construct for treating alternative complement pathway-related diseases, and uses thereof. Specifically, the present application relates to CR2-FH molecules for treating alternative complement pathway-related diseases, constructs encoding the CR2-FH molecules, and uses thereof.

### BACKGROUND

The complement system is an important host defense system that plays a significant role in immune regulatory mechanisms such as regulating humoral immunity and cellular immunity, catabolizing immune complexes, and clearing apoptotic cells. The complement system consists of a variety of soluble protein molecules, whose components include complement intrinsic components, various regulatory factors, complement receptors, and other molecules. Complement cascade reaction has three activation pathways: classical pathway/alternative pathway/lectin pathway, three pathways converge downstream at C3, and finally activate through C5 to produce membrane attack complex (MAC), play a cytolytic effect, participate in immunity, and is an important natural immune barrier for human body. However, inappropriate complement activation and its deposition on host cells can lead to complement mediated lysis of target cells, as well as tissue destruction due to the production of powerful inflammatory mediators. Abnormal complement activation is associated with the pathogenesis of age-related macular degeneration (AMD). Whole-genome studies have shown that genetic variations of various components in the complement cascade are associated with an increased risk of AMD, and C3, C5, anaphylatoxins C3a and C5a, as well as other acute phase reactant proteins, have been demonstrated to be present in patients' ocular drusen deposits. In AMD patients, levels of C3a, C3d, Bb, and C5a in plasma are relatively high, indicating that complement activation plays a role in the pathogenesis of AMD. In addition, alternative complement pathway-related diseases also include rheumatoid arthritis, C3 glomerulonephritis, membrane proliferative glomerulonephritis II (MPGN II), Factor H-related hemolytic uremic syndrome (HUS), paroxysmal nocturnal hemoglobinuria (PNH), systemic lupus erythematosus (SLE), lupus nephritis, stroke, myocardial infarction, acute respiratory distress syndrome (ARDS), sepsis, burns, inflammation associated with cardiopulmonary bypass and hemodialysis, plasmapheresis, platelet isolation, leukapheresis, extracorporeal membrane oxygenation (ECMO), heparin-induced extracorporeal LDL precipitation (HELP), and radiological contrast induced allergic reactions.

AMD is a debilitating and blinding disease that affects the macular or central area of the retina, and is the leading cause of irreversible visual loss in the elderly. In developed countries, AMD is a blinding disease with high incidence among people over 65 years old, affecting about 9% of the population worldwide. AMD is mainly divided into two types: dry and wet, wherein dry AMD accounts for more than 80%, which could develop into geographic atrophy (GA) in the advanced stage. The typical features of dry AMD are the formation of drusen associated with degenerative changes in retinal pigmented epithelium (RPE) cells, the presence of a visible pigmented area in the center of the macula, and the loss of photoreceptor cells-rod and cone cells. In the advanced stage of dry AMD, large area atrophy of RPE, atrophy of choroidal blood vessels, resulting in permanent central vision loss.

The factor H (FH) is a single-stranded glycoprotein composed of 1231 amino acids, with a molecular weight of 155 kDa, consisting of 20 short consensus repeats (SCR), which is a key inhibitor of the alternative pathway. Functional defects or insufficient level of FH may promote complement activation, thereby increasing the risk of local tissue damage. Under normal physiological conditions, C3 interacts with factor B (FB), factor D (FD), etc., producing very small amounts of C3b and C3bBb (C3 convertase), C3 convertase is rapidly affected by FH, and does not further reactivate C3 or subsequent complement components. In pathological conditions, FH has insufficient control of C3 convertase, C3 convertase hydrolyzes C3 protein into C3b molecule, and meanwhile generates anaphylatoxin C3a, and then complement cascade proceeds to its terminal cleavage pathway, which will generate anaphylatoxin C5a and MAC. Both of C5a and MAC could lead to strong inflammatory signals. Studies have proven that in dry AMD, due to dysfunction of RPE cells, the dynamic balance of retina is damaged, the decrease of FH leads to accumulation of C3 on RNA and protein levels, and RPE cells are more prone to damage due to oxidative stress. Endogenous FH helps regulate transcription and metabolic homeostasis, and protects RPE cells from oxidative stress damage. In addition, FH has single nucleotide polymorphism (SNP), which is the first complement SNPs found to be associated with dry AMD. Since complement plays an important role in host defense and catabolism of immune complexes, targeting complement inhibitors FH to complement activation and disease sites may improve its efficacy while reducing side effects from complement inhibition. The complement receptor 2 (CR2) is a complement receptor, a member of the C3 binding protein family, consisting of 15 or 16 SCR domains, the natural ligands of CR2 are iC3b, C3dg and C3d, which are decomposed fragments of C3. The cleavage of C3 initially results in the production and deposition of C3b on the activated cell surface, and C3b fragments participate in the production of enzyme complexes that amplify the complement cascade reaction. At the cell surface, C3b rapidly converts to inactive iC3b, particularly when deposited on a host surface containing a complement activation modulator. Even without membrane-bound complement regulatory factors, a significant level of iC3b can be formed due to the function of FH. Subsequently, iC3b is digested by factor I (FI) and other proteases into membrane binding fragments C3dg and C3d, but this process is relatively slow. Thus, the C3 ligand of CR2 is relatively long-lived once produced and is present at a high concentration in the complement activation site. CR2 can thus act as an effective targeting carrier that brings molecules to complement activation sites. Although wet AMD antibody drugs and gene therapy products have made good progress in the treatment of AMD, especially when VEGF inhibitors are used, gene therapy drugs for dry AMD have not been approved in China. Therefore, there is an urgent need for new therapeutic drugs to solve this problem. Based on the advantage of continuous action of gene therapy products, it is desirable to develop efficient therapeutic drugs for dry AMD.

Adeno-associated virus (AAV) was first discovered from laboratory adenovirus (AdV) preparations in the mid-1960s, and was soon found in human tissues afterwards. Due to its characteristics of good safety, wide range of host cells, low immunogenicity, efficient and long-term expression of exogenous genes, it becomes an important tool for gene delivery. Currently, the leading AAV genome design is capable of designing single-stranded DNA carried in the capsid into a self-complementary sequence. The advantage of this sequence is that it can be transcribed without the step of replicating single-stranded DNA into doublestranded DNA, and compared with the traditional single-stranded AAV genome, its gene expression is faster and the expression level is higher. To date, six gene therapy drugs using recombinant AAV as vectors have been approved for marketing worldwide. In addition to Novartis' Zolgensma (AAV9 type), there are also Glybera (AAV1 type) developed by UniQure and Luxturna (AAV2 type) developed by Spark Therapeutics, among others. Therefore, it can be seen that AAV gene therapy has great potential, and gene therapy has become the core technology to solve the dilemma of sustained function of drugs and restrained time

### SUMMARY

In one aspect, the present disclosure provides a complement receptor 2 (CR2)-factor H (FH) fusion protein (hereinafter referred to as CR2-FH fusion protein), comprising: a) a CR2 moiety comprising a CR2 fragment, and b) a FH moiety comprising a FH fragment, wherein the CR2 moiety comprises the first four N-terminal SCR domains of CR2 or a variant thereof (e.g., CR2(SCR1-SCR4) or a variant thereof, such as the amino acid sequence as shown in SEQ ID NO: 1 or SEQ ID NO: 46); the FH moiety comprises the first four N-terminal short consensus repeat (SCR) domains of FH (e.g., FH(SCR1-SCR4)). In some embodiments, provided is an isolated CR2-FH fusion protein. In some embodiments, the CR2 moiety and the FH moiety are directly or indirectly fused to each other in the form of a fusion protein. In some embodiments, the CR2 moiety and the FH moiety are covalently linked. In some embodiments, the CR2 moiety and the FH moiety are optionally linked by a linker sequence. In some embodiments, the CR2 moiety and the FH moiety are linked by an amino acid linker sequence. The linker sequence comprises a sequence represented by (G₄S)ₙ and/or an endogenous linking sequence, wherein n is an integer greater than 0.

A "isolated" substance refers to a substance or component that has been artificially obtained to be "isolated", which exists in a sufficiently pure state. In certain embodiments, the purity of the fusion protein is at least 90%, 93%, 95%, 96%, 97%, 98%, 99%, determined by electrophoresis (e.g., SDS-PAGE, isoelectric focusing, capillary electrophoresis), or chromatography (e.g., ion exchange chromatography or reversed phase HPLC).

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2 or a variant thereof (e.g., CR2(SCR1-SCR4) or a variant thereof, such as the amino acid sequence as shown in SEQ ID NO: 1 or SEQ ID NO: 46); the FH moiety comprises two or more FH fragments comprising the first four N-terminal SCR domains of FH (e.g., FH(SCR1-SCR4)), preferably, the two or more FH fragments are linked by a linker sequence, more preferably, the linker sequence comprises a sequence represented by (G₄S)ₙ and/or an endogenous linking sequence, wherein n is an integer greater than 0.

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2 or a variant thereof (e.g., CR2(SCR1-SCR4) or a variant thereof, such as the amino acid sequence as shown in SEQ ID NO: 1 or SEQ ID NO: 46); the FH moiety comprises the first four N-terminal SCR domains of FH (e.g., FH(SCR1-SCR4), the N-terminal eighth SCR domain of FH (e.g., FH(SCR8)), and the N-terminal nineteenth to twentieth SCR domains of FH (e.g., FH(SCR19-SCR20)).

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2 or a variant thereof (e.g., CR2(SCR1-SCR4) or a variant thereof, such as the amino acid sequence as shown in SEQ ID NO: 1 or SEQ ID NO: 46); wherein the FH moiety comprises the first four N-terminal SCR domains of FH (e.g., FH(SCR1-SCR4) and the N-terminal eighteenth to twentieth SCR domains of FH (e.g., FH(SCR18-SCR20)).

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2 or a variant thereof (e.g., CR2(SCR1-SCR4) or a variant thereof, such as the amino acid sequence as shown in SEQ ID NO: 1 or SEQ ID NO: 46); wherein the FH moiety comprises the first four N-terminal SCR domains of FH (e.g., FH(SCR1-SCR4), the N-terminal eighteenth SCR domain of FH (e.g., FH(SCR18)), and the N-terminal twentieth SCR domain of FH(e.g., FH(SCR20)).

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2 or a variant thereof (e.g., CR2(SCR1-SCR4) or a variant thereof, such as the amino acid sequence as shown in SEQ ID NO: 1 or SEQ ID NO: 46); wherein the FH moiety comprises the first four N-terminal SCR domains of two FHs (e.g., FH(SCR1-SCR4) and the N-terminal seventh SCR domain of FH (e.g., FH(SCR7)).

In some embodiments, the linker sequence comprises a sequence represented by (G₄S)ₙ and/or an endogenous linking sequence, wherein n is an integer greater than 0.

In some embodiments, the domains are linked by a linker sequence, and the linker sequence comprises a sequence represented by (G₄S)ₙ and/or an endogenous linking sequence, wherein n is an integer greater than 0.

In some embodiments, the first four N-terminal short consensus repeat (SCR) domains of FH comprise an amino acid sequence as shown in SEQ ID NO: 2.

In some embodiments, the N-terminal eighth SCR domain of FH comprises an amino acid sequence as shown in SEQ ID NO: 3.

In some embodiments, the N-terminal nineteenth to twentieth SCR domains of FH comprise an amino acid sequence as shown in SEQ ID NO: 4.

In some embodiments, the N-terminal eighteenth to twentieth SCR domains of FH comprise an amino acid sequence as shown in SEQ ID NO: 7.

In some embodiments, the N-terminal eighteenth SCR domain of FH comprises an amino acid sequence as shown in SEQ ID NO: 39.

In some embodiments, the N-terminal twentieth SCR domains of FH comprise an amino acid sequence as shown in SEQ ID NO: 40.

In some embodiments, the N-terminal seventh SCR domain of FH comprises an amino acid sequence as shown in SEQ ID NO: 43.

In some embodiments, the CR2 moiety or the variant thereof comprises an amino acid sequence as shown in SEQ ID NO: 1 or SEQ ID NO: 46.

In some embodiments, the CR2 moiety and the FH moiety are linked by a linker sequence, preferably, the linker sequence is represented by (G₄S)ₙ, wherein n is an integer greater than 0.

In some embodiments, the CR2-FH fusion protein comprises an amino acid sequence as shown in SEQ ID NO: 5, SEQ ID NO: 8, SEQ ID NO: 10, SEQ ID NO: 41, SEQ ID NO: 44, SEQ ID NO: 47, SEQ ID NO: 49, SEQ ID NO: 51, SEQ ID NO: 53, or SEQ ID NO: 55.

In some embodiments, the CR2-FH fusion protein comprises a signal peptide sequence, preferably, the signal peptide sequence is located at the N-terminus, more preferably, the amino acid sequence of the signal peptide is as shown in SEQ ID NO: 17.

In some embodiments, the CR2-FH fusion protein comprises an amino acid sequence as shown in SEQ ID NO: 6, SEQ ID NO: 9, SEQ ID NO: 11, SEQ ID NO: 42, SEQ ID NO: 45, SEQ ID NO: 48, SEQ ID NO: 50, SEQ ID NO: 52, SEQ ID NO: 54, or SEQ ID NO: 56.

In another aspect, the present disclosure provides a polynucleotide encoding the CR2-FH fusion protein described herein. In some embodiments, the polynucleotide sequence encoding the first four N-terminal short consensus repeat (SCR) domains of FH comprises a sequence as shown in SEQ ID NO: 19, 28 or 29. In some embodiments, the polynucleotide sequence encoding the N-terminal eighth SCR domain of FH comprises a sequence as shown in SEQ ID NO: 20. In some embodiments, the polynucleotide sequence encoding the N-terminal nineteenth to twentieth SCR domains of FH comprises a sequence as shown in SEQ ID NO: 21. In some embodiments, the polynucleotide sequence encoding the N-terminal eighteenth to twentieth SCR domains of FH comprises a sequence as shown in SEQ ID NO: 24. In some embodiments, the polynucleotide sequence encoding the CR2 moiety comprises a sequence as shown in SEQ ID NO: 18. In some embodiments, the polynucleotide sequence encoding the N-terminal eighteenth SCR domain of FH comprises a sequence as shown in SEQ ID NO: 57. In some embodiments, the polynucleotide sequence encoding the N-terminal twentieth SCR domain of FH comprises a sequence as shown in SEQ ID NO: 58. In some embodiments, the polynucleotide sequence encoding the N-terminal seventh SCR domain of FH comprises an amino acid sequence as shown in SEQ ID NO: 61.

In some embodiments, the nucleotide sequence encoding the CR2-FH fusion protein comprises a sequence as shown in SEQ ID NO: 22, SEQ ID NO: 25, SEQ ID NO: 30, SEQ ID NO: 59, SEQ ID NO: 62, SEQ ID NO: 65, SEQ ID NO: 67, SEQ ID NO: 70, SEQ ID NO: 72, or SEQ ID NO: 74.

In some embodiments, the polynucleotide sequence encoding the CR2-FH fusion protein comprises a polynucleotide sequence encoding a signal peptide sequence, preferably, the polynucleotide sequence encoding the signal peptide is located at the 5' end of the polynucleotide sequence encoding the CR2-FH fusion protein, more preferably, the polynucleotide sequence encoding the signal peptide is as shown in SEQ ID NO: 37 or 38.

In some embodiments, the polynucleotide sequence encoding the CR2-FH fusion protein comprises a sequence as shown in SEQ ID NO: 23, SEQ ID NO: 26, SEQ ID NO: 31, SEQ ID NO: 60, SEQ ID NO: 63, SEQ ID NO: 66, SEQ ID NO: 68, SEQ ID NO: 71, SEQ ID NO: 73, or SEQ ID NO: 75.

In another aspect, the present disclosure provides a vector encoding the polynucleotide described herein. In some embodiments, the vector is at least one selected from adeno-associated virus (AAV) vector, adenovirus vector, RNA virus vector, lentiviral vector, and vaccinia virus vector.

In another aspect, the present disclosure provides a host cell comprising the polynucleotide described herein or the vector described herein.

In another aspect, the present disclosure provides an AAV particle comprising the AAV vector described herein.

In another aspect, the present disclosure provides a pharmaceutical composition comprising the CR2-FH fusion protein described herein, the polynucleotide described herein, the vector described herein, the host cell described herein, and the AAV particle described herein,

and a pharmaceutically acceptable carrier.

In some embodiments, the composition is suitable for intraocular, intravenous, intra-arterial, subcutaneous, endotracheal or inhalation administration.

In another aspect, the present disclosure provides use of the CR2-FH fusion protein described herein, the polynucleotide described herein, the vector described herein, the host cells described herein, the AAV particle described herein, or the pharmaceutical composition described herein in the preparation of a medicament for treating an alternative complement pathway-related disease in a subject.

The present disclosure provides a method for treating an alternative complement pathway-related disease in a subject in need thereof, wherein an effective amount of the CR2-FH fusion protein described herein, the polynucleotide described herein, the vector described herein, the host cell described herein, the AAV particle described herein, or the pharmaceutical composition described herein is administered.

In some embodiments, the alternative complement pathway-related disease is an inflammatory disease or an autoimmune disease.

In some embodiments, the alternative complement pathway-related disease is age-related macular degeneration, preferably dry age-related macular degeneration.

In some embodiments, the alternative complement pathway-related disease is a symptom of microangiopathic hemolytic anemia, thrombocytopenia, or acute renal failure.

In some embodiments, the alternative complement pathway-related disease is selected from the group consisting of macular degeneration, ischemia reperfusion, organ transplant rejection, drusen-related diseases, pregnancy-related diseases, adverse drug reactions, and post-cardiopulmonary bypass complications.

In some embodiments, the alternative complement pathway-related disease is selected from the group consisting of age-related macular degeneration (AMD), rheumatoid arthritis, C3 glomerulonephritis, membrane proliferative glomerulonephritis II (MPGN II), Factor H-related hemolytic uremic syndrome (HUS), paroxysmal nocturnal hemoglobinuria (PNH), systemic lupus erythematosus (SLE), lupus nephritis, stroke, myocardial infarction, acute respiratory distress syndrome (ARDS), sepsis, burns, inflammation associated with cardiopulmonary bypass and hemodialysis, plasmapheresis, platelet isolation, leukapheresis, extracorporeal membrane oxygenation (ECMO), heparin-induced extracorporeal LDL precipitation (HELP), and radiological contrast induced allergic reactions.

### BRIEF DESCRIPTION OF THE DRAWINGS

FIG. 1 shows a schematic diagram of XMDC025, XMDC026, XMDC029, CR2-FH(1-4) and CR2-FH(1-5).
FIGs. 2A-E show vector information of ssAAV-XMDC025, ssAAV-XMDC026, ssAAV-XMDC029, ssAAV-CR2-FH(1-4), and ssAAV-CR2-FH(1-5).
FIGs. 3A-B show the effect of AAV-XMDC025, AAV-XMDC026, AAV-XMDC029, AAV-CR2-FH(1-4), and AAV-CR2-FH(1-5) on the thickness of outer nuclear layer of the retina in PEG-400-induced dry AMD model mice.
FIGs. 4A-B show the effect of AAV-XMDC025, AAV-XMDC026, AAV-XMDC029, AAV-CR2-FH(1-4), and AAV-CR2-FH(1-5) on the nuclear density of outer nuclear layer of the retina in PEG-400-induced dry AMD model mice.
FIGs. 5A-B show retinal HE staining images of PEG-400-induced dry AMD model mice injected with AAV-XMDC025, AAV-XMDC026, AAV-XMDC029, AAV-CR2-FH(1-4), and AAV-CR2-FH(1-5).
FIGS. 6A-B show the effect of AAV-XMDC025, AAV-XMDC026, AAV-XMDC029, AAV-CR2-FH(1-4), AAV-CR2-FH(1-5) on the number of retinal cone cells in PEG-400-induced dry AMD model mice.
FIGs. 7A-B show the immunofluorescence staining images of cone cells in PEG-400-induced dry AMD model mice injected with AAV-XMDC025, AAV-XMDC026, AAV-XMDC029, AAV-CR2-FH(1-4), and AAV-CR2-FH(1-5).
FIGs. 8A-B show the effects of AAV-XMDC025, AAV-XMDC026, AAV-XMDC029, AAV-CR2-FH(1-4), and AAV-CR2-FH(1-5) on the thickness of retinal rod outer segments in PEG-400-induced dry AMD model mice.
FIGs. 9A-B show the immunofluorescence staining images of retinal rod cells in PEG-400-induced dry AMD model mice treated with AAV-XMDC025, AAV-XMDC026, AAV-XMDC029, AAV-CR2-FH(1-4), and AAV-CR2-FH(1-5).
FIGs. 10A-B show the effects of AAV-XMDC025, AAV-XMDC026, AAV-XMDC029, AAV-CR2-FH(1-4), and AAV-CR2-FH(1-5) on RPE cells in PEG-400-induced dry AMD model mice.
FIGS. 11A-B show F-actin staining images of RPE cells in PEG-400-induced dry AMD model mice injected with AAV-XMDC025, AAV-XMDC026, AAV-XMDC029, AAV-CR2-FH(1-4), and AAV-CR2-FH(1-5).
FIG. 12 shows a schematic diagram of XMDC061, XMDC062, XMDC025-(CR2-R36A K41A K67A), XMDC026-(CR2-R36A K41A K67A), XMDC029-(CR2-R36A K41A K67A), XMDC061-(CR2-R36A K41A K67A), and XMDC062-(CR2-R36A K41A K67A).
FIGs. 13A-G show vector information of ssAAV-XMDC061, ssAAV-XMDC062, ssAAV-XMDC025-(CR2-R36A K41A K67A), ssAAV-XMDC026-(CR2-R36A K41A K67A), ssAAV-XMDC029-(CR2-R36A K41A K67A), ssAAV-XMDC061-(CR2-R36A K41A K67A) and ss-AAVXMDC062-(CR2-R36A K41A K67A).
FIGs. 14A-B show schematic diagram of CR2-FH(SCR1-5+1-5) and vector information diagram of ssAAV-CR2-FH(SCR1-5+1-5).
FIG. 15 shows the effects of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5) on the thickness of the outer nuclear layer of the retina in PEG-400-induced dry AMD model mice.
FIG. 16 shows the effects of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5) on the nuclear density of the outer nuclear layer of the retina in PEG-400-induced dry AMD model mice.
FIG. 17 shows retinal HE staining images of PEG-400-induced dry AMD model mice injected with AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5).
FIG. 18 shows the effects of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5) on the number of cone cells of the retina in PEG-400-induced dry AMD model mice.
FIG. 19 shows cone cell immunofluorescence staining image of PEG-400-induced dry AMD model mice injected with AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5).
FIG. 20 shows the effects of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5) on the thickness of the outer segments of rod cells of the retina in PEG-400-induced dry AMD model mice.
FIG. 21 shows immunofluorescence staining images of retinal rod cells in PEG-400-induced dry AMD model mice with AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5).
FIG. 22 shows the effects of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5) on RPE cells in PEG-400-induced dry AMD model mice.
FIG. 23 shows F-actin staining image of RPE cells of PEG-400-induced dry AMD model mice injected with AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5).

### DETAILED DESCRIPTION

The following description of the present disclosure is merely intended to illustrate various different embodiments of the present disclosure. Accordingly, specific modifications discussed should not be construed as limiting the scope of the disclosure. It will be apparent to those skilled in the art that various different equivalents, variations, and modifications can be made without departing from the scope of the disclosure, and it should be understood that such equivalent embodiments are to be encompassed herein. All references, including publications, patents, and patent applications cited herein are hereby incorporated by reference in their entirety.

In one aspect, the present disclosure provides a complement receptor 2 (CR2)-factor H (FH) fusion protein (hereinafter referred to as CR2-FH fusion protein), comprising: a) a CR2 moiety comprising a CR2 fragment, and b) a FH moiety comprising a FH fragment, wherein the CR2 moiety comprises the first four N-terminal SCR domains of CR2 or a variant thereof; the FH moiety comprises the first four N-terminal short consensus repeat (SCR) domains of FH. In some embodiments, provided is an isolated CR2-FH fusion protein. In some embodiments, the CR2 moiety and the FH moiety are directly or indirectly fused to each other in the form of a fusion protein. In some embodiments, the CR2 moiety and the FH moiety are covalently linked. In some embodiments, the CR2 moiety and the FH moiety are linked by an amino acid linker sequence. In some embodiments, the first four N-terminal short consensus repeat (SCR) domains of FH comprise an amino acid sequence as shown in SEQ ID NO: 2. In some embodiments, the CR2 moiety comprises an amino acid sequence as shown in SEQ ID NO: 1 or SEQ ID NO: 46. In some embodiments, the CR2 moiety and the FH moiety are linked by a linker sequence, preferably, the linker sequence comprises a sequence represented by (G₄S)ₙ and/or an endogenous linking sequence, wherein n is an integer greater than 0.

As used herein, "fusion protein" refers to two or more peptides, polypeptides or proteins operably linked to each another. In some embodiments, the CR2 moiety and the FH moiety in the CR2-FH fusion protein are directly fused to each other. In some embodiments, the CR2 moiety and the FH moiety are linked by an amino acid linker sequence. Examples of linker sequences are known in the art and include, for example, (Gly₄Ser), (Gly₄Ser)₂, (Gly₄Ser)₃, (Gly₃Ser)₄, (SerGly₄), (SerGly₄)₂, (SerGly₄)₃ and (SerGly₄)₄. The linking sequence may also comprise a "natural" linking sequence found between different domains of a complement factor, which is also referred to as endogenous linking sequence. The order of the CR2 moiety and the FH moiety in the fusion protein may vary. For example, in some embodiments, the C-terminus of the CR2 moiety is fused (directly or indirectly) to the N-terminus of FH moiety of the molecule. In some embodiments, the N-terminus of the CR2 moiety is fused (directly or indirectly) to the C-terminus of FH moiety of the molecule.

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2; the FH moiety comprises two or more FH fragments comprising the first four N-terminal SCR domains of FH, preferably, the two or more FH fragments are linked by a linker sequence, more preferably the linker sequence comprises a sequence represented by (G₄S)ₙ and/or an endogenous sequence, wherein n is an integer greater than 0. In some embodiments, the first four N-terminal short consensus repeat (SCR) domains of FH comprise an amino acid sequence as shown in SEQ ID NO: 2. In some embodiments, the CR2 moiety comprises an amino acid sequence as shown in SEQ ID NO: 1 or SEQ ID NO: 46.

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2; the FH moiety comprises the first four N-terminal SCR domains of FH, the N-terminal eighth SCR domain of FH, and the N-terminal nineteenth to twentieth SCR domains of FH. In some embodiments, the first four N-terminal short consensus repeat (SCR) domains of FH comprise an amino acid sequence as shown in SEQ ID NO: 2. In some embodiments, the CR2 moiety comprises an amino acid sequence as shown in SEQ ID NO: 1 or SEQ ID NO: 46. In some embodiments, the N-terminal eighth SCR domain of FH comprises an amino acid sequence as shown in SEQ ID NO: 3. In some embodiments, the N-terminal nineteenth to twentieth SCR domains of FH comprise an amino acid sequence as shown in SEQ ID NO: 4. In some embodiments, the CR2 moiety and the FH moiety are linked by a linker sequence, preferably, the linker sequence comprises a sequence represented by (G₄S)ₙ and/or an endogenous linking sequence, wherein n is an integer greater than 0.

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2; the FH moiety comprises the first four N-terminal SCR domains of FH, and the N-terminal eighteenth to twentieth SCR domains of FH. In some embodiments, the first four N-terminal short consensus repeat (SCR) domains of FH comprise an amino acid sequence as shown in SEQ ID NO: 2. In some embodiments, the CR2 moiety comprises an amino acid sequence as shown in SEQ ID NO: 1. In some embodiments, the N-terminal eighteenth to twentieth SCR domains of FH comprise an amino acid sequence as shown in SEQ ID NO: 7. In some embodiments, the CR2 moiety and the FH moiety are linked by a linker sequence, preferably, the linker sequence comprises a sequence represented by (G₄S)ₙ and/or an endogenous linking sequence, wherein n is an integer greater than 0.

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2 or a variant thereof (e.g., CR2(SCR1-SCR4) or a variant thereof, such as the amino acid sequence as shown in SEQ ID NO: 1 or SEQ ID NO: 46); wherein the FH moiety comprises the first four N-terminal SCR domains of FH (e.g., FH(SCR1-SCR4), the N-terminal eighteenth SCR domain of FH (e.g., FH(SCR18)), and the N-terminal twentieth SCR domain of FH(e.g., FH(SCR20)). In some embodiments, the N-terminal eighteenth SCR domain of FH comprises an amino acid sequence as shown in SEQ ID NO: 39. In some embodiments, the N-terminal twentieth SCR domains of FH comprise an amino acid sequence as shown in SEQ ID NO: 40. In some embodiments, the CR2 moiety and the FH moiety are linked by a linker sequence, preferably, the linker sequence comprises a sequence represented by (G₄S)ₙ and/or an endogenous linking sequence, wherein n is an integer greater than 0.

In some embodiments, the provided CR2-FH fusion protein comprises: a) a CR2 moiety comprising a CR2 fragment, and b) a FH moiety comprising a FH fragment, wherein the CR2 moiety comprises the first four N-terminal SCR domains of CR2 or a variant thereof (e.g., CR2(SCR1-SCR4) or a variant thereof, such as the amino acid sequence as shown in SEQ ID NO: 1 or SEQ ID NO: 46); wherein the FH moiety comprises two copies of the first four N-terminal SCR domains of FH (e.g., FH(SCR1-SCR4) and the N-terminal seventh SCR domain of FH (e.g., FH(SCR7)). In some embodiments, the N-terminal seventh SCR domain of FH comprises an amino acid sequence as shown in SEQ ID NO: 43.

In some embodiments, the linker sequence comprises a sequence represented by (G₄S)ₙ and/or an FH endogenous linking sequence, wherein n is an integer greater than 0.

In some embodiments, the domains are linked by a linker sequence, and the linker sequence comprises a sequence represented by (G₄S)ₙ and/or an FH endogenous linking sequence, wherein n is an integer greater than 0.

In some embodiments, the CR2-FH fusion protein comprises an amino acid sequence as shown in SEQ ID NO: 5, SEQ ID NO: 8, SEQ ID NO: 10, SEQ ID NO: 41, SEQ ID NO: 44, SEQ ID NO: 47, SEQ ID NO: 49, SEQ ID NO: 51, SEQ ID NO: 53, or SEQ ID NO: 55 or an amino acid sequence having at least about 50%, 60%, 70%, 80%, 90%, 91%, 92%, 93%, 94%, 95%, 96%, 97%, 98%, or 99% identity to SEQ ID NO: 5, SEQ ID NO: 8, or SEQ ID NO: 10, wherein the CR2-FH fusion protein has a dual function of binding to a CR2 ligand and inhibiting complement activation of the alternative pathway. The CR2-FH fusion protein can bind to CR2 ligands with a binding affinity of any one of approximately 10%, 20%, 30%, 40%, 50%, 60%, 70%, 80%, 90%, or 100% of that of the CR2 protein. Binding affinity can be determined by any method known in the art, including, for example, surface plasmon resonance, calorimetry titration, ELISA, and flow cytometry. CR2-FH fusion proteins can also inhibit complement activation of the alternative pathway, with a complement inhibitory activity of any one or higher of about 50%, 60%, 70%, 80%, 90%, or 100% of the complement inhibitory activity of the FH protein.

In some embodiments, provided is an isolated CR2-FH fusion protein. In some embodiments, the CR2-FH fusion protein forms a dimer or multimer.

In some embodiments, the CR2-FH fusion protein comprises a signal peptide sequence, preferably, the signal peptide sequence is located at the N-terminus, more preferably, the amino acid sequence of the signal peptide is as shown in SEQ ID NO: 17.

In some embodiments, the CR2-FH fusion protein comprises an amino acid sequence as shown in SEQ ID NO: 6, SEQ ID NO: 9, SEQ ID NO: 11, SEQ ID NO: 42, SEQ ID NO: 45, SEQ ID NO: 48, SEQ ID NO: 50, SEQ ID NO: 52, SEQ ID NO: 54, or SEQ ID NO: 56, or an amino acid sequence having an identity of at least about 50%, 60%, 70%, 80%, 90%, 91%, 92%, 93%, 94%, 95%, 96%, 97%, 98%, or 99% to SEQ ID NO: 6, SEQ ID NO: 9, or SEQ ID NO: 11.

### CR2 Moiety

The CR2 moiety described herein comprises CR2 or a fragment thereof. CR2 is a transmembrane protein expressed primarily on mature B cells and follicular dendritic cells. CR2 is a member of the C3 binding protein family. The natural ligands of CR2 are iC3b, C3dg, and C3d, which are decomposed fragments of C3. The cleavage of C3 initially results in the production and deposition of C3b on the activated cell surface, and C3b fragments participate in the production of enzyme complexes that amplify the complement cascade reaction. At the cell surface, C3b rapidly converts to inactive iC3b, particularly when deposited on a host surface containing a complement activation modulator. Even without membrane-bound complement regulatory factors, a significant level of iC3b can be formed due to the function of FH. Subsequently, iC3b is digested by factor I (FI) and other proteases into membrane binding fragments C3dg and C3d, but this process is relatively slow. Thus, the C3 ligands of CR2 are relatively long-lived once produced and are present at a high concentration in the complement activation site. CR2 can thus act as an effective targeting carrier that brings molecules to complement activation sites. CR2 contains an extracellular portion having 15 or 16 repeat units called short consensus repeat (SCR domains). The SCR domain has a typical framework of highly conserved residues comprising four cysteines, two proline, one tryptophan, and several other partially conserved glycine and hydrophobic residues. The SCR1-4 domain is located at amino acid positions 23-271 of human CR2 protein sequence. In some embodiments of the present disclosure, the CR2 moiety comprises the first four N-terminal SCR domains of CR2. In some embodiments, the CR2 moiety comprises an amino acid sequence as shown in SEQ ID NO: 1. Variants of the CR2 moiety may also comprise some mutation sites, and a variant of the CR2 moiety comprises an amino acid sequence as shown in SEQ ID NO: 46.

### H Factor Moiety (FH Moiety)

The FH moiety of the CR2-FH fusion protein described herein comprises FH or a fragment thereof. Complement factor H(FH) is a single polypeptide chain plasma glycoprotein. The protein consists of 20 repeat SCR domains of approximately 60 amino acids arranged in a string of 20 bead-like sequences. H factor binds to C3b, accelerates the decay of alternative pathway C3 convertase (C3Bb), and acts as a cofactor for the hydrolytic inactivation of C3b protein. C3b protein hydrolysis resulted in cleavage of C3b in the presence of H factor. The SCR1-4 domain is located at amino acid positions 21-262 of human complement factor H protein sequence. In some embodiments of the present disclosure, the FH moiety comprises two or more FH fragments comprising the first four N-terminal SCR domains of FH, preferably the two or more FH fragments are linked by a linker sequence, more preferably the linker sequence is presented by (G₄S)ₙ, wherein n is an integer greater than 0. In some embodiments, the first four N-terminal short consensus repeat (SCR) domains of FH comprise an amino acid sequence as shown in SEQ ID NO: 2. In some embodiments of the present disclosure, the FH moiety comprises the first four N-terminal SCR domains of FH, the N-terminal eighth SCR domain of FH, and the N-terminal nineteenth to twentieth SCR domains of FH. In some embodiments of the present disclosure, the FH moiety comprises the first four N-terminal SCR domains of FH and the N-terminal eighteenth to twentieth SCR domains of FH. In some embodiments, the FH is a wild-type FH. In some embodiments, the FH moiety is a fragment of wild-type FH or formed by the direct or indirect linkage of several fragments of wild-type FH.

In another aspect, the present disclosure provides a polynucleotide encoding the CR2-FH fusion protein described herein. In some embodiments, the polynucleotide sequence encoding the first four N-terminal short consensus repeat (SCR) domains of FH comprises a sequence as shown in SEQ ID NO: 19, 28 or 29. In some embodiments, the polynucleotide sequence encoding the N-terminal eighth SCR domain of FH comprises a sequence as shown in SEQ ID NO: 20. In some embodiments, the polynucleotide sequence encoding the N-terminal nineteenth to twentieth SCR domains of FH comprises a sequence as shown in SEQ ID NO: 21. In some embodiments, the polynucleotide sequence encoding the N-terminal eighteenth to twentieth SCR domains of FH comprises a sequence as shown in SEQ ID NO: 24. In some embodiments, the polynucleotide sequence encoding the CR2 moiety comprises a sequence as shown in SEQ ID NO: 18. In some embodiments, the polynucleotide sequence encoding the N-terminal eighteenth SCR domain of FH comprises a polynucleotide sequence as shown in SEQ ID NO: 57. In some embodiments, the polynucleotide sequence encoding the N-terminal twentieth SCR domain of FH comprises a polynucleotide sequence as shown in SEQ ID NO: 58. In some embodiments, the polynucleotide sequence encoding the N-terminal seventh SCR domain of FH comprises a polynucleotide sequence as shown in SEQ ID NO: 61.

In some embodiments, the nucleotide sequence encoding the CR2-FH fusion protein comprises a sequence as shown in SEQ ID NO: 22, SEQ ID NO: 25, SEQ ID NO: 30, SEQ ID NO: 59, SEQ ID NO: 62, SEQ ID NO: 65, SEQ ID NO: 67, SEQ ID NO: 70, SEQ ID NO: 72, or SEQ ID NO: 74, or a sequence having at least about 50%, 60%, 70%, 80%, 90%, 91%, 92%, 93%, 94%, 95%, 96%, 97%, 98%, or 99% identity to SEQ ID NO: 22, SEQ ID NO: 25, SEQ ID NO: 30, SEQ ID NO: 59, SEQ ID NO: 62, SEQ ID NO: 65, SEQ ID NO: 67, SEQ ID NO: 70, SEQ ID NO: 72, or SEQ ID NO: 74.

In some embodiments, the polynucleotide sequence is DNA or RNA, such as an mRNA sequence.

In some embodiments, the polynucleotide sequence encoding the CR2-FH fusion protein comprises a polynucleotide sequence encoding a signal peptide sequence, preferably, the polynucleotide sequence encoding the signal peptide is located at the 5' end of the polynucleotide sequence encoding the CR2-FH fusion protein, more preferably, the polynucleotide sequence encoding the signal peptide is as shown in SEQ ID NO: 37 or 38.

In some embodiments, the polynucleotide sequence encoding the CR2-FH fusion protein comprises a sequence as shown in SEQ ID NO: 23, SEQ ID NO: 26, SEQ ID NO: 31, SEQ ID NO: 60, SEQ ID NO: 63, SEQ ID NO: 66, SEQ ID NO: 68, SEQ ID NO: 71, SEQ ID NO: 73, or SEQ ID NO: 75, or a sequence having at least about 50%, 60%, 70%, 80%, 90%, 91%, 92%, 93%, 94%, 95%, 96%, 97%, 98%, or 99% identity to SEQ ID NO: 23, SEQ ID NO: 26, SEQ ID NO: 31, SEQ ID NO: 60, SEQ ID NO: 63, SEQ ID NO: 66, SEQ ID NO: 68, SEQ ID NO: 71, SEQ ID NO: 73, or SEQ ID NO: 75.

In another aspect, the present disclosure provides a vector encoding the polynucleotide described herein. In some embodiments, the vector is at least one selected from adeno-associated virus (AAV) vector, adenovirus vector, RNA virus vector, lentiviral vector, and vaccinia virus vector.

In another aspect, the present disclosure provides a host cell comprising the polynucleotide described herein or the vector described herein.

In another aspect, the present disclosure provides an AAV particle comprising the AAV vector described herein. In some embodiments, the AAV particle is produced by a production cell line comprising one or more nucleic acids encoding an AAV vector, a nucleic acid encoding AAV rep and cap, and a nucleic acid encoding an AAV helper virus function.

In some embodiments, the serotype of the AAV is AAV1, AAV2, AAV5, AAV6, AAV7, AAV8, AAVrh8, AAVrh8R, AAV9, AAV10, or AAVrh10. In some embodiments, the AAV vector comprises one or more AAV inverted terminal repeat (ITR) sequences flanking it. In some embodiments, the heterologous nucleic acid is flanked by two AAV ITRs. In some embodiments, the AAV ITR is an ITR of AAV1, AAV2, AAV3, AAV4, AAV5, AAV6, AAV7, AAV8, AAVrh8, AAVrh8R, AAV9, AAV10, AAVrh10 serotypes. In some embodiments, the AAV ITR is an AAV2 ITR. In some embodiments, the ITR and capsid of the AAV particles are derived from the same AAV serotype. In some embodiments, the ITR and the capsid are derived from AAV2. In other embodiments, the ITR and capsid of the AAV virus particles are derived from different AAV serotypes.

In some embodiments, the AAV vector comprises one or more promoters, enhancers, or polyadenylation signals.

In another aspect, the present disclosure provides a pharmaceutical composition comprising the CR2-FH fusion protein described herein, the polynucleotide described herein, the vector described herein, the host cell described herein, and the AAV particle described herein,
and a pharmaceutically acceptable carrier.

In some embodiments, the composition is suitable for intraocular, intravenous, intra-arterial, subcutaneous, endotracheal or inhalation administration.

In another aspect, the present disclosure provides a use of the CR2-FH fusion protein described herein, the polynucleotide described herein, the vector described herein, the host cells described herein, the AAV particle described herein, or the pharmaceutical composition described herein in the preparation of a medicament for treating an alternative complement pathway-related disease in a subject.

The present disclosure provides a method for treating an alternative complement pathway-related disease in a subject in need thereof, wherein an effective amount of the CR2-FH fusion protein described herein, the polynucleotide described herein, the vector described herein, the host cell described herein, the AAV particle described herein, or the pharmaceutical composition described herein is administered.

In some embodiments, the alternative complement pathway-related disease is an inflammatory disease or an autoimmune disease.

In some embodiments, the alternative complement pathway-related disease is age-related macular degeneration, preferably dry age-related macular degeneration.

In some embodiments, the alternative complement pathway-related disease is a symptom of microangiopathic hemolytic anemia, thrombocytopenia, or acute renal failure.

In some embodiments, the alternative complement pathway-related disease is selected from the group consisting of macular degeneration, ischemia reperfusion, organ transplant rejection, drusen-related diseases, pregnancy-related diseases, adverse drug reactions, and post-cardiopulmonary bypass complications.

In some embodiments, the alternative complement pathway-related disease is selected from the group consisting of age-related macular degeneration (AMD), rheumatoid arthritis, C3 glomerulonephritis, membrane proliferative glomerulonephritis II (MPGN II), Factor H-related hemolytic uremic syndrome (HUS), paroxysmal nocturnal hemoglobinuria (PNH), systemic lupus erythematosus (SLE), lupus nephritis, stroke, myocardial infarction, acute respiratory distress syndrome (ARDS), sepsis, burns, inflammation associated with cardiopulmonary bypass and hemodialysis, plasmapheresis, platelet isolation, leukapheresis, extracorporeal membrane oxygenation (ECMO), heparin-induced extracorporeal LDL precipitation (HELP), and radiological contrast induced allergic reactions.

In some embodiments, the subject is a mammal, preferably a human.

The present disclosure will be described in further detail below. However, the manner of implementing the present disclosure is not limited to the following embodiments.

### Example 1: Construction of AAV Plasmid Vector Expressing CR2 and FH Fragment Genes

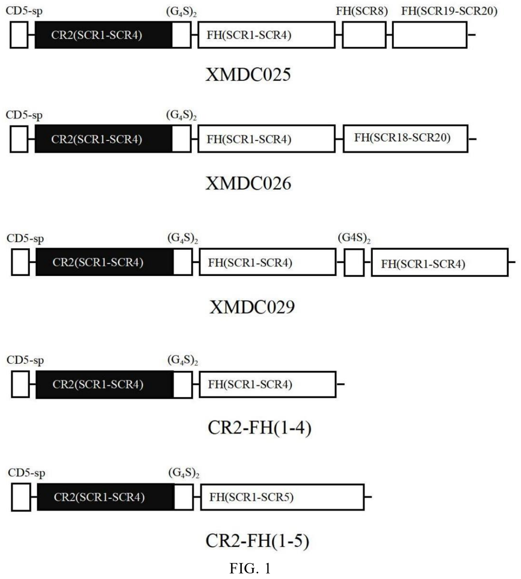

According to the amino acid sequence of CR2 (Gene ID: 1380) SCR1-SCR4 (SEQ ID NO: 1), and FH (Gene ID: 3075) SCR1-SCR4 (SEQ ID NO: 2), SCR8 (SEQ ID NO: 3), SCR19-SCR20 (SEQ ID NO: 4) published on NCBI, adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+FH(SCR8)+linker+FH(SCR19-SCR20)+linker was constructed. The nucleotide sequence was designed according to human codon preference, with a BamH I restriction site introduced at the 5' end and an EcoR V restriction site introduced at the 3' end. It was named XMDC025 (the full-length gene was synthesized by Sangon Biotech (Shanghai) Co., Ltd.), and its structural diagram is shown in Figure 1.

According to the amino acid sequence of CR2 (Gene ID: 1380) SCR1-SCR4 (SEQ ID NO: 1), and FH (Gene ID: 3075) SCR1-SCR4 (SEQ ID NO: 2), SCR18-SCR20 (SEQ ID NO: 7), adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+FH(SCR18-SCR20)+linker was constructed. The nucleotide sequence was designed according to human codon preference, with a BamH I restriction site introduced at the 5' end and an EcoR V restriction site introduced at the 3' end. It was named XMDC026 (the full-length gene was synthesized by Sangon Biotech (Shanghai) Co., Ltd.), and its structural diagram is shown in Figure 1.

According to the amino acid sequence of CR2 (Gene ID: 1380) SCR1-SCR4 (SEQ ID NO: 1) and FH (Gene ID: 3075) SCR1-SCR4 (SEQ ID NO: 2), adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+(G₄S)₂+linker+FH(SCR1-SCR4)+linker was constructed. The nucleotide sequence was designed according to human codon preference, with a BamH I restriction site introduced at the 5' end and an EcoR V restriction site introduced at the 3' end. It was named XMDC029 (the full-length gene was synthesized by Suzhou Genewiz Biotechnology Co., Ltd.), and its structural diagram is shown in Figure 1.

According to the amino acid sequence of CR2 (Gene ID: 1380) SCR1-SCR4 (SEQ ID NO: 1) and FH (Gene ID: 3075) SCR1-SCR4 (SEQ ID NO: 2), adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker was constructed. The nucleotide sequence was designed according to human codon preference, with a BamH I restriction site introduced at the 5' end and an EcoR V restriction site introduced at the 3' end It was named CR2-FH(1-4) (the full-length gene was synthesized by Sangon Biotech (Shanghai) Co., Ltd.), and its structural diagram is shown in Figure 1.

According to the amino acid sequence of CR2 (Gene ID: 1380) SCR1-SCR4 (SEQ ID NO: 1) and FH (Gene ID: 3075) SCR1-SCR5 (SEQ ID NO: 14), adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR5)+linker was constructed. The nucleotide sequence was designed according to human codon preference, with a BamH I restriction site introduced at the 5' end and an EcoR V restriction site introduced at the 3' end. It was named CR2-FH(1-5) (the full-length gene was synthesized by Sangon Biotech (Shanghai) Co., Ltd.), and its structural diagram is shown in Figure 1.

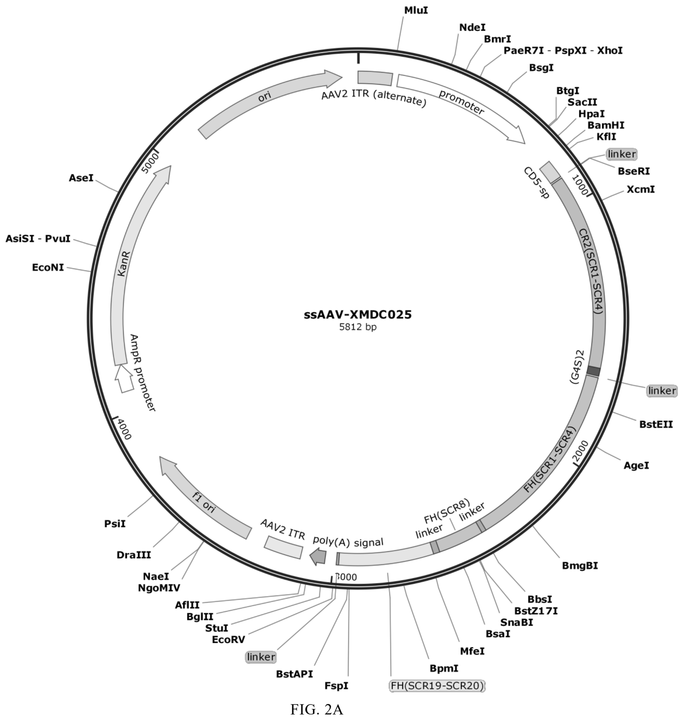

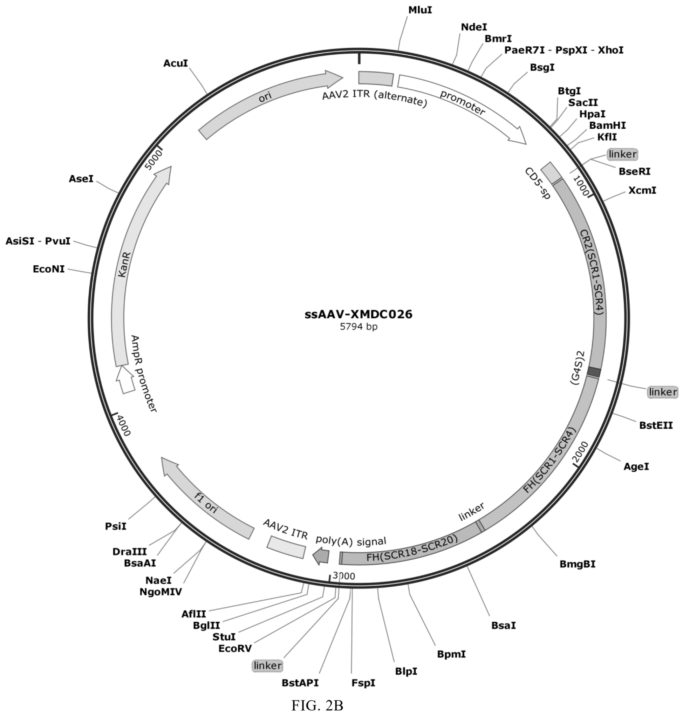

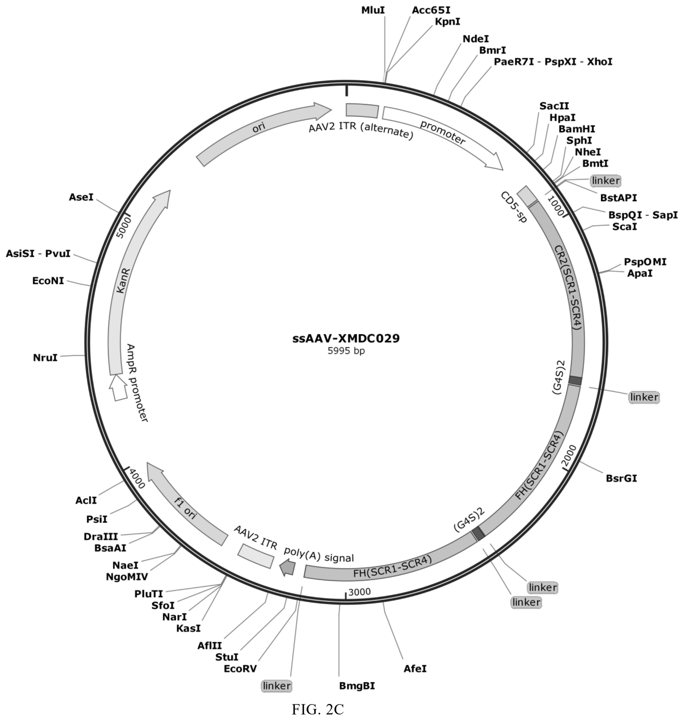

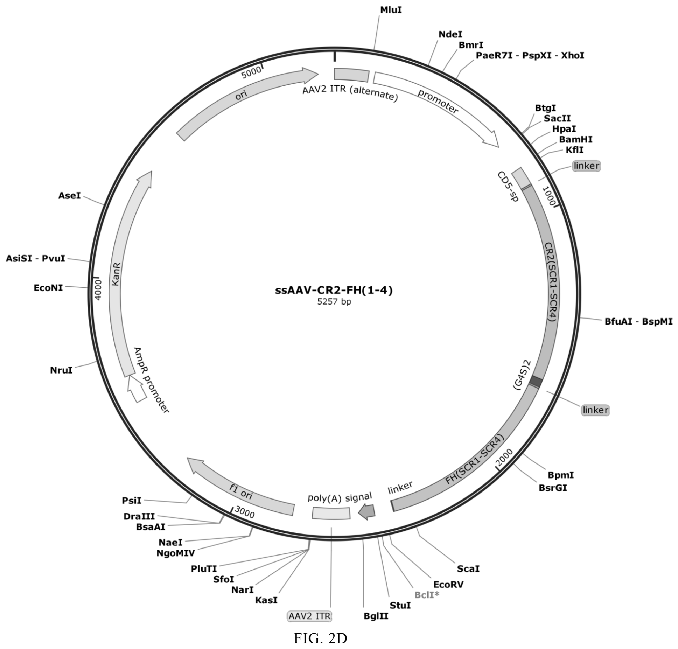

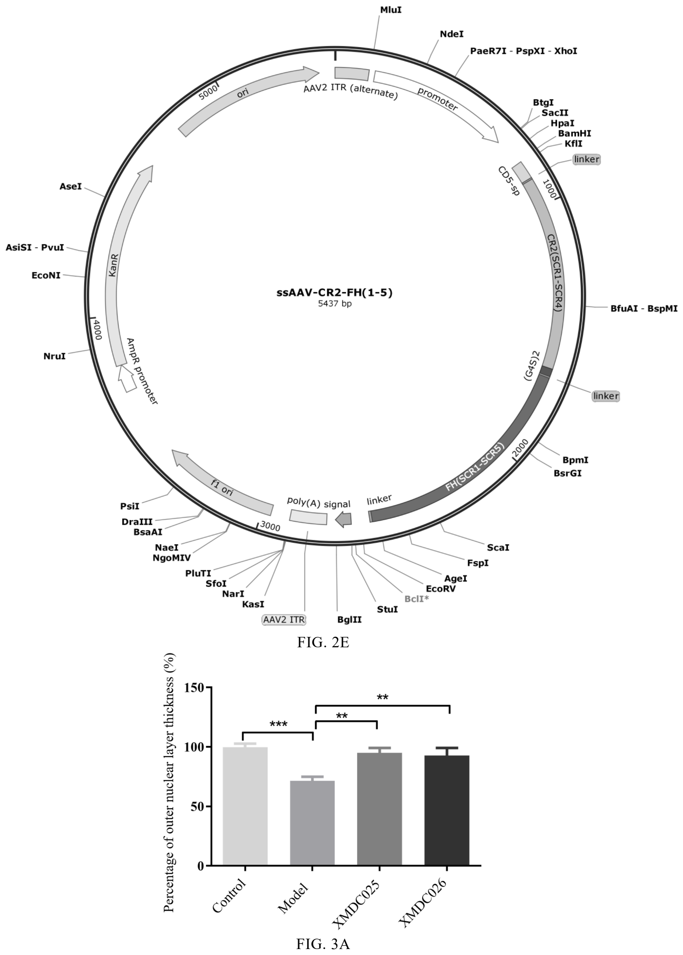

The ssAAV-XMDC025, ssAAV-XMDC026, ssAAV-XMDC029, ssAAV-CR2-FH(1-4), and ssAAV-CR2-FH(1-5) vectors were constructed by double digestion of XMDC025, XMDC026, XMDC029, CR2-FH(1-4), CR2-FH(1-5), and ssAAV plasmids with BamH I/EcoR V, followed by conventional molecular biology operations such as ligation, transformation, as well as clone screening and identification. The vector information is shown in Figure 2. High-quality plasmid DNA was obtained for later use using an endotoxin-free plasmid extraction kit (MN).

The endogenous linker in the above structure is the endogenous linking sequence in the CR2 or FH structure. Therefore, those skilled in the art should understand that the linker sequence in the structure is determined according to different CR2 or FH structure sequences linked at the C-terminus or the N-terminus thereof.

**Table1. Sequence information**

| **Product s No.** | **Sequence Fragment Names** | **Amino Acid Sequences** | **SEQ ID NO** | **Nucleic Acid Sequences** | **SEQ ID NO** |
|---|---|---|---|---|---|
| XMDC 025 | CR2(SCR1 -SCR4) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VC | 1 | tgcggctctcctccacccattctcaatggaaggatctcctactaca gcacccccatagctgtcggcaccgttatccgatacagttgttccg gtactttccggcttatcggcgaaaagtctttgctgtgcattaccaag gataaagtggacgggacttgggacaaacccgcacctaagtgcg agtattttaacaaatatagcagctgccctgagcctatagtacccgg ggggtataaaatccggggctctactccctatcgtcatggcgattct gtgaccttcgcatgtaaaactaatttttcaatgaatggcaacaagtc tgtatggtgtcaagcaaataacatgtggggacctacccgcctgcc aacctgtgtgtcagtgtttcccctggaatgtccagccctccctatg atccacaacggacatcacaccagcgaaaacgttggctccatcgc accagggctctctgtgacttactcttgcgagtccgggtacctgctc gtgggtgaaaagatcatcaactgcctcagtagtggtaaatggtcc gccgtgcctcccacatgtgaagaggcccggtgcaagagcctgg gccggttccccaacggaaaagtgaaggaacctcctatcttgagg gttggtgtgaccgctaactttttctgcgacgaggggtacaggctc caagggcctccctctagtcggtgcgtaatcgccggtcaaggagt cgcatggactaagatgcctgtgtgt | 18 |
| | FH(SCR1-SCR4) | CNELPPRRNTEILTGSW SDQTYPEGTQAIYKCR PGYRSLGNVIMVCRKG EWVALNPLRKCQKRPC GHPGDTPFGTFTLTGG NVFEYGVKAVYTCNEG YQLLGEINYRECDTDG WTNDIPICEVVKCLPVT APENGKIVSSAMEPDR EYHFGQAVRFVCNSGY KIEGDEEMHCSDDGFW SKEKPKCVEISCKSPDV INGSPISQKIIYKENERF QYKCNMGYEYSERGD AVCTESGWRPLPSC | 2 | tgcaacgaactgcctcctagaagaaacaccgaaatcctcacagg ctcctggtcagatcagacctaccctgagggtacacaggccatct ataaatgccgcccgggttaccgctccttgggcaatgttattatggt ctgccggaaaggggaatgggtggccttgaaccccctcagaaag tgccaaaaaagaccctgcggacatccaggagacacacctttcg ggacatttaccttgaccggtggtaacgtgttcgagtacggagtga aggcagtctatacttgcaatgagggctaccagctcctgggggaa ataaattatcgcgaatgtgatacagacggctggacgaacgacatt cctatctgtgaggtggtcaaatgtctgcccgtcactgctcctgaaa acggcaagatagtgagttctgcaatggagccggatagggagtat cattttggtcaggccgtgcgctttgtgtgcaatagtgggtataaaat cgagggggatgaagaaatgcactgtagcgacgatggattctggt ctaaagagaaacctaagtgtgtggagattagttgcaagagtcca gacgtgattaacggtagccccatttcccaaaagataatatacaag gaaaacgaaagattccagtataaatgcaacatgggctacgagta tagcgagcggggcgatgccgtgtgtactgaaagcggctggcgc cccctgccttcctgc | 19 |
| | FH(SCR8) | CSKSSIDIENGFISESQY TYALKEKAKYQCKLG YVTADGETSGSITCGK DGWSAQPTC | 3 | tgttccaaaagcagcatcgacatcgaaaacggctttatatccgag agccagtatacgtatgcgctgaaggaaaaggccaagtatcagtg caagctcggttatgtcacagctgatggcgagacctccgggtctat cacatgcggcaaagacggctggtccgctcagcctacctgc | 20 |
| | FH(SCR19-SCR20) | CGPPPPIDNGDITSFPLS VYAPASSVEYQCQNLY QLEGNKRITCRNGQWS EPPKCLHPCVISREIME NYNIALRWTAKQKLYS RTGESVEFVCKRGYRL SSRSHTLRTTCWDGKL EYPTC | 4 | tgtgggccacctcccccaattgataatggtgatataacctccttcc ctcttagtgtttacgccccagcatccagcgtggagtatcaatgcca aaatctgtaccaactggaggggaacaaacggataacctgccgc aacggccaatggtccgagccacctaagtgcctgcatccatgtgt gatcagtcgggagatcatggagaactacaacattgccctgaggt ggactgctaagcagaagctctatagccgaacaggcgagtcagt ggagtttgtgtgtaaacggggatacaggctgtccagtaggagcc acaccctgcgcactacttgctgggatgggaagctggaatatcca acttgc | 21 |
| | CR2(SCR1 SCR4)+(G₄ S)₂+linker+ FH(SCR1-SCR4)+link er+FH(SCR 8)+linker+F H(SCR19-SCR20) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VCGGGGSGGGGSEDC NELPPRRNTEILTGSWS DQTYPEGTQAIYKCRP GYRSLGNVIMVCRKGE WVALNPLRKCQKRPCG HPGDTPFGTFTLTGGN VFEYGVKAVYTCNEGY QLLGEINYRECDTDGW TNDIPICEVVKCLPVTA PENGKIVSSAMEPDRE YHFGQAVRFVCNSGYK IEGDEEMHCSDDGFWS KEKPKCVEISCKSPDVI NGSPISQKIIYKENERFQ YKCNMGYEYSERGDA VCTESGWRPLPSCEIRV KTCSKSSIDIENGFISES QYTYALKEKAKYQCK LGYVTADGETSGSITCG KDGWSAQPTCIKSDST GKCGPPPPIDNGDITSFP LSVYAPASSVEYQCQN LYQLEGNKRITCRNGQ WSEPPKCLHPCVISREI MENYNIALRWTAKQKL YSRTGESVEFVCKRGY RLSSRSHTLRTTCWDG KLEYPTC | 5 | tgcggctctcctccacccattctcaatggaaggatctcctactaca gcacccccatagctgtcggcaccgttatccgatacagttgttccg gtactttccggcttatcggcgaaaagtctttgctgtgcattaccaag gataaagtggacgggacttgggacaaacccgcacctaagtgcg agtattttaacaaatatagcagctgccctgagcctatagtacccgg ggggtataaaatccggggctctactccctatcgtcatggcgattct gtgaccttcgcatgtaaaactaatttttcaatgaatggcaacaagtc tgtatggtgtcaagcaaataacatgtggggacctacccgcctgcc aacctgtgtgtcagtgtttcccctggaatgtccagccctccctatg atccacaacggacatcacaccagcgaaaacgttggctccatcgc accagggctctctgtgacttactcttgcgagtccgggtacctgctc gtgggtgaaaagatcatcaactgcctcagtagtggtaaatggtcc gccgtgcctcccacatgtgaagaggcccggtgcaagagcctgg gccggttccccaacggaaaagtgaaggaacctcctatcttgagg gttggtgtgaccgctaactttttctgcgacgaggggtacaggctc caagggcctccctctagtcggtgcgtaatcgccggtcaaggagt cgcatggactaagatgcctgtgtgtggcggaggtgggtcgggt ggcggcggatctgaggactgcaacgaactgcctcctagaagaa acaccgaaatcctcacaggctcctggtcagatcagacctaccct gagggtacacaggccatctataaatgccgcccgggttaccgctc cttgggcaatgttattatggtctgccggaaaggggaatgggtggc cttgaaccccctcagaaagtgccaaaaaagaccctgcggacatc caggagacacacctttcgggacatttaccttgaccggtggtaacg tgttcgagtacggagtgaaggcagtctatacttgcaatgagggct accagctcctgggggaaataaattatcgcgaatgtgatacagac ggctggacgaacgacattcctatctgtgaggtggtcaaatgtctg cccgtcactgctcctgaaaacggcaagatagtgagttctgcaatg gagccggatagggagtatcattttggtcaggccgtgcgctttgtg tgcaatagtgggtataaaatcgagggggatgaagaaatgcactg tagcgacgatggattctggtctaaagagaaacctaagtgtgtgga gattagttgcaagagtccagacgtgattaacggtagccccatttc ccaaaagataatatacaaggaaaacgaaagattccagtataaat gcaacatgggctacgagtatagcgagcggggcgatgccgtgtg tactgaaagcggctggcgccccctgccttcctgcgagattaggg tgaagacctgttccaaaagcagcatcgacatcgaaaacggcttta tatccgagagccagtatacgtatgcgctgaaggaaaaggccaa gtatcagtgcaagctcggttatgtcacagctgatggcgagacctc cgggtctatcacatgcggcaaagacggctggtccgctcagccta cctgcattaagagcgatagtacaggcaaatgtgggccacctccc ccaattgataatggtgatataacctccttccctcttagtgtttacgcc ccagcatccagcgtggagtatcaatgccaaaatctgtaccaactg gaggggaacaaacggataacctgccgcaacggccaatggtcc gagccacctaagtgcctgcatccatgtgtgatcagtcgggagat catggagaactacaacattgccctgaggtggactgctaagcaga agctctatagccgaacaggcgagtcagtggagtttgtgtgtaaac ggggatacaggctgtccagtaggagccacaccctgcgcactac ttgctgggatgggaagctggaatatccaacttgc | 22 |
| | CD5-sp+linker+ CR2(SCR1 SCR4)+(G₄ S)₂+linker+ FH(SCR1-SCR4)+link er+FH(SCR 8)+linker+F H(SCR19-SCR20)-+linker | MPMGSLQPLATLYLLG MLVASCLGISCGSPPPIL NGRISYYSTPIAVGTVIR YSCSGTFRLIGEKSLLCI TKDKVDGTWDKPAPK CEYFNKYSSCPEPIVPG GYKIRGSTPYRHGDSV TFACKTNFSMNGNKSV WCQANNMWGPTRLPT CVSVFPLECPALPMIHN GHHTSENVGSIAPGLSV TYSCESGYLLVGEKIIN CLSSGKWSAVPPTCEE ARCKSLGRFPNGKVKE PPILRVGVTANFFCDEG YRLQGPPSSRCVIAGQ GVAWTKMPVCGGGGS GGGGSEDCNELPPRRN TEILTGSWSDQTYPEGT QAIYKCRPGYRSLGNVI MVCRKGEWVALNPLR KCQKRPCGHPGDTPFG TFTLTGGNVFEYGVKA VYTCNEGYQLLGEINY RECDTDGWTNDIPICE VVKCLPVTAPENGKIVS SAMEPDREYHFGQAVR FVCNSGYKIEGDEEMH CSDDGFWSKEKPKCVE ISCKSPDVINGSPISQKII YKENERFQYKCNMGY EYSERGDAVCTESGWR PLPSCEIRVKTCSKSSID IENGFISESQYTYALKE KAKYQCKLGYVTADG ETSGSITCGKDGWSAQ PTCIKSDSTGKCGPPPPI DNGDITSFPLSVYAPAS SVEYQCQNLYQLEGNK RITCRNGQWSEPPKCL HPCVISREIMENYNIAL RWTAKQKLYSRTGESV EFVCKRGYRLSSRSHT LRTTCWDGKLEYPTCA KR | 6 | atgccaatggggtccctacaacccctggcaacactatatctgcta ggtatgctcgtggcctcctgtttaggaatttcatgcggctctcctcc acccattctcaatggaaggatctcctactacagcacccccatagc tgtcggcaccgttatccgatacagttgttccggtactttccggctta tcggcgaaaagtctttgctgtgcattaccaaggataaagtggacg ggacttgggacaaacccgcacctaagtgcgagtattttaacaaat atagcagctgccctgagcctatagtacccggggggtataaaatc cggggctctactccctatcgtcatggcgattctgtgaccttcgcat gtaaaactaatttttcaatgaatggcaacaagtctgtatggtgtcaa gcaaataacatgtggggacctacccgcctgccaacctgtgtgtc agtgtttcccctggaatgtccagccctccctatgatccacaacgg acatcacaccagcgaaaacgttggctccatcgcaccagggctct ctgtgacttactcttgcgagtccgggtacctgctcgtgggtgaaa agatcatcaactgcctcagtagtggtaaatggtccgccgtgcctc ccacatgtgaagaggcccggtgcaagagcctgggccggttccc caacggaaaagtgaaggaacctcctatcttgagggttggtgtga ccgctaactttttctgcgacgaggggtacaggctccaagggcct ccctctagtcggtgcgtaatcgccggtcaaggagtcgcatggac taagatgcctgtgtgtggcggaggtgggtcgggtggcggcgga tctgaggactgcaacgaactgcctcctagaagaaacaccgaaat cctcacaggctcctggtcagatcagacctaccctgagggtacac aggccatctataaatgccgcccgggttaccgctccttgggcaatg ttattatggtctgccggaaaggggaatgggtggccttgaaccccc tcagaaagtgccaaaaaagaccctgcggacatccaggagacac acctttcgggacatttaccttgaccggtggtaacgtgttcgagtac ggagtgaaggcagtctatacttgcaatgagggctaccagctcct gggggaaataaattatcgcgaatgtgatacagacggctggacg aacgacattcctatctgtgaggtggtcaaatgtctgcccgtcactg ctcctgaaaacggcaagatagtgagttctgcaatggagccggat agggagtatcattttggtcaggccgtgcgctttgtgtgcaatagtg ggtataaaatcgagggggatgaagaaatgcactgtagcgacga tggattctggtctaaagagaaacctaagtgtgtggagattagttgc aagagtccagacgtgattaacggtagccccatttcccaaaagata atatacaaggaaaacgaaagattccagtataaatgcaacatggg ctacgagtatagcgagcggggcgatgccgtgtgtactgaaagc ggctggcgccccctgccttcctgcgagattagggtgaagacctg ttccaaaagcagcatcgacatcgaaaacggctttatatccgagag ccagtatacgtatgcgctgaaggaaaaggccaagtatcagtgca agctcggttatgtcacagctgatggcgagacctccgggtctatca catgcggcaaagacggctggtccgctcagcctacctgcattaag agcgatagtacaggcaaatgtgggccacctcccccaattgataa tggtgatataacctccttccctcttagtgtttacgccccagcatcca gcgtggagtatcaatgccaaaatctgtaccaactggaggggaac aaacggataacctgccgcaacggccaatggtccgagccaccta agtgcctgcatccatgtgtgatcagtcgggagatcatggagaact acaacattgccctgaggtggactgctaagcagaagctctatagc cgaacaggcgagtcagtggagtttgtgtgtaaacggggatacag gctgtccagtaggagccacaccctgcgcactacttgctgggatg ggaagctggaatatccaacttgcgccaagagg | 23 |
| XMDC 026 | CR2(SCR1 -SCR4) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VC | 1 | tgcggctctcctccacccattctcaatggaaggatctcctactaca gcacccccatagctgtcggcaccgttatccgatacagttgttccg gtactttccggcttatcggcgaaaagtctttgctgtgcattaccaag gataaagtggacgggacttgggacaaacccgcacctaagtgcg agtattttaacaaatatagcagctgccctgagcctatagtacccgg ggggtataaaatccggggctctactccctatcgtcatggcgattct gtgaccttcgcatgtaaaactaatttttcaatgaatggcaacaagtc tgtatggtgtcaagcaaataacatgtggggacctacccgcctgcc aacctgtgtgtcagtgtttcccctggaatgtccagccctccctatg atccacaacggacatcacaccagcgaaaacgttggctccatcgc accagggctctctgtgacttactcttgcgagtccgggtacctgctc gtgggtgaaaagatcatcaactgcctcagtagtggtaaatggtcc gccgtgcctcccacatgtgaagaggcccggtgcaagagcctgg gccggttccccaacggaaaagtgaaggaacctcctatcttgagg gttggtgtgaccgctaactttttctgcgacgaggggtacaggctc caagggcctccctctagtcggtgcgtaatcgccggtcaaggagt cgcatggactaagatgcctgtgtgt | 18 |
| | FH(SCR1-SCR4) | CNELPPRRNTEILTGSW SDQTYPEGTQAIYKCR PGYRSLGNVIMVCRKG EWVALNPLRKCQKRPC GHPGDTPFGTFTLTGG | 2 | tgcaacgaactgcctcctagaagaaacaccgaaatcctcacagg ctcctggtcagatcagacctaccctgagggtacacaggccatct ataaatgccgcccgggttaccgctccttgggcaatgttattatggt ctgccggaaaggggaatgggtggccttgaaccccctcagaaag tgccaaaaaagaccctgcggacatccaggagacacacctttcg | 19 |
| | | NVFEYGVKAVYTCNEG YQLLGEINYRECDTDG WTNDIPICEVVKCLPVT APENGKIVSSAMEPDR EYHFGQAVRFVCNSGY KIEGDEEMHCSDDGFW SKEKPKCVEISCKSPDV INGSPISQKIIYKENERF QYKCNMGYEYSERGD AVCTESGWRPLPSC | | ggacatttaccttgaccggtggtaacgtgttcgagtacggagtga aggcagtctatacttgcaatgagggctaccagctcctgggggaa ataaattatcgcgaatgtgatacagacggctggacgaacgacatt cctatctgtgaggtggtcaaatgtctgcccgtcactgctcctgaaa acggcaagatagtgagttctgcaatggagccggatagggagtat cattttggtcaggccgtgcgctttgtgtgcaatagtgggtataaaat cgagggggatgaagaaatgcactgtagcgacgatggattctggt ctaaagagaaacctaagtgtgtggagattagttgcaagagtcca gacgtgattaacggtagccccatttcccaaaagataatatacaag gaaaacgaaagattccagtataaatgcaacatgggctacgagta tagcgagcggggcgatgccgtgtgtactgaaagcggctggcgc cccctgccttcctgc | |
| | FH(SCR18-SCR20) | CVNPPTVQNAYIVSRQ MSKYPSGERVRYQCRS PYEMFGDEEVMCLNG NWTEPPQCKDSTGKCG PPPPIDNGDITSFPLSVY APASSVEYQCQNLYQL EGNKRITCRNGQWSEP PKCLHPCVISREIMENY NIALRWTAKQKLYSRT GESVEFVCKRGYRLSS RSHTLRTTCWDGKLEY PTC | 7 | tgcgtgaaccctccaacagtccaaaatgcctatatagtctctagg cagatgagtaaatacccaagtggagagagagtgagataccagt gccggtctccttacgagatgttcggcgacgaggaagtgatgtgc ctgaacggcaattggacagagccaccacaatgtaaggatagtac aggcaaatgtgggccacctcccccaattgataatggtgatataac ctccttccctcttagtgtttacgccccagcatccagcgtggagtat caatgccaaaatctgtaccaactggaggggaacaaacggataa cctgccgcaacggccaatggtccgagccacctaagtgcctgcat ccatgtgtgatcagtcgggagatcatggagaactacaacattgcc ctgaggtggactgctaagcagaagctctatagccgaacaggcg agtcagtggagtttgtgtgtaaacggggatacaggctgtccagta ggagccacaccctgcgcactacttgctgggatgggaagctgga atatccaacttgc | 24 |
| | CR2(SCR1 SCR4)+(G₄ S)₂+linker+ FH(SCR1-SCR4)+link er+FH(SCR 18-SCR20) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VCGGGGSGGGGSEDC NELPPRRNTEILTGSWS DQTYPEGTQAIYKCRP GYRSLGNVIMVCRKGE WVALNPLRKCQKRPCG HPGDTPFGTFTLTGGN VFEYGVKAVYTCNEGY QLLGEINYRECDTDGW TNDIPICEVVKCLPVTA PENGKIVSSAMEPDRE YHFGQAVRFVCNSGYK IEGDEEMHCSDDGFWS KEKPKCVEISCKSPDVI NGSPISQKIIYKENERFQ YKCNMGYEYSERGDA VCTESGWRPLPSCERD TSCVNPPTVQNAYIVSR QMSKYPSGERVRYQCR SPYEMFGDEEVMCLNG NWTEPPQCKDSTGKCG | 8 | tgcggctctcctccacccattctcaatggaaggatctcctactaca gcacccccatagctgtcggcaccgttatccgatacagttgttccg gtactttccggcttatcggcgaaaagtctttgctgtgcattaccaag gataaagtggacgggacttgggacaaacccgcacctaagtgcg agtattttaacaaatatagcagctgccctgagcctatagtacccgg ggggtataaaatccggggctctactccctatcgtcatggcgattct gtgaccttcgcatgtaaaactaatttttcaatgaatggcaacaagtc tgtatggtgtcaagcaaataacatgtggggacctacccgcctgcc aacctgtgtgtcagtgtttcccctggaatgtccagccctccctatg atccacaacggacatcacaccagcgaaaacgttggctccatcgc accagggctctctgtgacttactcttgcgagtccgggtacctgctc gtgggtgaaaagatcatcaactgcctcagtagtggtaaatggtcc gccgtgcctcccacatgtgaagaggcccggtgcaagagcctgg gccggttccccaacggaaaagtgaaggaacctcctatcttgagg gttggtgtgaccgctaactttttctgcgacgaggggtacaggctc caagggcctccctctagtcggtgcgtaatcgccggtcaaggagt cgcatggactaagatgcctgtgtgtggcggaggtgggtcgggt ggcggcggatctgaggactgcaacgaactgcctcctagaagaa acaccgaaatcctcacaggctcctggtcagatcagacctaccct gagggtacacaggccatctataaatgccgcccgggttaccgctc cttgggcaatgttattatggtctgccggaaaggggaatgggtggc cttgaaccccctcagaaagtgccaaaaaagaccctgcggacatc caggagacacacctttcgggacatttaccttgaccggtggtaacg tgttcgagtacggagtgaaggcagtctatacttgcaatgagggct accagctcctgggggaaataaattatcgcgaatgtgatacagac ggctggacgaacgacattcctatctgtgaggtggtcaaatgtctg cccgtcactgctcctgaaaacggcaagatagtgagttctgcaatg gagccggatagggagtatcattttggtcaggccgtgcgctttgtg tgcaatagtgggtataaaatcgagggggatgaagaaatgcactg tagcgacgatggattctggtctaaagagaaacctaagtgtgtgga gattagttgcaagagtccagacgtgattaacggtagccccatttc ccaaaagataatatacaaggaaaacgaaagattccagtataaat gcaacatgggctacgagtatagcgagcggggcgatgccgtgtg tactgaaagcggctggcgccccctgccttcctgcgagcgggata caagctgcgtgaaccctccaacagtccaaaatgcctatatagtct | 25 |
| | | PPPPIDNGDITSFPLSVY APASSVEYQCQNLYQL EGNKRITCRNGQWSEP PKCLHPCVISREIMENY NIALRWTAKQKLYSRT GESVEFVCKRGYRLSS RSHTLRTTCWDGKLEY PTC | | ctaggcagatgagtaaatacccaagtggagagagagtgagata ccagtgccggtctccttacgagatgttcggcgacgaggaagtga tgtgcctgaacggcaattggacagagccaccacaatgtaaggat agtacaggcaaatgtgggccacctcccccaattgataatggtgat ataacctccttccctcttagtgtttacgccccagcatccagcgtgg agtatcaatgccaaaatctgtaccaactggaggggaacaaacgg ataacctgccgcaacggccaatggtccgagccacctaagtgcct gcatccatgtgtgatcagtcgggagatcatggagaactacaacat tgccctgaggtggactgctaagcagaagctctatagccgaacag gcgagtcagtggagtttgtgtgtaaacggggatacaggctgtcc agtaggagccacaccctgcgcactacttgctgggatgggaagct ggaatatccaacttgc | |
| | CD5-sp+linker+ CR2(SCR1 SCR4)+(G₄ S)₂+linker+ FH(SCR1-SCR4)+link er+FH(SCR 18-SCR20)+lin ker | MPMGSLQPLATLYLLG MLVASCLGISCGSPPPIL NGRISYYSTPIAVGTVIR YSCSGTFRLIGEKSLLCI TKDKVDGTWDKPAPK CEYFNKYSSCPEPIVPG GYKIRGSTPYRHGDSV TFACKTNFSMNGNKSV WCQANNMWGPTRLPT CVSVFPLECPALPMIHN GHHTSENVGSIAPGLSV TYSCESGYLLVGEKIIN CLSSGKWSAVPPTCEE ARCKSLGRFPNGKVKE PPILRVGVTANFFCDEG YRLQGPPSSRCVIAGQ GVAWTKMPVCGGGGS GGGGSEDCNELPPRRN TEILTGSWSDQTYPEGT QAIYKCRPGYRSLGNVI MVCRKGEWVALNPLR KCQKRPCGHPGDTPFG TFTLTGGNVFEYGVKA VYTCNEGYQLLGEINY RECDTDGWTNDIPICE VVKCLPVTAPENGKIVS SAMEPDREYHFGQAVR FVCNSGYKIEGDEEMH CSDDGFWSKEKPKCVE ISCKSPDVINGSPISQKII YKENERFQYKCNMGY EYSERGDAVCTESGWR PLPSCERDTSCVNPPTV QNAYIVSRQMSKYPSG ERVRYQCRSPYEMFGD EEVMCLNGNWTEPPQ CKDSTGKCGPPPPIDNG DITSFPLSVYAPASSVEY QCQNLYQLEGNKRITC RNGQWSEPPKCLHPCV ISREIMENYNIALRWTA KQKLYSRTGESVEFVC KRGYRLSSRSHTLRTTC WDGKLEYPTCAKR | 9 | atgccaatggggtccctacaacccctggcaacactatatctgcta ggtatgctcgtggcctcctgtttaggaatttcatgcggctctcctcc acccattctcaatggaaggatctcctactacagcacccccatagc tgtcggcaccgttatccgatacagttgttccggtactttccggctta tcggcgaaaagtctttgctgtgcattaccaaggataaagtggacg ggacttgggacaaacccgcacctaagtgcgagtattttaacaaat atagcagctgccctgagcctatagtacccggggggtataaaatc cggggctctactccctatcgtcatggcgattctgtgaccttcgcat gtaaaactaatttttcaatgaatggcaacaagtctgtatggtgtcaa gcaaataacatgtggggacctacccgcctgccaacctgtgtgtc agtgtttcccctggaatgtccagccctccctatgatccacaacgg acatcacaccagcgaaaacgttggctccatcgcaccagggctct ctgtgacttactcttgcgagtccgggtacctgctcgtgggtgaaa agatcatcaactgcctcagtagtggtaaatggtccgccgtgcctc ccacatgtgaagaggcccggtgcaagagcctgggccggttccc caacggaaaagtgaaggaacctcctatcttgagggttggtgtga ccgctaactttttctgcgacgaggggtacaggctccaagggcct ccctctagtcggtgcgtaatcgccggtcaaggagtcgcatggac taagatgcctgtgtgtggcggaggtgggtcgggtggcggcgga tctgaggactgcaacgaactgcctcctagaagaaacaccgaaat cctcacaggctcctggtcagatcagacctaccctgagggtacac aggccatctataaatgccgcccgggttaccgctccttgggcaatg ttattatggtctgccggaaaggggaatgggtggccttgaaccccc tcagaaagtgccaaaaaagaccctgcggacatccaggagacac acctttcgggacatttaccttgaccggtggtaacgtgttcgagtac ggagtgaaggcagtctatacttgcaatgagggctaccagctcct gggggaaataaattatcgcgaatgtgatacagacggctggacg aacgacattcctatctgtgaggtggtcaaatgtctgcccgtcactg ctcctgaaaacggcaagatagtgagttctgcaatggagccggat agggagtatcattttggtcaggccgtgcgctttgtgtgcaatagtg ggtataaaatcgagggggatgaagaaatgcactgtagcgacga tggattctggtctaaagagaaacctaagtgtgtggagattagttgc aagagtccagacgtgattaacggtagccccatttcccaaaagata atatacaaggaaaacgaaagattccagtataaatgcaacatggg ctacgagtatagcgagcggggcgatgccgtgtgtactgaaagc ggctggcgccccctgccttcctgcgagcgggatacaagctgcg tgaaccctccaacagtccaaaatgcctatatagtctctaggcagat gagtaaatacccaagtggagagagagtgagataccagtgccgg tctccttacgagatgttcggcgacgaggaagtgatgtgcctgaac ggcaattggacagagccaccacaatgtaaggatagtacaggca aatgtgggccacctcccccaattgataatggtgatataacctcctt ccctcttagtgtttacgccccagcatccagcgtggagtatcaatgc caaaatctgtaccaactggaggggaacaaacggataacctgcc gcaacggccaatggtccgagccacctaagtgcctgcatccatgt gtgatcagtcgggagatcatggagaactacaacattgccctgag gtggactgctaagcagaagctctatagccgaacaggcgagtca gtggagtttgtgtgtaaacggggatacaggctgtccagtaggag ccacaccctgcgcactacttgctgggatgggaagctggaatatc | 26 |
| | | | | caacttgcgccaagagg | |
| | CR2(SCR1 -SCR4) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VC | 1 | tgcggcagcccccctcccatcctgaacggcagaatcagctacta cagcacccccatcgccgtgggcaccgtgatcagatacagctgc agcggcaccttcagactgatcggcgagaagagcctgctgtgcat caccaaggacaaggtggacggcacctgggacaagcccgccc ccaagtgcgagtacttcaacaagtacagcagctgccccgagcc catcgtgcctggcggctacaagatcagaggcagcaccccctac agacacggcgacagcgtgaccttcgcctgcaagaccaacttca gcatgaacggcaacaagagcgtgtggtgccaagccaacaacat gtggggccccacaagactgcccacctgcgtgagcgtgttcccc ctggagtgccccgccctgcccatgatccacaacggccaccaca caagcgagaacgtgggcagcatcgcccccggcctgagcgtga cctacagctgcgagagcggctacctgctggtgggcgagaagat catcaactgcctgagcagcggcaagtggagcgccgtgcccccc acctgcgaggaggctagatgcaagagcctgggcagattcccca acggcaaggtgaaggagcctcctatcctgagagtgggcgtgac cgccaacttcttctgcgacgagggctacagactgcaaggccccc ctagcagcagatgtgtgatcgctggccaaggcgtggcctggac caaaatgcccgtctgc | 27 |
| XMDC 029 | FH(SCR1-SCR4) | CNELPPRRNTEILTGSW SDQTYPEGTQAIYKCR PGYRSLGNVIMVCRKG EWVALNPLRKCQKRPC GHPGDTPFGTFTLTGG NVFEYGVKAVYTCNEG YQLLGEINYRECDTDG WTNDIPICEVVKCLPVT APENGKIVSSAMEPDR EYHFGQAVRFVCNSGY KIEGDEEMHCSDDGFW SKEKPKCVEISCKSPDV INGSPISQKIIYKENERF QYKCNMGYEYSERGD AVCTESGWRPLPSC | 2 | tgcaacgagctgccccctagacggaacacagagatcctgaccg gcagctggagcgatcaaacatatcccgagggcacccaagccat ctacaaatgcagacctggctacagaagcctgggcaatgtgatca tggtctgccggaaaggggagtgggtcgctctgaaccccctgcg gaagtgtcagaagagaccttgcggccaccccggcgacacacc cttcgggaccttcacactgaccggcggcaacgtgtttgagtacg gcgtcaaggccgtgtacacctgtaacgaggggtatcagctgctg ggcgagatcaactacagagagtgcgataccgacggctggacc aatgacatccccatctgcgaggtggtgaagtgtctgcccgtgac cgcccccgaaaacggcaagatcgtgagcagcgccatggagcc cgaccgggagtaccattttgggcaagctgtgagattcgtctgcaa tagcggctacaagatcgaaggcgacgaggagatgcactgtagc gacgacggcttctggtccaaagagaaacccaaatgcgtggaga tctcctgcaaaagccccgatgtgatcaacggcagccccatctcc caaaaaattatctacaaggaaaacgaacggtttcagtacaaatgc aacatgggctacgaatactccgagcggggggatgccgtgtgca ccgagagcggctggcggcctctgcctagctgt | 28 |
| | | | | tgcaatgaactgcctcctagacggaacaccgaaatcctgaccgg gagctggagcgaccaaacataccccgaggggacacaagccat ctacaagtgcagacctgggtatagatccctcgggaacgtgatcat ggtctgtcggaagggcgagtgggtcgctctgaaccctctcagaa agtgtcagaaacggccctgcgggcaccccggcgataccccctt cggcacattcaccctgaccggggggaacgtgttcgagtacggc gtgaaggccgtctacacctgcaacgagggctaccaactcctgg gcgaaatcaattacagagaatgcgacacagatggctggaccaa cgacatccctatctgcgaggtcgtgaaatgtctccccgtgacagc ccctgagaacggcaagattgtcagcagcgctatggaacctgata gagagtaccatttcggccaagccgtcagatttgtgtgcaattccg gctacaaaattgagggggacgaagagatgcactgctccgacga tggcttctggagcaaggaaaaacccaagtgcgtggagattagct gcaagagccccgacgtgatcaacgggagccccatctcccaaaa aatcatctacaaggagaacgagagatttcagtataagtgcaacat gggctatgagtacagcgagcggggggacgccgtgtgtaccga atccgggtggagacccctgcctagctgc | 29 |
| | CR2(SCR1 SCR4)+(G ₄S)₂+linker +FH(SCR1 - SCR4)+lin ker+(G₄S)₂ +linker+F H(SCR1-SCR4) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS | 10 | tgcggcagcccccctcccatcctgaacggcagaatcagctacta cagcacccccatcgccgtgggcaccgtgatcagatacagctgc agcggcaccttcagactgatcggcgagaagagcctgctgtgcat caccaaggacaaggtggacggcacctgggacaagcccgccc ccaagtgcgagtacttcaacaagtacagcagctgccccgagcc catcgtgcctggcggctacaagatcagaggcagcaccccctac | 30 |
| | | MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VCGGGGSGGGGSEDC NELPPRRNTEILTGSWS DQTYPEGTQAIYKCRP GYRSLGNVIMVCRKGE WVALNPLRKCQKRPCG HPGDTPFGTFTLTGGN VFEYGVKAVYTCNEGY QLLGEINYRECDTDGW TNDIPICEVVKCLPVTA PENGKIVSSAMEPDRE YHFGQAVRFVCNSGYK IEGDEEMHCSDDGFWS KEKPKCVEISCKSPDVI NGSPISQKIIYKENERFQ YKCNMGYEYSERGDA VCTESGWRPLPSCEGG GGSGGGGSEDCNELPP RRNTEILTGSWSDQTYP EGTQAIYKCRPGYRSL GNVIMVCRKGEWVAL NPLRKCQKRPCGHPGD TPFGTFTLTGGNVFEYG VKAVYTCNEGYQLLGE INYRECDTDGWTNDIPI CEVVKCLPVTAPENGKI VSSAMEPDREYHFGQA VRFVCNSGYKIEGDEE MHCSDDGFWSKEKPK CVEISCKSPDVINGSPIS QKIIYKENERFQYKCN MGYEYSERGDAVCTES GWRPLPSC | | agacacggcgacagcgtgaccttcgcctgcaagaccaacttca gcatgaacggcaacaagagcgtgtggtgccaagccaacaacat gtggggccccacaagactgcccacctgcgtgagcgtgttcccc ctggagtgccccgccctgcccatgatccacaacggccaccaca caagcgagaacgtgggcagcatcgcccccggcctgagcgtga cctacagctgcgagagcggctacctgctggtgggcgagaagat catcaactgcctgagcagcggcaagtggagcgccgtgcccccc acctgcgaggaggctagatgcaagagcctgggcagattcccca acggcaaggtgaaggagcctcctatcctgagagtgggcgtgac cgccaacttcttctgcgacgagggctacagactgcaaggccccc ctagcagcagatgtgtgatcgctggccaaggcgtggcctggac caaaatgcccgtctgcgggggggggggcagcgggggcggg ggcagcgaggattgcaacgagctgccccctagacggaacaca gagatcctgaccggcagctggagcgatcaaacatatcccgagg gcacccaagccatctacaaatgcagacctggctacagaagcct gggcaatgtgatcatggtctgccggaaaggggagtgggtcgct ctgaaccccctgcggaagtgtcagaagagaccttgcggccacc ccggcgacacacccttcgggaccttcacactgaccggcggcaa cgtgtttgagtacggcgtcaaggccgtgtacacctgtaacgagg ggtatcagctgctgggcgagatcaactacagagagtgcgatacc gacggctggaccaatgacatccccatctgcgaggtggtgaagt gtctgcccgtgaccgcccccgaaaacggcaagatcgtgagcag cgccatggagcccgaccgggagtaccattttgggcaagctgtg agattcgtctgcaatagcggctacaagatcgaaggcgacgagg agatgcactgtagcgacgacggcttctggtccaaagagaaacc caaatgcgtggagatctcctgcaaaagccccgatgtgatcaacg gcagccccatctcccaaaaaattatctacaaggaaaacgaacgg tttcagtacaaatgcaacatgggctacgaatactccgagcgggg ggatgccgtgtgcaccgagagcggctggcggcctctgcctagc tgtgaagggggcggcgggagcggcgggggcggcagcgagg actgcaatgaactgcctcctagacggaacaccgaaatcctgacc gggagctggagcgaccaaacataccccgaggggacacaagc catctacaagtgcagacctgggtatagatccctcgggaacgtgat catggtctgtcggaagggcgagtgggtcgctctgaaccctctca gaaagtgtcagaaacggccctgcgggcaccccggcgataccc ccttcggcacattcaccctgaccggggggaacgtgttcgagtac ggcgtgaaggccgtctacacctgcaacgagggctaccaactcc tgggcgaaatcaattacagagaatgcgacacagatggctggac caacgacatccctatctgcgaggtcgtgaaatgtctccccgtgac agcccctgagaacggcaagattgtcagcagcgctatggaacct gatagagagtaccatttcggccaagccgtcagatttgtgtgcaatt ccggctacaaaattgagggggacgaagagatgcactgctccga cgatggcttctggagcaaggaaaaacccaagtgcgtggagatta gctgcaagagccccgacgtgatcaacgggagccccatctccca aaaaatcatctacaaggagaacgagagatttcagtataagtgcaa catgggctatgagtacagcgagcggggggacgccgtgtgtacc gaatccgggtggagacccctgcctagctgc | |
| | CD5-sp+linker+ CR2(SCR1 SCR4)+(G ₄S)₂+linker +FH(SCR1 SCR4)+lin ker+(G₄S)₂ +linker+F H(SCR1-SCR4)+linker | MPMGSLQPLATLYLLG MLVASCLGISCGSPPPIL NGRISYYSTPIAVGTVIR YSCSGTFRLIGEKSLLCI TKDKVDGTWDKPAPK CEYFNKYSSCPEPIVPG GYKIRGSTPYRHGDSV TFACKTNFSMNGNKSV WCQANNMWGPTRLPT CVSVFPLECPALPMIHN GHHTSENVGSIAPGLSV TYSCESGYLLVGEKIIN CLSSGKWSAVPPTCEE | 11 | atgcccatgggcagcctgcagcccctggccaccctgtacctgct gggcatgctggtggctagctgcctgggcatcagctgcggcagc ccccctcccatcctgaacggcagaatcagctactacagcacccc catcgccgtgggcaccgtgatcagatacagctgcagcggcacc ttcagactgatcggcgagaagagcctgctgtgcatcaccaagga caaggtggacggcacctgggacaagcccgcccccaagtgcga gtacttcaacaagtacagcagctgccccgagcccatcgtgcctg gcggctacaagatcagaggcagcaccccctacagacacggcg acagcgtgaccttcgcctgcaagaccaacttcagcatgaacggc aacaagagcgtgtggtgccaagccaacaacatgtggggcccca caagactgcccacctgcgtgagcgtgttccccctggagtgcccc gccctgcccatgatccacaacggccaccacacaagcgagaac gtgggcagcatcgcccccggcctgagcgtgacctacagctgcg | 31 |
| | | ARCKSLGRFPNGKVKE PPILRVGVTANFFCDEG YRLQGPPSSRCVIAGQ GVAWTKMPVCGGGGS GGGGSEDCNELPPRRN TEILTGSWSDQTYPEGT QAIYKCRPGYRSLGNVI MVCRKGEWVALNPLR KCQKRPCGHPGDTPFG TFTLTGGNVFEYGVKA VYTCNEGYQLLGEINY RECDTDGWTNDIPICE VVKCLPVTAPENGKIVS SAMEPDREYHFGQAVR FVCNSGYKIEGDEEMH CSDDGFWSKEKPKCVE ISCKSPDVINGSPISQKII YKENERFQYKCNMGY EYSERGDAVCTESGWR PLPSCEGGGGSGGGGS EDCNELPPRRNTEILTG SWSDQTYPEGTQAIYK CRPGYRSLGNVIMVCR KGEWVALNPLRKCQK RPCGHPGDTPFGTFTLT GGNVFEYGVKAVYTC NEGYQLLGEINYRECD TDGWTNDIPICEVVKC LPVTAPENGKIVSSAME PDREYHFGQAVRFVCN SGYKIEGDEEMHCSDD GFWSKEKPKCVEISCK SPDVINGSPISQKIIYKE NERFQYKCNMGYEYS ERGDAVCTESGWRPLP SCE | | agagcggctacctgctggtgggcgagaagatcatcaactgcct gagcagcggcaagtggagcgccgtgccccccacctgcgagg aggctagatgcaagagcctgggcagattccccaacggcaaggt gaaggagcctcctatcctgagagtgggcgtgaccgccaacttct tctgcgacgagggctacagactgcaaggcccccctagcagcag atgtgtgatcgctggccaaggcgtggcctggaccaaaatgccc gtctgcgggggggggggcagcgggggcgggggcagcgagg attgcaacgagctgccccctagacggaacacagagatcctgac cggcagctggagcgatcaaacatatcccgagggcacccaagc catctacaaatgcagacctggctacagaagcctgggcaatgtga tcatggtctgccggaaaggggagtgggtcgctctgaaccccctg cggaagtgtcagaagagaccttgcggccaccccggcgacaca cccttcgggaccttcacactgaccggcggcaacgtgtttgagtac ggcgtcaaggccgtgtacacctgtaacgaggggtatcagctgct gggcgagatcaactacagagagtgcgataccgacggctggac caatgacatccccatctgcgaggtggtgaagtgtctgcccgtga ccgcccccgaaaacggcaagatcgtgagcagcgccatggagc ccgaccgggagtaccattttgggcaagctgtgagattcgtctgca atagcggctacaagatcgaaggcgacgaggagatgcactgtag cgacgacggcttctggtccaaagagaaacccaaatgcgtggag atctcctgcaaaagccccgatgtgatcaacggcagccccatctc ccaaaaaattatctacaaggaaaacgaacggtttcagtacaaatg caacatgggctacgaatactccgagcggggggatgccgtgtgc accgagagcggctggcggcctctgcctagctgtgaagggggc ggcgggagcggcgggggcggcagcgaggactgcaatgaact gcctcctagacggaacaccgaaatcctgaccgggagctggagc gaccaaacataccccgaggggacacaagccatctacaagtgca gacctgggtatagatccctcgggaacgtgatcatggtctgtcgga agggcgagtgggtcgctctgaaccctctcagaaagtgtcagaaa cggccctgcgggcaccccggcgatacccccttcggcacattca ccctgaccggggggaacgtgttcgagtacggcgtgaaggccgt ctacacctgcaacgagggctaccaactcctgggcgaaatcaatt acagagaatgcgacacagatggctggaccaacgacatccctat ctgcgaggtcgtgaaatgtctccccgtgacagcccctgagaacg gcaagattgtcagcagcgctatggaacctgatagagagtaccatt tcggccaagccgtcagatttgtgtgcaattccggctacaaaattg agggggacgaagagatgcactgctccgacgatggcttctggag caaggaaaaacccaagtgcgtggagattagctgcaagagcccc gacgtgatcaacgggagccccatctcccaaaaaatcatctacaa ggagaacgagagatttcagtataagtgcaacatgggctatgagt acagcgagcggggggacgccgtgtgtaccgaatccgggtgga gacccctgcctagctgcgag | |
| CR2-FH(1-4) | CR2(SCR1 -SCR4) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VC | 1 | tgcggctctcctccacccattctcaatggaaggatctcctactaca gcacccccatagctgtcggcaccgttatccgatacagttgttccg gtactttccggcttatcggcgaaaagtctttgctgtgcattaccaag gataaagtggacgggacttgggacaaacccgcacctaagtgcg agtattttaacaaatatagcagctgccctgagcctatagtacccgg ggggtataaaatccggggctctactccctatcgtcatggcgattct gtgaccttcgcatgtaaaactaatttttcaatgaatggcaacaagtc tgtatggtgtcaagcaaataacatgtggggacctacccgcctgcc aacctgtgtgtcagtgtttcccctggaatgtccagccctccctatg atccacaacggacatcacaccagcgaaaacgttggctccatcgc accagggctctctgtgacttactcttgcgagtccgggtacctgctc gtgggtgaaaagatcatcaactgcctcagtagtggtaaatggtcc gccgtgcctcccacatgtgaagaggcccggtgcaagagcctgg gccggttccccaacggaaaagtgaaggaacctcctatcttgagg gttggtgtgaccgctaactttttctgcgacgaggggtacaggctc caagggcctccctctagtcggtgcgtaatcgccggtcaaggagt cgcatggactaagatgcctgtgtgt | 18 |
| | FH(SCR1-SCR4) | CNELPPRRNTEILTGSW | 2 | tgtaatgagctgcccccaagacggaatacagagatcctcacagg | 19 |
| | | SDQTYPEGTQAIYKCR PGYRSLGNVIMVCRKG EWVALNPLRKCQKRPC GHPGDTPFGTFTLTGG NVFEYGVKAVYTCNEG YQLLGEINYRECDTDG WTNDIPICEVVKCLPVT APENGKIVSSAMEPDR EYHFGQAVRFVCNSGY KIEGDEEMHCSDDGFW SKEKPKCVEISCKSPDV INGSPISQKIIYKENERF QYKCNMGYEYSERGD AVCTESGWRPLPSC | | ctcttggtccgatcaaacttatccagagggtacccaggcaatttac aagtgcagacctggatacaggagcctgggcaatgtgattatggt gtgccgcaagggggagtgggtggcccttaatcctctccggaagt gtcagaaaagaccatgcggacaccctggagatacacctttcggt acctttacccttaccggcggcaatgtcttcgagtatggcgtcaag gccgtgtacacttgtaacgagggataccagctgctgggggaaat aaactatcgtgagtgtgacactgacgggtggactaacgacatcc ccatttgcgaggtggtcaagtgccttcctgtaaccgctcccgaaa atggtaagatcgtatcttccgcaatggagcctgatcgggaatacc actttggacaagccgttcggttcgtatgtaattcagggtataaaatt gagggcgatgaggagatgcactgcagtgatgacggcttttggtc aaaggaaaagccaaagtgcgtagagatcagttgtaagtctcctg acgttattaacgggagtcccatcagtcagaagatcatttacaagg aaaacgagaggttccagtataaatgcaatatgggatatgagtact ccgaaagaggggacgccgtgtgcacagagtccggatggcgac ctttgccatcttgt | |
| | CR2(SCR1 SCR4)+(G ₄S)₂+linker +FH(SCR1 -SCR4) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VCGGGGSGGGGSEDC NELPPRRNTEILTGSWS DQTYPEGTQAIYKCRP GYRSLGNVIMVCRKGE WVALNPLRKCQKRPCG HPGDTPFGTFTLTGGN VFEYGVKAVYTCNEGY QLLGEINYRECDTDGW TNDIPICEVVKCLPVTA PENGKIVSSAMEPDRE YHFGQAVRFVCNSGYK IEGDEEMHCSDDGFWS KEKPKCVEISCKSPDVI NGSPISQKIIYKENERFQ YKCNMGYEYSERGDA VCTESGWRPLPSC | 12 | tgcggctctcctccacccattctcaatggaaggatctcctactaca gcacccccatagctgtcggcaccgttatccgatacagttgttccg gtactttccggcttatcggcgaaaagtctttgctgtgcattaccaag gataaagtggacgggacttgggacaaacccgcacctaagtgcg agtattttaacaaatatagcagctgccctgagcctatagtacccgg ggggtataaaatccggggctctactccctatcgtcatggcgattct gtgaccttcgcatgtaaaactaatttttcaatgaatggcaacaagtc tgtatggtgtcaagcaaataacatgtggggacctacccgcctgcc aacctgtgtgtcagtgtttcccctggaatgtccagccctccctatg atccacaacggacatcacaccagcgaaaacgttggctccatcgc accagggctctctgtgacttactcttgcgagtccgggtacctgctc gtgggtgaaaagatcatcaactgcctcagtagtggtaaatggtcc gccgtgcctcccacatgtgaagaggcccggtgcaagagcctgg gccggttccccaacggaaaagtgaaggaacctcctatcttgagg gttggtgtgaccgctaactttttctgcgacgaggggtacaggctc caagggcctccctctagtcggtgcgtaatcgccggtcaaggagt cgcatggactaagatgcctgtgtgtggcggaggtgggtcgggt ggcggcggatctgaagactgtaatgagctgcccccaagacgga atacagagatcctcacaggctcttggtccgatcaaacttatccag agggtacccaggcaatttacaagtgcagacctggatacaggag cctgggcaatgtgattatggtgtgccgcaagggggagtgggtg gcccttaatcctctccggaagtgtcagaaaagaccatgcggaca ccctggagatacacctttcggtacctttacccttaccggcggcaat gtcttcgagtatggcgtcaaggccgtgtacacttgtaacgaggga taccagctgctgggggaaataaactatcgtgagtgtgacactga cgggtggactaacgacatccccatttgcgaggtggtcaagtgcc ttcctgtaaccgctcccgaaaatggtaagatcgtatcttccgcaat ggagcctgatcgggaataccactttggacaagccgttcggttcgt atgtaattcagggtataaaattgagggcgatgaggagatgcactg cagtgatgacggcttttggtcaaaggaaaagccaaagtgcgtag agatcagttgtaagtctcctgacgttattaacgggagtcccatcag tcagaagatcatttacaaggaaaacgagaggttccagtataaatg caatatgggatatgagtactccgaaagaggggacgccgtgtgc acagagtccggatggcgacctttgccatcttgt | 32 |
| | CD5-sp+linker+ CR2(SCR1 SCR4)+(G ₄S)₂+linker +FH(SCR1 - SCR4)+lin ker | MPMGSLQPLATLYLLG MLVASCLGISCGSPPPIL NGRISYYSTPIAVGTVIR YSCSGTFRLIGEKSLLCI TKDKVDGTWDKPAPK CEYFNKYSSCPEPIVPG GYKIRGSTPYRHGDSV TFACKTNFSMNGNKSV | 13 | atgccaatggggtccctacaacccctggcaacactatatctgcta ggtatgctcgtggcctcctgtttaggaatttcatgcggctctcctcc acccattctcaatggaaggatctcctactacagcacccccatagc tgtcggcaccgttatccgatacagttgttccggtactttccggctta tcggcgaaaagtctttgctgtgcattaccaaggataaagtggacg ggacttgggacaaacccgcacctaagtgcgagtattttaacaaat atagcagctgccctgagcctatagtacccggggggtataaaatc cggggctctactccctatcgtcatggcgattctgtgaccttcgcat | 33 |
| | | WCQANNMWGPTRLPT CVSVFPLECPALPMIHN GHHTSENVGSIAPGLSV TYSCESGYLLVGEKIIN CLSSGKWSAVPPTCEE ARCKSLGRFPNGKVKE PPILRVGVTANFFCDEG YRLQGPPSSRCVIAGQ GVAWTKMPVCGGGGS GGGGSEDCNELPPRRN TEILTGSWSDQTYPEGT QAIYKCRPGYRSLGNVI MVCRKGEWVALNPLR KCQKRPCGHPGDTPFG TFTLTGGNVFEYGVKA VYTCNEGYQLLGEINY RECDTDGWTNDIPICE VVKCLPVTAPENGKIVS SAMEPDREYHFGQAVR FVCNSGYKIEGDEEMH CSDDGFWSKEKPKCVE ISCKSPDVINGSPISQKII YKENERFQYKCNMGY EYSERGDAVCTESGWR PLPSCE | | gtaaaactaatttttcaatgaatggcaacaagtctgtatggtgtcaa gcaaataacatgtggggacctacccgcctgccaacctgtgtgtc agtgtttcccctggaatgtccagccctccctatgatccacaacgg acatcacaccagcgaaaacgttggctccatcgcaccagggctct ctgtgacttactcttgcgagtccgggtacctgctcgtgggtgaaa agatcatcaactgcctcagtagtggtaaatggtccgccgtgcctc ccacatgtgaagaggcccggtgcaagagcctgggccggttccc caacggaaaagtgaaggaacctcctatcttgagggttggtgtga ccgctaactttttctgcgacgaggggtacaggctccaagggcct ccctctagtcggtgcgtaatcgccggtcaaggagtcgcatggac taagatgcctgtgtgtggcggaggtgggtcgggtggcggcgga tctgaagactgtaatgagctgcccccaagacggaatacagagat cctcacaggctcttggtccgatcaaacttatccagagggtaccca ggcaatttacaagtgcagacctggatacaggagcctgggcaatg tgattatggtgtgccgcaagggggagtgggtggcccttaatcctc tccggaagtgtcagaaaagaccatgcggacaccctggagatac acctttcggtacctttacccttaccggcggcaatgtcttcgagtatg gcgtcaaggccgtgtacacttgtaacgagggataccagctgct gggggaaataaactatcgtgagtgtgacactgacgggtggactaa cgacatccccatttgcgaggtggtcaagtgccttcctgtaaccgc tcccgaaaatggtaagatcgtatcttccgcaatggagcctgatcg ggaataccactttggacaagccgttcggttcgtatgtaattcaggg tataaaattgagggcgatgaggagatgcactgcagtgatgacgg cttttggtcaaaggaaaagccaaagtgcgtagagatcagttgtaa gtctcctgacgttattaacgggagtcccatcagtcagaagatcatt tacaaggaaaacgagaggttccagtataaatgcaatatgggatat gagtactccgaaagaggggacgccgtgtgcacagagtccggat ggcgacctttgccatcttgtgaa | |
| CR2-FH(1-5) | CR2(SCR1 -SCR4) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VC | 1 | tgcggctctcctccacccattctcaatggaaggatctcctactaca gcacccccatagctgtcggcaccgttatccgatacagttgttccg gtactttccggcttatcggcgaaaagtctttgctgtgcattaccaag gataaagtggacgggacttgggacaaacccgcacctaagtgcg agtattttaacaaatatagcagctgccctgagcctatagtacccgg ggggtataaaatccggggctctactccctatcgtcatggcgattct gtgaccttcgcatgtaaaactaatttttcaatgaatggcaacaagtc tgtatggtgtcaagcaaataacatgtggggacctacccgcctgcc aacctgtgtgtcagtgtttcccctggaatgtccagccctccctatg atccacaacggacatcacaccagcgaaaacgttggctccatcgc accagggctctctgtgacttactcttgcgagtccgggtacctgctc gtgggtgaaaagatcatcaactgcctcagtagtggtaaatggtcc gccgtgcctcccacatgtgaagaggcccggtgcaagagcctgg gccggttccccaacggaaaagtgaaggaacctcctatcttgagg gttggtgtgaccgctaactttttctgcgacgaggggtacaggctc caagggcctccctctagtcggtgcgtaatcgccggtcaaggagt cgcatggactaagatgcctgtgtgt | 18 |
| | FH(SCR1-SCR5) | CNELPPRRNTEILTGSW SDQTYPEGTQAIYKCR PGYRSLGNVIMVCRKG EWVALNPLRKCQKRPC GHPGDTPFGTFTLTGG NVFEYGVKAVYTCNEG YQLLGEINYRECDTDG WTNDIPICEVVKCLPVT APENGKIVSSAMEPDR EYHFGQAVRFVCNSGY KIEGDEEMHCSDDGFW SKEKPKCVEISCKSPDV INGSPISQKIIYKENERF QYKCNMGYEYSERGD AVCTESGWRPLPSCEE | 14 | tgtaatgagctgcccccaagacggaatacagagatcctcacagg ctcttggtccgatcaaacttatccagagggtacccaggcaatttac aagtgcagacctggatacaggagcctgggcaatgtgattatggt gtgccgcaagggggagtgggtggcccttaatcctctccggaagt gtcagaaaagaccatgcggacaccctggagatacacctttcggt acctttacccttaccggcggcaatgtcttcgagtatggcgtcaag gccgtgtacacttgtaacgagggataccagctgctgggggaaat aaactatcgtgagtgtgacactgacgggtggactaacgacatcc ccatttgcgaggtggtcaagtgccttcctgtaaccgctcccgaaa atggtaagatcgtatcttccgcaatggagcctgatcgggaatacc actttggacaagccgttcggttcgtatgtaattcagggtataaaatt gagggcgatgaggagatgcactgcagtgatgacggcttttggtc aaaggaaaagccaaagtgcgtagagatcagttgtaagtctcctg acgttattaacgggagtcccatcagtcagaagatcatttacaagg aaaacgagaggttccagtataaatgcaatatgggatatgagtact | 34 |
| | | KSCDNPYIPNGDYSPLR IKHRTGDEITYQCRNGF YPATRGNTAKCTSTGW IPAPRC | | ccgaaagaggggacgccgtgtgcacagagtccggatggcgac ctttgccatcttgtgaagaaaagtcttgtgacaacccctatattcct aacggagattactctcctctgcgcatcaagcaccgaactgggga cgagatcacttaccaatgtcgaaacggcttctaccctgctaccag aggtaacactgccaagtgtaccagcaccggttggattcccgccc ccagatgc | |
| | CR2(SCR1 SCR4)+(G ₄S)₂+linker +FH(SCR1 -SCR5) | CGSPPPILNGRISYYSTP IAVGTVIRYSCSGTFRLI GEKSLLCITKDKVDGT WDKPAPKCEYFNKYSS CPEPIVPGGYKIRGSTP YRHGDSVTFACKTNFS MNGNKSVWCQANNM WGPTRLPTCVSVFPLE CPALPMIHNGHHTSEN VGSIAPGLSVTYSCESG YLLVGEKIINCLSSGKW SAVPPTCEEARCKSLGR FPNGKVKEPPILRVGVT ANFFCDEGYRLQGPPS SRCVIAGQGVAWTKMP VCGGGGSGGGGSEDC NELPPRRNTEILTGSWS DQTYPEGTQAIYKCRP GYRSLGNVIMVCRKGE WVALNPLRKCQKRPCG HPGDTPFGTFTLTGGN VFEYGVKAVYTCNEGY QLLGEINYRECDTDGW TNDIPICEVVKCLPVTA PENGKIVSSAMEPDRE YHFGQAVRFVCNSGYK IEGDEEMHCSDDGFWS KEKPKCVEISCKSPDVI NGSPISQKIIYKENERFQ YKCNMGYEYSERGDA VCTESGWRPLPSCEEK SCDNPYIPNGDYSPLRI KHRTGDEITYQCRNGF YPATRGNTAKCTSTGW IPAPRC | 15 | tgcggctctcctccacccattctcaatggaaggatctcctactaca gcacccccatagctgtcggcaccgttatccgatacagttgttccg gtactttccggcttatcggcgaaaagtctttgctgtgcattaccaag gataaagtggacgggacttgggacaaacccgcacctaagtgcg agtattttaacaaatatagcagctgccctgagcctatagtacccgg ggggtataaaatccggggctctactccctatcgtcatggcgattct gtgaccttcgcatgtaaaactaatttttcaatgaatggcaacaagtc tgtatggtgtcaagcaaataacatgtggggacctacccgcctgcc aacctgtgtgtcagtgtttcccctggaatgtccagccctccctatg atccacaacggacatcacaccagcgaaaacgttggctccatcgc accagggctctctgtgacttactcttgcgagtccgggtacctgctc gtgggtgaaaagatcatcaactgcctcagtagtggtaaatggtcc gccgtgcctcccacatgtgaagaggcccggtgcaagagcctgg gccggttccccaacggaaaagtgaaggaacctcctatcttgagg gttggtgtgaccgctaactttttctgcgacgaggggtacaggctc caagggcctccctctagtcggtgcgtaatcgccggtcaaggagt cgcatggactaagatgcctgtgtgtggcggaggtgggtcgggt ggcggcggatctgaagactgtaatgagctgcccccaagacgga atacagagatcctcacaggctcttggtccgatcaaacttatccag agggtacccaggcaatttacaagtgcagacctggatacaggag cctgggcaatgtgattatggtgtgccgcaagggggagtgggtg gcccttaatcctctccggaagtgtcagaaaagaccatgcggaca ccctggagatacacctttcggtacctttacccttaccggcggcaat gtcttcgagtatggcgtcaaggccgtgtacacttgtaacgaggga taccagctgctgggggaaataaactatcgtgagtgtgacactga cgggtggactaacgacatccccatttgcgaggtggtcaagtgcc ttcctgtaaccgctcccgaaaatggtaagatcgtatcttccgcaat ggagcctgatcgggaataccactttggacaagccgttcggttcgt atgtaattcagggtataaaattgagggcgatgaggagatgcactg cagtgatgacggcttttggtcaaaggaaaagccaaagtgcgtag agatcagttgtaagtctcctgacgttattaacgggagtcccatcag tcagaagatcatttacaaggaaaacgagaggttccagtataaatg caatatgggatatgagtactccgaaagaggggacgccgtgtgc acagagtccggatggcgacctttgccatcttgtgaagaaaagtct tgtgacaacccctatattcctaacggagattactctcctctgcgcat caagcaccgaactggggacgagatcacttaccaatgtcgaaac ggcttctaccctgctaccagaggtaacactgccaagtgtaccag caccggttggattcccgcccccagatgc | 35 |
| | CD5-sp+linker+ CR2(SCR1 SCR4)+(G ₄S)₂+linker +FH(SCR1 - SCR5)+lin ker | MPMGSLQPLATLYLLG MLVASCLGISCGSPPPIL NGRISYYSTPIAVGTVIR YSCSGTFRLIGEKSLLCI TKDKVDGTWDKPAPK CEYFNKYSSCPEPIVPG GYKIRGSTPYRHGDSV TFACKTNFSMNGNKSV WCQANNMWGPTRLPT CVSVFPLECPALPMIHN GHHTSENVGSIAPGLSV TYSCESGYLLVGEKIIN CLSSGKWSAVPPTCEE ARCKSLGRFPNGKVKE PPILRVGVTANFFCDEG YRLOGPPSSRCVIAGO GVAWTKMPVCGGGGS GGGGSEDCNELPPRRN TEILTGSWSDQTYPEGT QAIYKCRPGYRSLGNVI MVCRKGEWVALNPLR KCQKRPCGHPGDTPFG TFTLTGGNVFEYGVKA VYTCNEGYQLLGEINY RECDTDGWTNDIPICE VVKCLPVTAPENGKIVS SAMEPDREYHFGQAVR FVCNSGYKIEGDEEMH CSDDGFWSKEKPKCVE ISCKSPDVINGSPISQKII YKENERFQYKCNMGY EYSERGDAVCTESGWR PLPSCEEKSCDNPYIPN GDYSPLRIKHRTGDEIT YQCRNGFYPATRGNTA KCTSTGWIPAPRCTLK | 16 | atgccaatggggtccctacaacccctggcaacactatatctgcta ggtatgctcgtggcctcctgtttaggaatttcatgcggctctcctcc acccattctcaatggaaggatctcctactacagcacccccatagc tgtcggcaccgttatccgatacagttgttccggtactttccggctta tcggcgaaaagtctttgctgtgcattaccaaggataaagtggacg ggacttgggacaaacccgcacctaagtgcgagtattttaacaaat atagcagctgccctgagcctatagtacccggggggtataaaatc cggggctctactccctatcgtcatggcgattctgtgaccttcgcat gtaaaactaatttttcaatgaatggcaacaagtctgtatggtgtcaa gcaaataacatgtggggacctacccgcctgccaacctgtgtgtc agtgtttcccctggaatgtccagccctccctatgatccacaacgg acatcacaccagcgaaaacgttggctccatcgcaccagggctct ctgtgacttactcttgcgagtccgggtacctgctcgtgggtgaaa agatcatcaactgcctcagtagtggtaaatggtccgccgtgcctc ccacatgtgaagaggcccggtgcaagagcctgggccggttccc caacggaaaagtgaaggaacctcctatcttgagggttggtgtga ccgctaactttttctgcgacgaggggtacaggctccaagggcct ccctctagtcggtgcgtaatcgccggtcaaggagtcgcatggac taagatgcctgtgtgtggcggaggtgggtcgggtggcggcgga tctgaagactgtaatgagctgcccccaagacggaatacagagat cctcacaggctcttggtccgatcaaacttatccagagggtaccca ggcaatttacaagtgcagacctggatacaggagcctgggcaatg tgattatggtgtgccgcaagggggagtgggtggcccttaatcctc tccggaagtgtcagaaaagaccatgcggacaccctggagatac acctttcggtacctttacccttaccggcggcaatgtcttcgagtatg gcgtcaaggccgtgtacacttgtaacgagggataccagctgct gggggaaataaactatcgtgagtgtgacactgacgggtggactaa cgacatccccatttgcgaggtggtcaagtgccttcctgtaaccgc tcccgaaaatggtaagatcgtatcttccgcaatggagcctgatcg ggaataccactttggacaagccgttcggttcgtatgtaattcaggg tataaaattgagggcgatgaggagatgcactgcagtgatgacgg cttttggtcaaaggaaaagccaaagtgcgtagagatcagttgtaa gtctcctgacgttattaacgggagtcccatcagtcagaagatcatt tacaaggaaaacgagaggttccagtataaatgcaatatgggatat gagtactccgaaagaggggacgccgtgtgcacagagtccggat ggcgacctttgccatcttgtgaagaaaagtcttgtgacaaccccta tattcctaacggagattactctcctctgcgcatcaagcaccgaact ggggacgagatcacttaccaatgtcgaaacggcttctaccctgct accagaggtaacactgccaagtgtaccagcaccggttggattcc cgcccccagatgcacacttaaa | 36 |
| - | CD5-sp signal peptide | MPMGSLQPLATLYLLG MLVASCLG | 17 | atgccaatggggtccctacaacccctggcaacactatatctgcta ggtatgctcgtggcctcctgtttagga | 37 |
| | | | | atgcccatgggcagcctgcagcccctggccaccctgtacctgct gggcatgctggtggctagctgcctgggc | 38 |

### Example 2: Preparation and Identification of Recombinant AAV Viruses

Recombinant AAV viruses were prepared using a triple-plasmid packaging system. The helper plasmid (phelper), AAV Cap and Rep proteins expression plasmid, and target vector expression plasmid (ssAAV-XMDC025, ssAAV-XMDC026, ssAAV-XMDC029, ssAAV-CR2-FH(1-4), ssAAV-CR2-FH(1-5)) were mixed at a mass ratio of 2:1:1, and formed transfection complexes with PEI transfection reagent. These complexes were used to transfect HEK293T cells for the packaging of AAV-XMDC025, AAV-XMDC026, AAV-XMDC029, AAV-CR2-FH(1-4), and AAV-CR2-FH(1-5) viruses. The supernatant was collected twice on the 3rd and 7th days after transfection, respectively, to obtain AAV virus particles containing the target genes. Different gradients of iodixanol (15%, 25%, 40% and 60%) were used for density gradient centrifugation (Beckman's ultracentrifuge) to obtain purified AAV viruses. For the obtained purified AAV viruses, the quality of AAV was identified by transmission electron microscopy, and the titers of AAV viruses were quantified by qPCR.

### Example 3: PEG-400-induced Dry AMD Mouse Model and Administration of AAV-XMDC025, AAV-XMDC026, AAV-XMDC029, AAV-CR2-FH(1-4), AAV-CR2-FH(1-5) via Injection

A dry AMD mouse model was induced using polyethylene glycol (PEG). Mice treated with PEG exhibited retinal pathological changes similar to the clinical pathological features of dry AMD, such as retinal structural destruction, RPE cell damage, and photoreceptor loss. The specific method is as follows:

Seventy 4-week-old male C57BL/6J mice of SPF grade (purchased from Beijing Vital River Laboratory Animal Technology Co., Ltd.), weighing approximately 14 g, were raised according to their rhythm in an environment with alternating 12-hour light-dark cycle.

Since a certain period of time is required for the expression of the target gene to reach a stable level after AAV injection, bilateral intravitreal injection of the drug on Day 1, and bilateral subretinal injection of PEG-400 on Day 22 were conducted to establish the model, and the eyeballs were enucleated on Day 27 for frozen sectioning, followed by staining to observe the structures of the retina and RPE. The treatment is shown in Table 2.

**Table 2. Bilateral intravitreal injection of the mice on Day 1**

| **XMDC025, XMDC026 Administration(D1)** | | | | | | |
|---|---|---|---|---|---|---|
| Group | Administration (Injection on D1) | Concentration (vg/mL) | Volume (µL) | Dosage (vg/eye) | Administration Route | Numbers of Animals |
| Control | PBS | - | 2 | - | Intravitreal injection | 5 |
| Model | PBS | - | 2 | - | Intravitreal injection | 5 |
| XMDC025 | AAV-XMDC025 | 1.57×10¹² | 2 | 3.14×10⁹ | Intravitreal injection | 5 |
| XMDC026 | AAV-XMDC026 | 1.57×10¹² | 2 | 3.14×10⁹ | Intravitreal injection | 5 |

| **XMDC029, CR2-FH(1-4), CR2-FH(1-5) Administration(D1)** | | | | | | |
|---|---|---|---|---|---|---|
| Group | Administration (Injection on D1) | Concentration (vg/mL) | Volume (µL) | Dosage (vg/eye) | Administration Route | Numbers of Animals |
| Control | PBS | - | 2 | - | Intravitreal injection | 10 |
| Model | PBS | - | 2 | - | Intravitreal injection | 10 |
| XMDC029 | AAV-XMDC029 | 2.8×1012 | 2 | 5.6×10⁹ | Intravitreal injection | 10 |
| CR2-FH(1-4) | AAV-CR2-FH(1-4) | 2.8×1012 | 2 | 5.6×10⁹ | Intravitreal injection | 10 |
| CR2-FH(1-5) | AAV-CR2-FH(1-5) | 2.8×1012 | 2 | 5.6×10⁹ | Intravitreal injection | 10 |

The specific operation steps are as follows:

Intravitreal injection administration: Both eyes of a mouse were dripped with 5% mydriatic solution for mydriasis, and the animal was anesthetized by intraperitoneal injection of 5% chloral hydrate at 10 mL/kg (Sangon). The anesthetized animal was placed in a lateral position on the operating table. 1-2 mm posterior to the corneoscleral limbus in the superotemporal or superonasal quadrant was selected as the needle insertion site (syringe: Hamilton needle, 7632-01). Care was taken to avoid damaging the posterior lens capsule and other retinal parts. The needle was inserted into the vitreous cavity, the bolus injection was conducted. After a 10 seconds pause, the needle was slowly withdrawn, erythromycin eye ointment was applied, and the animal was returned to the cage.

### Example 4: Evaluation of the Effects of AAV-XMDC025, AAV-XMDC026, AAV-XMDC029, AAV-CR2-FH(1-4), and AAV-CR2-FH(1-5) on the Thickness and Nuclear Density of the Outer Nuclear Layer of the Retina in PEG-400-induced Dry AMD Model Mice by Hematoxylin-eosin (HE) Staining

The eyeballs of the mice in Example 3 were enucleated. One eye was taken and fixed in 4% paraformaldehyde (biosharp) at 4°C overnight. After washing 4 times with PBS (biosharp), the cornea and lens were peeled off, and the eye cups including the retina, choroid, sclera, etc., were dehydrated with 30% sucrose (Sangon). After sinking to the bottom, 10 µm thick frozen sections were prepared and subjected to HE staining. After staining, photos were taken, and Image J was used to measure the thickness and nuclear density of the outer nuclear layer (ONL) of the retinal sections, and the differences were analyzed.

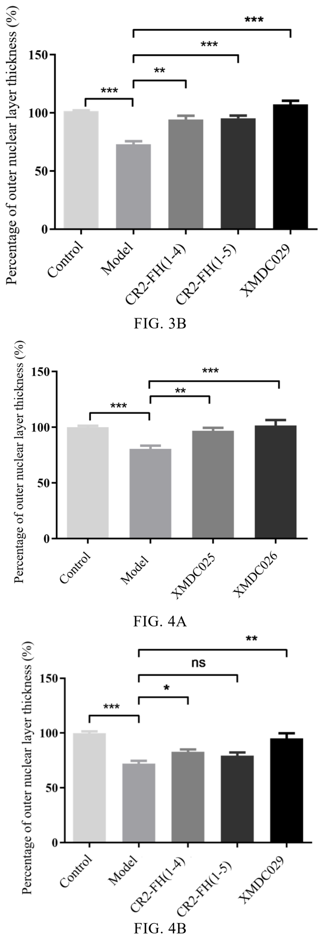

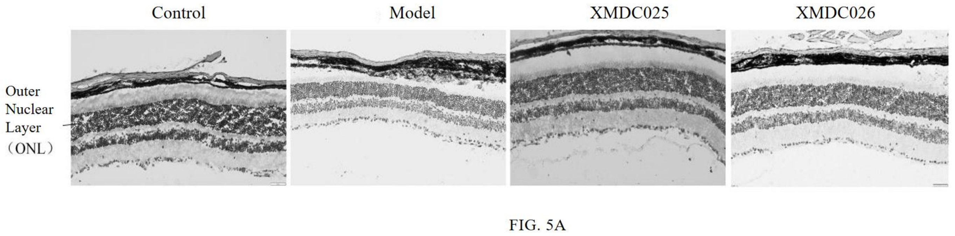

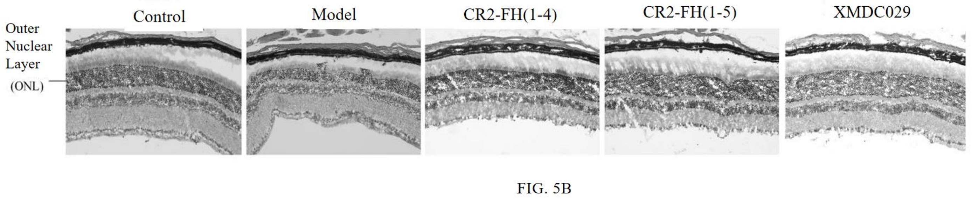

The results are shown in Figures 3, 4, and 5. Compared with the control group, the thickness and nuclear density of the outer nuclear layer of the retina in the model group mice were significantly reduced, and the retinal structures were obviously damaged. Compared with the model group, injections of AAV-XMDC025, AAV-XMDC026, AAV-XMDC029, and AAV-CR2-FH(1-4) could significantly increase the thickness and nuclear density of the outer nuclear layer and improve retinal structural damage; injection of AAV-CR2-FH(1-5) could only significantly increase the thickness of the outer nuclear layer. The improvement effect of AAV-XMDC029 on retinal thickness was better than that of AAV-CR2-FH(1-4), and its improvement effect on retinal nuclear density was better than that of AAV-CR2-FH(1-4) and AAV-CR2-FH(1-5).

### Example 5: Evaluation of the Effects of AAV-XMDC025, AAV-XMDC026, AAV-XMDC029, AAV-CR2-FH(1-4), and AAV-CR2-FH(1-5) on Retinal Photoreceptor Cells in PEG-400-induced Dry AMD Model Mice by Immunofluorescence Staining

The frozen sections from Example 4 were air-dried at room temperature in a slide box, and the tissues on the slides were circled with an immunohistochemical pen (Vectorlabs). The polylysine-coated slides (Shitai) were placed in a humid chamber, and the tissues on the slides were washed with PBS, incubated at room temperature for 5 minutes, the PBS on the slides was poured off, and this washing step was repeated 5 times, 10 minutes each time. 500 µL of blocking solution (5% goat serum (Beyotime) + 0.5% Triton 100 (Sangon)) was added to each slide, and incubated in a humid chamber at room temperature for 45 minutes. The blocking solution on the slides was poured off, and the primary antibody mixture: cone cell photosensitive substance Cone Arresting-1 (EMD Millipore Corp) and rod cell photosensitive substance Rodopsin-1 (Santa Cruz) was added, 200 µL per slide, and the slides were placed in a humid chamber and incubated overnight at 4°C. The next day, the slides were rinsed with PBS 5 times at room temperature, 5 minutes each time. The secondary antibody mixture: Anti-rabbit IgG Alexa Fluor 555 (Cell Signaling) and Donkey anti-Mouse IgG (H+L) Highly Cross-Adsorbed Alexa Fluor 488 (Invitrogen) diluted at 1:500 was added for counterstaining, and the slides were incubated in a humid chamber at room temperature in the dark for 2.5 hours. All subsequent steps required protection from light. The secondary antibody was removed, and the slides were rinsed with PBS 5 times at room temperature, 5 minutes each time. DAPI staining solution (Sigma) was added, 50 µL per slide, incubated at room temperature for 10 minutes, then rinsed with PBS 3 times, 5 minutes each time. The slides were mounted, and observed and photographed under a fluorescence microscope.

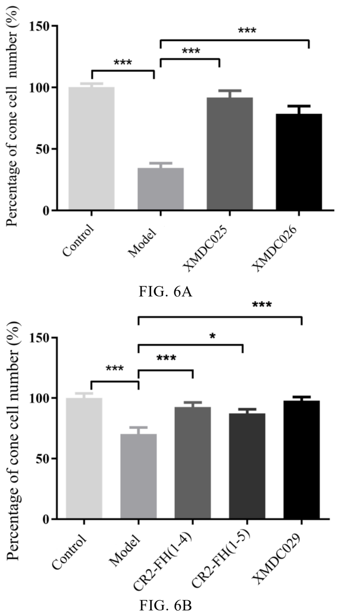

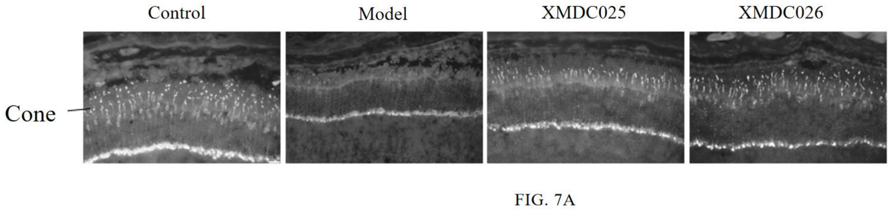

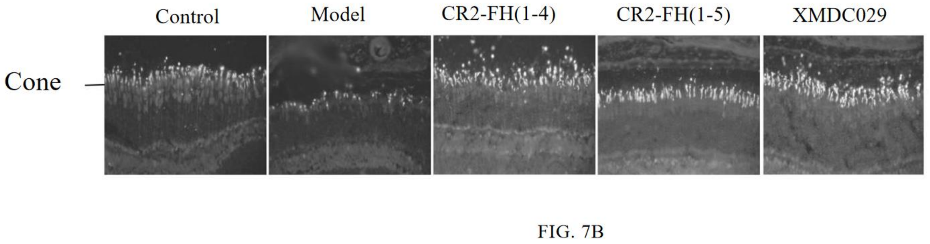

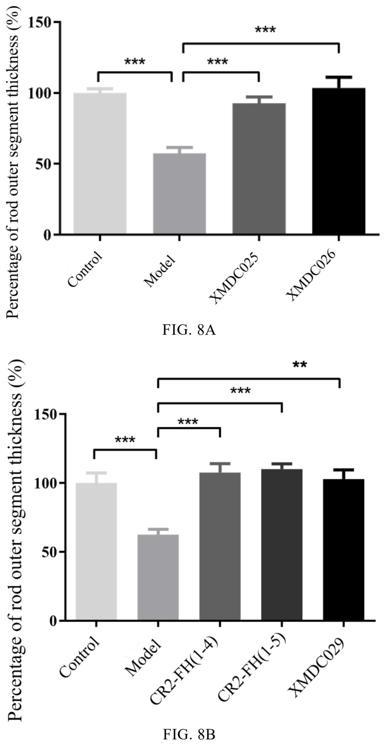

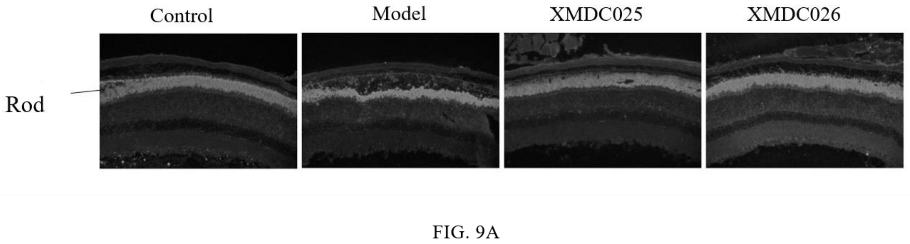

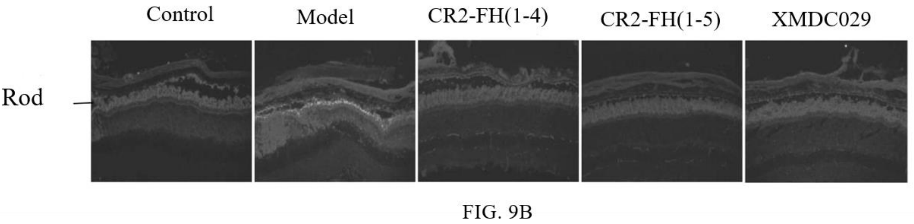

The results are shown in Figures 6, 7, 8, and 9. Compared with the control group, the model group showed reduced number of retinal cone cells, narrowed thickness of the outer segments of rod cells, and significantly damaged photoreceptor cells. Compared with the model group, injections of AAV-XMDC025, AAV-XMDC026, AAV-XMDC029, AAV-CR2-FH(1-4), and AAV-CR2-FH(1-5) all significantly improved the damage to cone and rod cells; the improvement effects of AAV-XMDC029 and AAV-CR2-FH(1-4) on cone cell damage were better than that of AAV-CR2-FH(1-5).

### Example 6: Evaluation of the Effects of AAV-XMDC025, AAV-XMDC026, AAV-XMDC029, AAV-CR2-FH(1-4), and AAV-CR2-FH(1-5) on RPE Cells in PEG-400-induced Dry AMD Model Mice by F-actin Staining

The other eyes removed from the mice in Example 3 were fixed in 4% paraformaldehyde at 4°C overnight; the cornea, lens, muscles, and retina were removed, and the sclera, choroid, and RPE complex were fixed in 4% paraformaldehyde at room temperature for 2 hours, then washed 3 times with PBS, 5 minutes each time; the sclera, choroid, and RPE complex were blocked in blocking solution (5% goat serum + 0.5% Triton X-100) at room temperature for 1 hour, stained with AlexaFluor^{®}594 phalloidin (Jackson ImmunoResearch) (1:40 methanol stock solution) on a shaker at room temperature for 40 minutes, then washed 6 times with PBS on a shaker, 5 minutes each time, stained with DAPI at room temperature for 30 minutes, then washed 3 times with PBS, 5 minutes each time. The sclera, choroid, and RPE complex were transferred to a glass slide, cut into about 8 pieces, mounted, and observed and photographed under a fluorescence microscope.

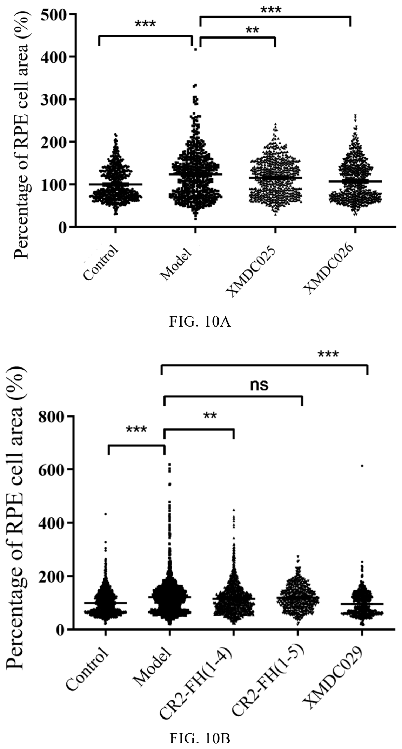

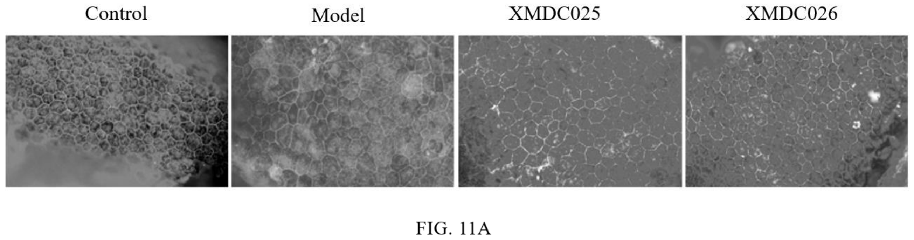

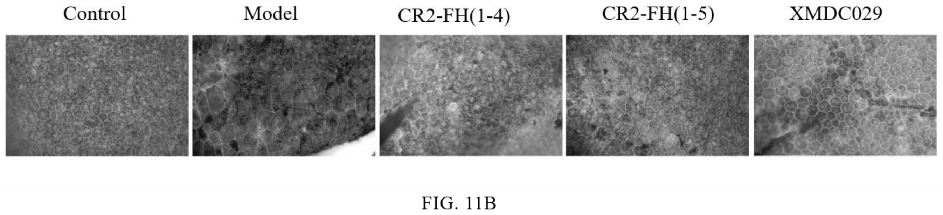

The results are shown in Figures 10 and 11. Compared with the control group, the RPE cells in the model group were significantly enlarged and obviously damaged. Compared with the model group, the injection of AAV-XMDC025, AAV-XMDC026, AAV-XMDC029, and AAV-CR2-FH(1-4) could significantly reduce the area of RPE cells and improve the damage of RPE cells, while the injection of AAV-CR2-FH(1-5) had no improvement effect on RPE cell damage. The improvement effect of AAV-XMDC029 on RPE cell damage was better than that of AAV-CR2-FH(1-4) and AAV-CR2-FH(1-5).

Combining the results of HE staining, immunofluorescence staining, and F-actin staining, the injection of AAV-XMDC029 demonstrated better improvement effect on the nuclear density of the outer nuclear layer of the retina and RPE cell damage in PEG-400-induced dry AMD mouse model compared to AAV-CR2-FH(1-4) and AAV-CR2-FH(1-5); better improvement effect on outer nuclear layer thickness compared to AAV-CR2-FH(1-4); and better improvement effect on cone cell compared to AAV-CR2-FH(1-5).

### Example 7: Construction of Other Plasmid Vectors

### Scheme 1

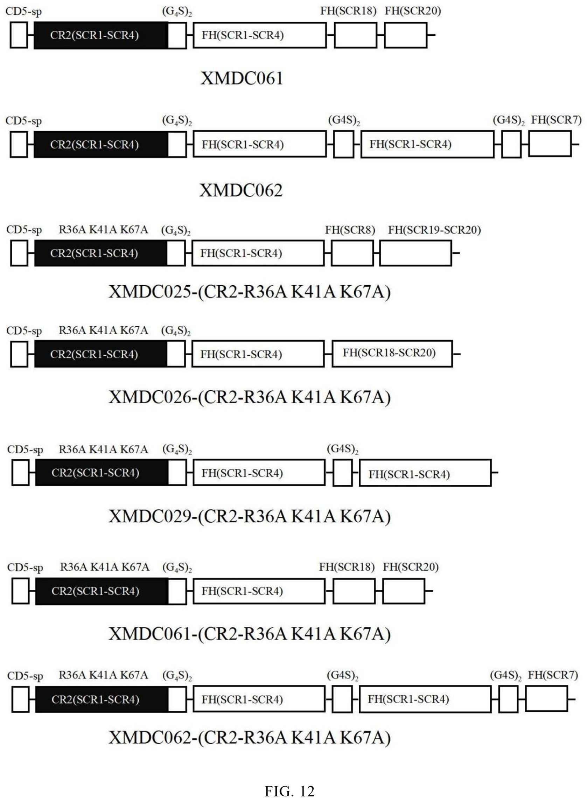

According to the amino acid sequences of CR2 (Gene ID: 1380) SCR1-SCR4 (SEQ ID NO: 1), FH (Gene ID: 3075) SCR1-SCR4 (SEQ ID NO: 2), SCR18 (SEQ ID NO: 39), and SCR20 (SEQ ID NO: 40) published on NCBI, adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+FH(SCR18)+linker+FH(SCR20)+linker was constructed. The nucleotide sequence was designed according to human codon preference, with a BamH I restriction site introduced at the 5' end and an EcoR V restriction site introduced at the 3' end. It was named XMDC061 (the full-length gene was synthesized by Suzhou Genewiz Biotechnology Co., Ltd.), and its structural diagram is shown in Figure 12.

### Scheme 2

According to the amino acid sequence of CR2 (Gene ID: 1380) SCR1-SCR4 (SEQ ID NO: 1) and FH (Gene ID: 3075) SCR1-SCR4 (SEQ ID NO: 2), SCR7 (SEQ ID NO: 43) published on NCBI, adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+(G₄S)₂+linker+FH(SCR1-SCR4)+linker+(G₄S)₂+linker+FH(SCR7)+linker was constructed. The nucleotide sequence was designed according to human codon preference, with a BamH I restriction site introduced at the 5' end and an EcoR V restriction site introduced at the 3' end. It was named XMDC062 (the full-length gene was synthesized by Suzhou Genewiz Biotechnology Co., Ltd.), and its structural diagram is shown in Figure 12.

### Scheme 3

According to the amino acid sequences of CR2 (Gene ID: 1380) SCR1-SCR4 R36A K41A K67A mutant (SEQ ID NO: 46), and FH (Gene ID: 3075) SCR1-SCR4 (SEQ ID NO: 2), SCR8 (SEQ ID NO: 3), SCR19-SCR20 (SEQ ID NO: 4) published on NCBI, adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+FH(SCR8)+linker+FH(SCR19-SCR20)+linker was constructed. The nucleotide sequence was designed according to human codon preference, with a BamH I restriction site introduced at the 5' end and an EcoR V restriction site introduced at the 3' end. It was named XMDC025-(CR2-R36A K41A K67A) (the full-length gene was synthesized by Sangon Biotech (Shanghai) Co., Ltd.), and its structural diagram is shown in Figure 12.

### Scheme 4

According to the amino acid sequence of CR2 (Gene ID: 1380) SCR1-SCR4 R36A K41A K67A mutant (SEQ ID NO: 46), and FH (Gene ID: 3075) SCR1-SCR4 (SEQ ID NO: 2), SCR18-SCR20 (SEQ ID NO: 7), adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+FH(SCR18-SCR20)+linker was constructed. The nucleotide sequence was designed according to human codon preference, with a BamH I restriction site introduced at the 5' end and an EcoR V restriction site introduced at the 3' end. It was named XMDC026-(CR2-R36A K41A K67A) (the full-length gene was synthesized by Sangon Biotech (Shanghai) Co., Ltd.), and its structural diagram is shown in Figure 12.

### Scheme 5

According to the amino acid sequence of CR2 (Gene ID: 1380) SCR1-SCR4 R36A K41A K67A mutant (SEQ ID NO: 46) and FH (Gene ID: 3075) SCR1-SCR4 (SEQ ID NO: 2), adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+(G₄S)₂+linker+FH(SCR1-SCR4)+linker was constructed. The nucleotide sequence was designed according to human codon preference, with a BamH I restriction site introduced at the 5' end and an EcoR V restriction site introduced at the 3' end. It was named XMDC029-(CR2-R36A K41A K67A) (the full-length gene was synthesized by Suzhou Genewiz Biotechnology Co., Ltd.), and its structural diagram is shown in Figure 12.

### Scheme 6

According to the amino acid sequences of CR2 (Gene ID: 1380) SCR1-SCR4 R36A K41A K67A mutant (SEQ ID NO: 46), FH (Gene ID: 3075) SCR1-SCR4 (SEQ ID NO: 2), SCR18 (SEQ ID NO: 39), and SCR20 (SEQ ID NO: 40) published on NCBI, adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+FH(SCR18)+linker+FH(SCR20)+linker was constructed. The nucleotide sequence was designed according to human codon preference, with a BamH I restriction site introduced at the 5' end and an EcoR V restriction site introduced at the 3' end. It was named XMDC061 - (CR2-R36A K41A K67A) (the full-length gene was synthesized by Suzhou Genewiz Biotechnology Co., Ltd.), and its structural diagram is shown in Figure 12.

### Scheme 7

According to the amino acid sequence of CR2 (Gene ID: 1380) SCR1-SCR4 R36A K41A K67A mutant (SEQ ID NO: 46) and FH (Gene ID: 3075) SCR1-SCR4 (SEQ ID NO: 2), SCR7 (SEQ ID NO: 43) published on NCBI, adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G₄S)₂+linker+FH(SCR1-SCR4)+linker+(G₄S)₂+linker+FH(SCR1-SCR4)+linker+(G₄S)₂+linker+FH(SCR7)+linker was constructed. The nucleotide sequence was designed according to human codon preference, with a BamH I restriction site introduced at the 5' end and an EcoR V restriction site introduced at the 3' end. It was named XMDC062-(CR2-R36A K41A K67A) (the full-length gene was synthesized by Suzhou Genewiz Biotechnology Co., Ltd.), and its structural diagram is shown in Figure 12.

The endogenous linker in the above structure is the endogenous linking sequence in the CR2 or FH structure. Therefore, those skilled in the art should understand that the linker sequence in the structure is determined according to different CR2 or FH structure sequences linked at the C-terminal or the N-terminal thereof.

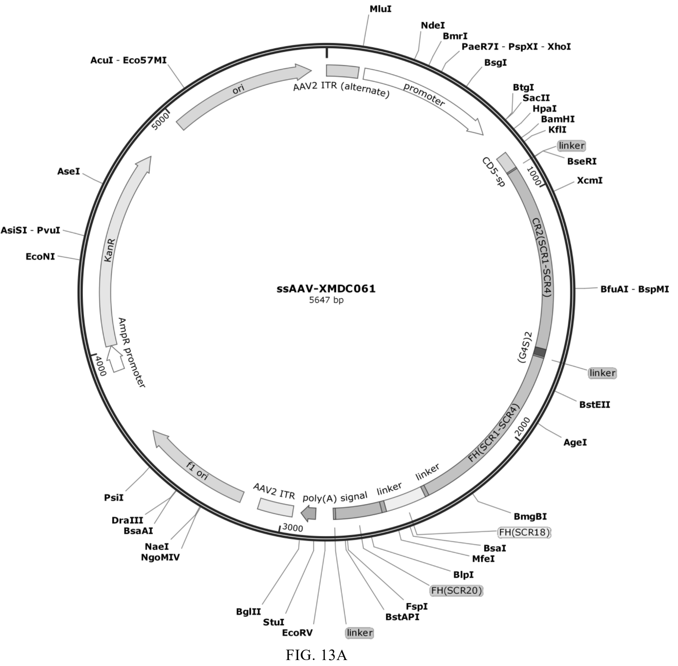

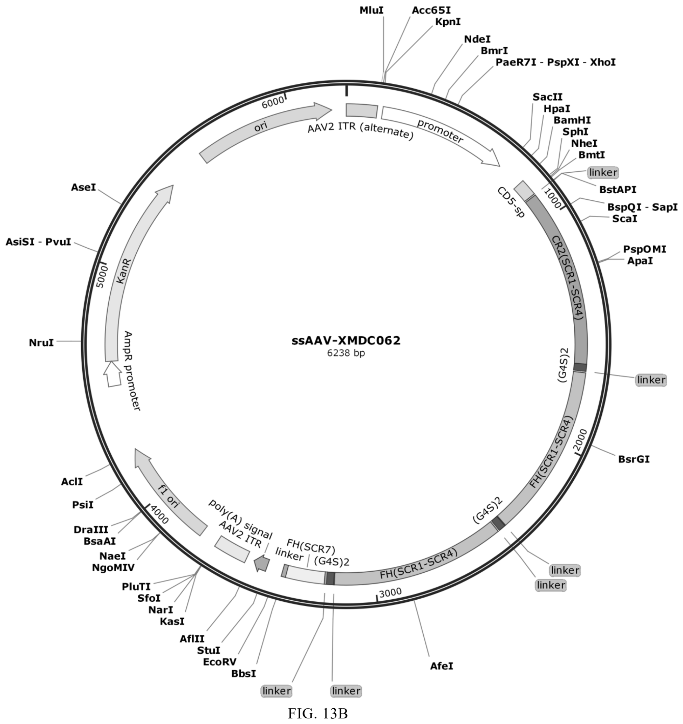

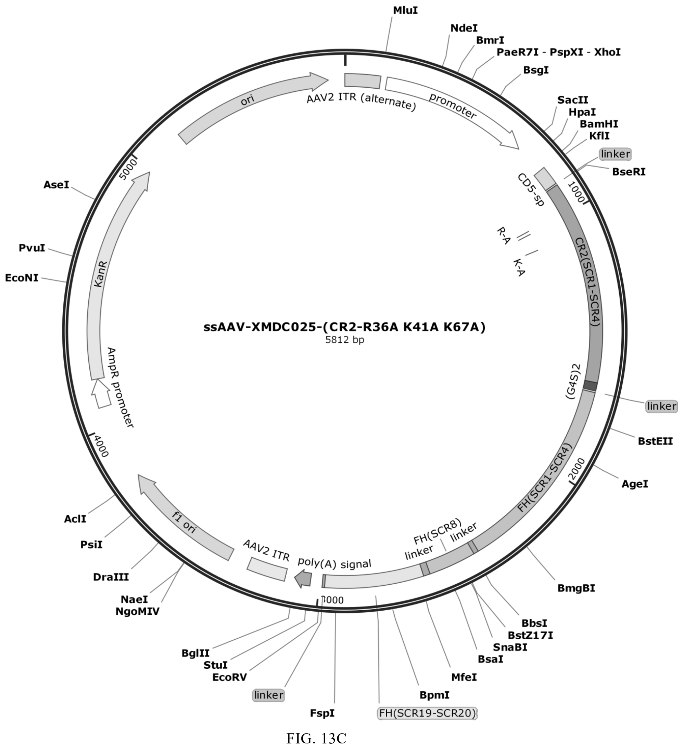

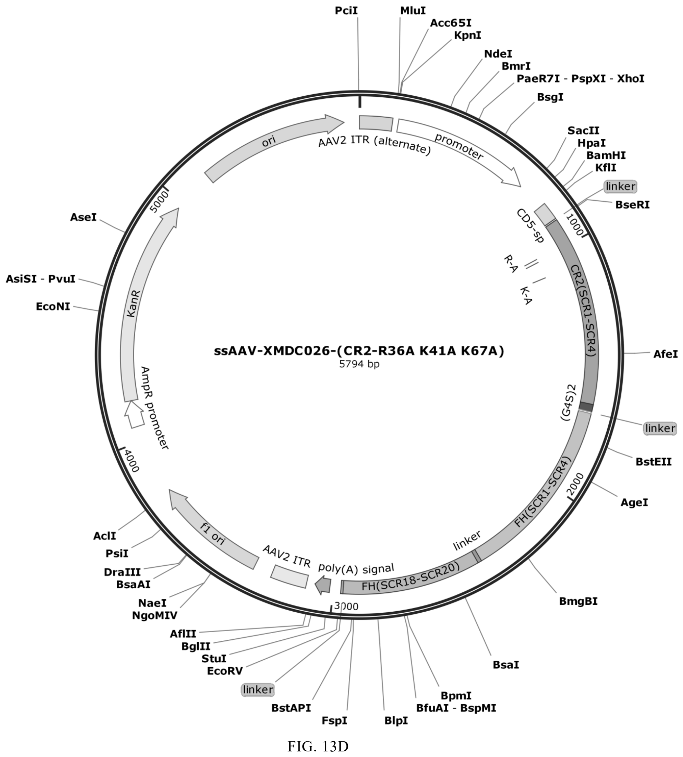

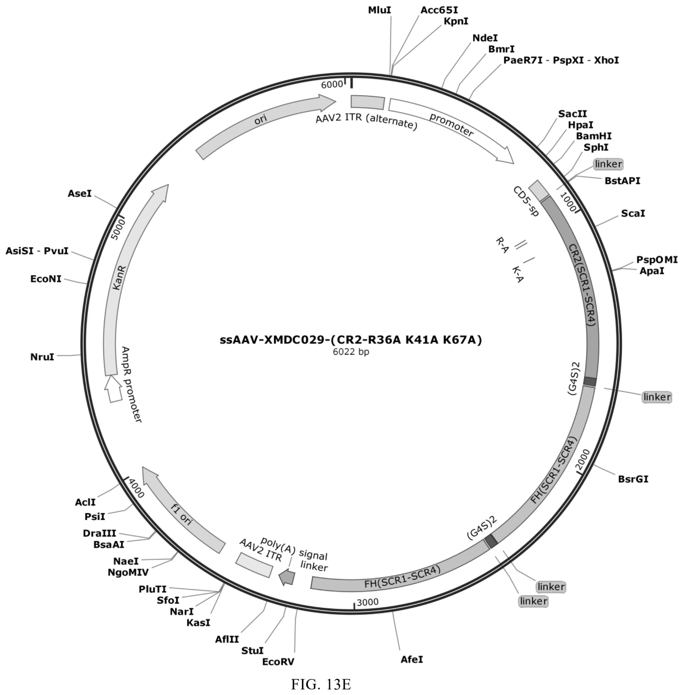

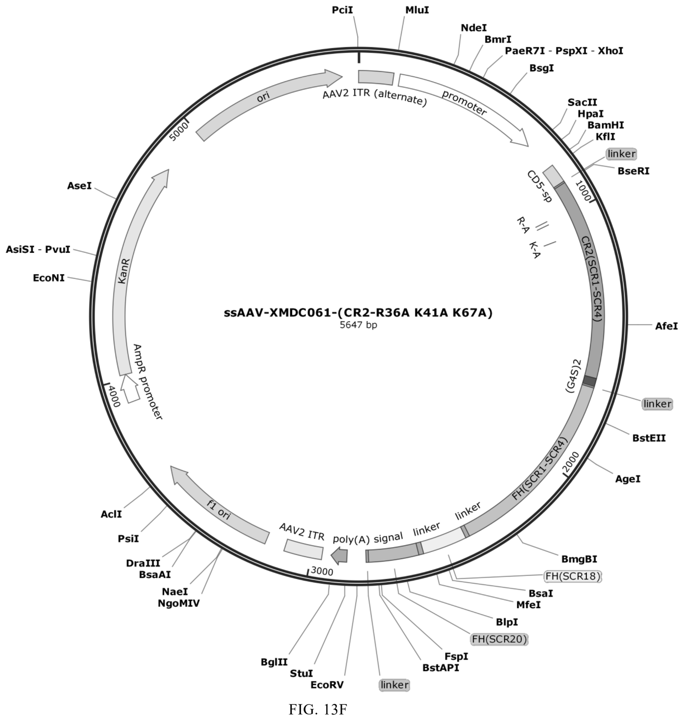

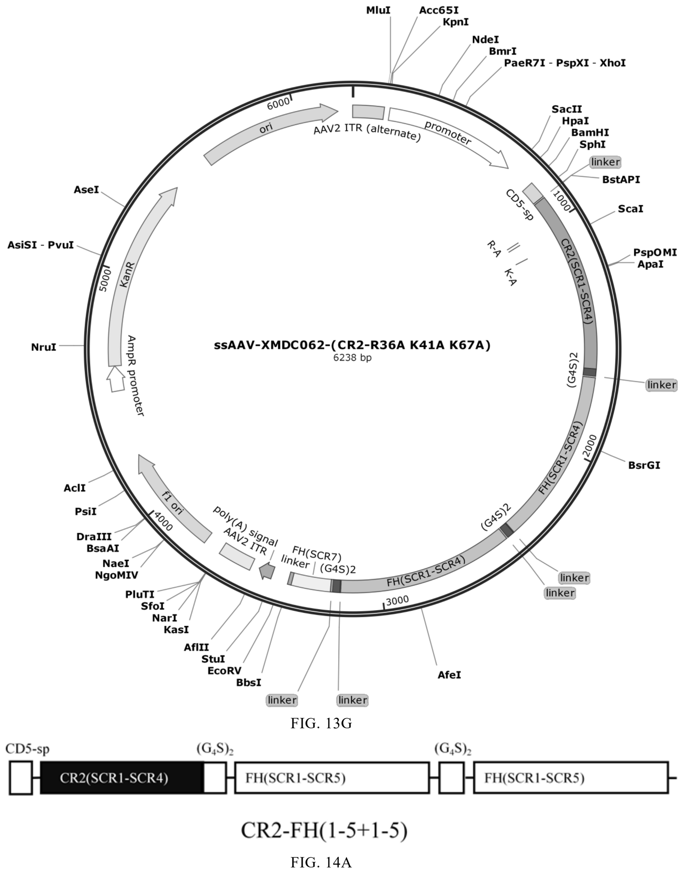

The ssAAV-XMDC061, ssAAV-XMDC062, ssAAV-XMDC025-(CR2-R36A K41A K67A), ssAAV-XMDC026-(CR2-R36A K41A K67A), ssAAV-XMDC029-(CR2-R36A K41A K67A), ssAAV-XMDC061-(CR2-R36A K41A K67A), and ssAAV-XMDC062-(CR2-R36A K41A K67A) vectors were constructed by double digestion of XMDC061, XMDC062, XMDC025-(CR2-R36A K41A K67A), XMDC026-(CR2-R36A K41A K67A), XMDC029-(CR2-R36A K41A K67A), XMDC061-(CR2-R36A K41A K67A), XMDC062-(CR2-R36A K41A K67A) and ssAAV plasmids with BamH I/EcoR V, followed by conventional molecular biology operations such as ligation, transformation, and clone screening and identification. The vector information is shown in Figure 13. High-quality plasmid DNA was obtained for later use using an endotoxin-free plasmid extraction kit (MN).

### Scheme 8

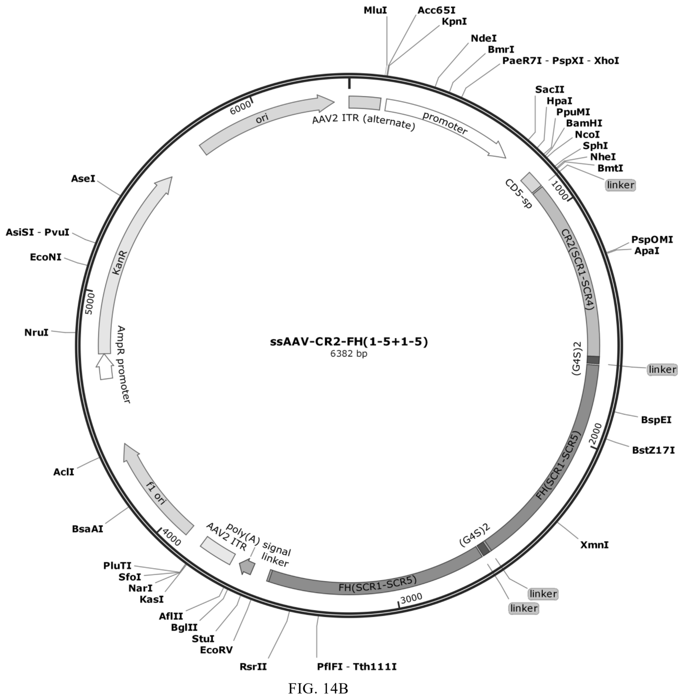

According to the amino acid sequence of CR2 (Gene ID: 1380) SCR1-SCR4 (SEQ ID NO: 1) and FH (Gene ID: 3075) SCR1-SCR5 (SEQ ID NO: 14) published on NCBI, adding the secretion signal peptide CD5-sp amino acid sequence (SEQ ID NO: 17) to the N-terminus of the composition, and using (G₄S)₂ and endogenous linker for linkage, an open reading frame with the structure of CD5-sp+linker+CR2(SCR1-SCR4)+(G_{4S})₂+linker+FH(SCR1-SCR5)+linker+(G₄S)₂+linker+FH(SCR1-SCR5)+linker was constructed. The nucleotide sequence was designed according to human codon preference, with a BamH I restriction site introduced at the 5' end and an EcoR V restriction site introduced at the 3' end. It was named CR2-FH(SCR1-5+1-5) (the full-length gene was synthesized by Suzhou Genewiz Biotechnology Co., Ltd.), and its structural diagram is shown in Figure 14.

The endogenous linker in the above structure is the endogenous linking sequence in the CR2 or FH structure. Therefore, those skilled in the art should understand that the linker sequence in the structure is determined according to different CR2 or FH structure sequences linked at the C-terminal or the N-terminal thereof.

The ssAAV-CR2-FH(SCR1-5+1-5) vector was constructed by double digestion of CR2-FH(SCR1-5+1-5) and ssAAV plasmid with BamH I/EcoR V, followed by conventional molecular biology operations such as ligation, transformation, and clone screening and identification. The vector information is shown in Figure 14. High-quality plasmid DNA was obtained for later use using an endotoxin-free plasmid extraction kit (MN).

**Table3. Sequence information**

| **Product s No.** | **Sequence Fragment Names** | **Amino Acid Sequences** | **SEQ ID NO** | **Nucleic Acid Sequences** | **SEQ ID NO** |
|---|---|---|---|---|---|
| XMDC 061 | CR2(SCR1 -SCR4) | CGSPPPILNGRISYYSTPIA VGTVIRYSCSGTFRLIGEK SLLCITKDKVDGTWDKPA PKCEYFNKYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVC | 1 | tgcggctctcctccacccattctcaatggaaggatctcctac tacagcacccccatagctgtcggcaccgttatccgatacag ttgttccggtactttccggcttatcggcgaaaagtctttgctg tgcattaccaaggataaagtggacgggacttgggacaaac ccgcacctaagtgcgagtattttaacaaatatagcagctgc cctgagcctatagtacccggggggtataaaatccggggct ctactccctatcgtcatggcgattctgtgaccttcgcatgtaa aactaatttttcaatgaatggcaacaagtctgtatggtgtcaa gcaaataacatgtggggacctacccgcctgccaacctgtg tgtcagtgtttcccctggaatgtccagccctccctatgatcc acaacggacatcacaccagcgaaaacgttggctccatcg caccagggctctctgtgacttactcttgcgagtccgggtac ctgctcgtgggtgaaaagatcatcaactgcctcagtagtgg taaatggtccgccgtgcctcccacatgtgaagaggcccgg tgcaagagcctgggccggttccccaacggaaaagtgaag gaacctcctatcttgagggttggtgtgaccgctaactttttct gcgacgaggggtacaggctccaagggcctccctctagtc ggtgcgtaatcgccggtcaaggagtcgcatggactaagat gcctgtgtgt | 18 |
| | FH(SCR1-SCR4) | CNELPPRRNTEILTGSWSD QTYPEGTQAIYKCRPGYR SLGNVIMVCRKGEWVALN PLRKCQKRPCGHPGDTPF GTFTLTGGNVFEYGVKAV YTCNEGYQLLGEINYREC | 2 | tgcaacgaactgcctcctagaagaaacaccgaaatcctca caggctcctggtcagatcagacctaccctgagggtacaca ggccatctataaatgccgcccgggttaccgctccttgggca atgttattatggtctgccggaaaggggaatgggtggccttg aaccccctcagaaagtgccaaaaaagaccctgcggacat ccaggagacacacctttcgggacatttaccttgaccggtgg | 19 |
| | | DTDGWTNDIPICEVVKCLP VTAPENGKIVSSAMEPDRE YHFGQAVRFVCNSGYKIE GDEEMHCSDDGFWSKEK PKCVEISCKSPDVINGSPIS QKIIYKENERFQYKCNMG YEYSERGDAVCTESGWRP LPSC | | taacgtgttcgagtacggagtgaaggcagtctatacttgca atgagggctaccagctcctgggggaaataaattatcgcga atgtgatacagacggctggacgaacgacattcctatctgtg aggtggtcaaatgtctgcccgtcactgctcctgaaaacggc aagatagtgagttctgcaatggagccggatagggagtatc attttggtcaggccgtgcgctttgtgtgcaatagtgggtata aaatcgagggggatgaagaaatgcactgtagcgacgatg gattctggtctaaagagaaacctaagtgtgtggagattagtt gcaagagtccagacgtgattaacggtagccccatttccca aaagataatatacaaggaaaacgaaagattccagtataaat gcaacatgggctacgagtatagcgagcggggcgatgcc gtgtgtactgaaagcggctggcgccccctgccttcctgc | |
| | FH(SCR18) | CVNPPTVQNAYIVSRQMS KYPSGERVRYQCRSPYEM FGDEEVMCLNGNWTEPPQ C | 39 | tgcgtgaaccctccaacagtccaaaatgcctatatagtctct aggcagatgagtaaatacccaagtggagagagagtgaga taccagtgccggtctccttacgagatgttcggcgacgagg aagtgatgtgcctgaacggcaattggacagagccaccac aatgt | 57 |
| | FH(SCR20) | CVISREIMENYNIALRWTA KQKLYSRTGESVEFVCKR GYRLSSRSHTLRTTCWDG KLEYPTC | 40 | tgtgtgatcagtcgggagatcatggagaactacaacattgc cctgaggtggactgctaagcagaagctctatagccgaaca ggcgagtcagtggagtttgtgtgtaaacggggatacaggc tgtccagtaggagccacaccctgcgcactacttgctgggat gggaagctggaatatccaacttgc | 58 |
| | CR2(SCR1 SCR4)+(G ₄S)₂+linker +FH(SCR1 - SCR4)+lin ker+FH(S CR18)+lin ker+FH(S CR20) | CGSPPPILNGRISYYSTPIA VGTVIRYSCSGTFRLIGEK SLLCITKDKVDGTWDKPA PKCEYFNKYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVCGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCERDTSCVNPPTV QNAYIVSRQMSKYPSGER VRYQCRSPYEMFGDEEVM CLNGNWTEPPQCKDSTGK CVISREIMENYNIALRWTA KQKLYSRTGESVEFVCKR GYRLSSRSHTLRTTCWDG KLEYPTC | 41 | tgcggctctcctccacccattctcaatggaaggatctcctac tacagcacccccatagctgtcggcaccgttatccgatacag ttgttccggtactttccggcttatcggcgaaaagtctttgctg tgcattaccaaggataaagtggacgggacttgggacaaac ccgcacctaagtgcgagtattttaacaaatatagcagctgc cctgagcctatagtacccggggggtataaaatccggggct ctactccctatcgtcatggcgattctgtgaccttcgcatgtaa aactaatttttcaatgaatggcaacaagtctgtatggtgtcaa gcaaataacatgtggggacctacccgcctgccaacctgtg tgtcagtgtttcccctggaatgtccagccctccctatgatcc acaacggacatcacaccagcgaaaacgttggctccatcg caccagggctctctgtgacttactcttgcgagtccgggtac ctgctcgtgggtgaaaagatcatcaactgcctcagtagtgg taaatggtccgccgtgcctcccacatgtgaagaggcccgg tgcaagagcctgggccggttccccaacggaaaagtgaag gaacctcctatcttgagggttggtgtgaccgctaactttttct gcgacgaggggtacaggctccaagggcctccctctagtc ggtgcgtaatcgccggtcaaggagtcgcatggactaagat gcctgtgtgtggcggaggtgggtcgggtggcggcggatc tgaggactgcaacgaactgcctcctagaagaaacaccga aatcctcacaggctcctggtcagatcagacctaccctgag ggtacacaggccatctataaatgccgcccgggttaccgct ccttgggcaatgttattatggtctgccggaaaggggaatgg gtggccttgaaccccctcagaaagtgccaaaaaagaccct gcggacatccaggagacacacctttcgggacatttaccttg accggtggtaacgtgttcgagtacggagtgaaggcagtct atacttgcaatgagggctaccagctcctgggggaaataaat tatcgcgaatgtgatacagacggctggacgaacgacattc ctatctgtgaggtggtcaaatgtctgcccgtcactgctcctg aaaacggcaagatagtgagttctgcaatggagccggatag ggagtatcattttggtcaggccgtgcgctttgtgtgcaatag tgggtataaaatcgagggggatgaagaaatgcactgtagc gacgatggattctggtctaaagagaaacctaagtgtgtgga gattagttgcaagagtccagacgtgattaacggtagcccca tttcccaaaagataatatacaaggaaaacgaaagattccag tataaatgcaacatgggctacgagtatagcgagcggggc gatgccgtgtgtactgaaagcggctggcgccccctgcctt cctgcgagcgggatacaagctgcgtgaaccctccaacag | 59 |
| | | | | tccaaaatgcctatatagtctctaggcagatgagtaaatacc caagtggagagagagtgagataccagtgccggtctcctta cgagatgttcggcgacgaggaagtgatgtgcctgaacgg caattggacagagccaccacaatgtaaggatagtacaggc aaatgtgtgatcagtcgggagatcatggagaactacaacat tgccctgaggtggactgctaagcagaagctctatagccga acaggcgagtcagtggagtttgtgtgtaaacggggataca ggctgtccagtaggagccacaccctgcgcactacttgctg ggatgggaagctggaatatccaacttgc | |
| | CD5-sp+linker+ CR2(SCR1 SCR4)+(G ₄S)₂+linker +FH(SCR1 - SCR4)+lin ker+FH(S CR18)+lin ker+FH(S CR20)+lin ker | MPMGSLQPLATLYLLGML VASCLGISCGSPPPILNGRI SYYSTPIAVGTVIRYSCSGT FRLIGEKSLLCITKDKVDG TWDKPAPKCEYFNKYSSC PEPIVPGGYKIRGSTPYRH GDSVTFACKTNFSMNGNK SVWCQANNMWGPTRLPT CVSVFPLECPALPMIHNGH HTSENVGSIAPGLSVTYSC ESGYLLVGEKIINCLSSGK WSAVPPTCEEARCKSLGR FPNGKVKEPPILRVGVTAN FFCDEGYRLQGPPSSRCVI AGQGVAWTKMPVCGGGG SGGGGSEDCNELPPRRNT EILTGSWSDQTYPEGTQAI YKCRPGYRSLGNVIMVCR KGEWVALNPLRKCQKRPC GHPGDTPFGTFTLTGGNVF EYGVKAVYTCNEGYQLLG EINYRECDTDGWTNDIPIC EVVKCLPVTAPENGKIVSS AMEPDREYHFGQAVRFVC NSGYKIEGDEEMHCSDDG FWSKEKPKCVEISCKSPDV INGSPISQKIIYKENERFQY KCNMGYEYSERGDAVCTE SGWRPLPSCERDTSCVNPP TVQNAYIVSRQMSKYPSG ERVRYQCRSPYEMFGDEE VMCLNGNWTEPPQCKDS TGKCVISREIMENYNIALR WTAKQKLYSRTGESVEFV CKRGYRLSSRSHTLRTTC WDGKLEYPTCAKR | 42 | atgccaatggggtccctacaacccctggcaacactatatct gctaggtatgctcgtggcctcctgtttaggaatttcatgcgg ctctcctccacccattctcaatggaaggatctcctactacag cacccccatagctgtcggcaccgttatccgatacagttgttc cggtactttccggcttatcggcgaaaagtctttgctgtgcatt accaaggataaagtggacgggacttgggacaaacccgca cctaagtgcgagtattttaacaaatatagcagctgccctgag cctatagtacccggggggtataaaatccggggctctactcc ctatcgtcatggcgattctgtgaccttcgcatgtaaaactaat ttttcaatgaatggcaacaagtctgtatggtgtcaagcaaat aacatgtggggacctacccgcctgccaacctgtgtgtcagt gtttcccctggaatgtccagccctccctatgatccacaacg gacatcacaccagcgaaaacgttggctccatcgcaccag ggctctctgtgacttactcttgcgagtccgggtacctgctcg tgggtgaaaagatcatcaactgcctcagtagtggtaaatgg tccgccgtgcctcccacatgtgaagaggcccggtgcaag agcctgggccggttccccaacggaaaagtgaaggaacct cctatcttgagggttggtgtgaccgctaactttttctgcgacg aggggtacaggctccaagggcctccctctagtcggtgcgt aatcgccggtcaaggagtcgcatggactaagatgcctgtg tgtggcggaggtgggtcgggtggcggcggatctgagga ctgcaacgaactgcctcctagaagaaacaccgaaatcctc acaggctcctggtcagatcagacctaccctgagggtacac aggccatctataaatgccgcccgggttaccgctccttgggc aatgttattatggtctgccggaaaggggaatgggtggcctt gaaccccctcagaaagtgccaaaaaagaccctgcggaca tccaggagacacacctttcgggacatttaccttgaccggtg gtaacgtgttcgagtacggagtgaaggcagtctatacttgc aatgagggctaccagctcctgggggaaataaattatcgcg aatgtgatacagacggctggacgaacgacattcctatctgt gaggtggtcaaatgtctgcccgtcactgctcctgaaaacg gcaagatagtgagttctgcaatggagccggatagggagta tcattttggtcaggccgtgcgctttgtgtgcaatagtgggtat aaaatcgagggggatgaagaaatgcactgtagcgacgat ggattctggtctaaagagaaacctaagtgtgtggagattag ttgcaagagtccagacgtgattaacggtagccccatttccc aaaagataatatacaaggaaaacgaaagattccagtataa atgcaacatgggctacgagtatagcgagcggggcgatgc cgtgtgtactgaaagcggctggcgccccctgccttcctgc gagcgggatacaagctgcgtgaaccctccaacagtccaa aatgcctatatagtctctaggcagatgagtaaatacccaagt ggagagagagtgagataccagtgccggtctccttacgag atgttcggcgacgaggaagtgatgtgcctgaacggcaatt ggacagagccaccacaatgtaaggatagtacaggcaaat gtgtgatcagtcgggagatcatggagaactacaacattgc cctgaggtggactgctaagcagaagctctatagccgaaca ggcgagtcagtggagtttgtgtgtaaacggggatacaggc tgtccagtaggagccacaccctgcgcactacttgctgggat gggaagctggaatatccaacttgcgccaagagg | 60 |
| XMDC 062 | CR2(SCR1 -SCR4) | CGSPPPILNGRISYYSTPIA VGTVIRYSCSGTFRLIGEK | 1 | tgcggcagcccccctcccatcctgaacggcagaatcagct actacagcacccccatcgccgtgggcaccgtgatcagata | 27 |
| | | SLLCITKDKVDGTWDKPA PKCEYFNKYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVC | | cagctgcagcggcaccttcagactgatcggcgagaagag cctgctgtgcatcaccaaggacaaggtggacggcacctg ggacaagcccgcccccaagtgcgagtacttcaacaagta cagcagctgccccgagcccatcgtgcctggcggctacaa gatcagaggcagcaccccctacagacacggcgacagcg tgaccttcgcctgcaagaccaacttcagcatgaacggcaa caagagcgtgtggtgccaagccaacaacatgtggggccc cacaagactgcccacctgcgtgagcgtgttccccctggag tgccccgccctgcccatgatccacaacggccaccacaca agcgagaacgtgggcagcatcgcccccggcctgagcgt gacctacagctgcgagagcggctacctgctggtgggcga gaagatcatcaactgcctgagcagcggcaagtggagcgc cgtgccccccacctgcgaggaggctagatgcaagagcct gggcagattccccaacggcaaggtgaaggagcctcctat cctgagagtgggcgtgaccgccaacttcttctgcgacgag ggctacagactgcaaggcccccctagcagcagatgtgtg atcgctggccaaggcgtggcctggaccaaaatgcccgtct gc | |
| | FH(SCR1-SCR4) | CNELPPRRNTEILTGSWSD QTYPEGTQAIYKCRPGYR SLGNVIMVCRKGEWVALN PLRKCQKRPCGHPGDTPF GTFTLTGGNVFEYGVKAV YTCNEGYQLLGEINYREC DTDGWTNDIPICEVVKCLP VTAPENGKIVSSAMEPDRE YHFGQAVRFVCNSGYKIE GDEEMHCSDDGFWSKEK PKCVEISCKSPDVINGSPIS QKIIYKENERFQYKCNMG YEYSERGDAVCTESGWRP LPSC | 2 | tgcaacgagctgccccctagacggaacacagagatcctg accggcagctggagcgatcaaacatatcccgagggcacc caagccatctacaaatgcagacctggctacagaagcctgg gcaatgtgatcatggtctgccggaaaggggagtgggtcg ctctgaaccccctgcggaagtgtcagaagagaccttgcgg ccaccccggcgacacacccttcgggaccttcacactgacc ggcggcaacgtgtttgagtacggcgtcaaggccgtgtaca cctgtaacgaggggtatcagctgctgggcgagatcaacta cagagagtgcgataccgacggctggaccaatgacatccc catctgcgaggtggtgaagtgtctgcccgtgaccgccccc gaaaacggcaagatcgtgagcagcgccatggagcccga ccgggagtaccattttgggcaagctgtgagattcgtctgca atagcggctacaagatcgaaggcgacgaggagatgcact gtagcgacgacggcttctggtccaaagagaaacccaaat gcgtggagatctcctgcaaaagccccgatgtgatcaacgg cagccccatctcccaaaaaattatctacaaggaaaacgaa cggtttcagtacaaatgcaacatgggctacgaatactccga gcggggggatgccgtgtgcaccgagagcggctggcgg cctctgcctagctgt | 28 |
| | | | | tgcaatgaactgcctcctagacggaacaccgaaatcctga ccgggagctggagcgaccaaacataccccgaggggaca caagccatctacaagtgcagacctgggtatagatccctcg ggaacgtgatcatggtctgtcggaagggcgagtgggtcg ctctgaaccctctcagaaagtgtcagaaacggccctgcgg gcaccccggcgatacccccttcggcacattcaccctgacc ggggggaacgtgttcgagtacggcgtgaaggccgtctac acctgcaacgagggctaccaactcctgggcgaaatcaatt acagagaatgcgacacagatggctggaccaacgacatcc ctatctgcgaggtcgtgaaatgtctccccgtgacagcccct gagaacggcaagattgtcagcagcgctatggaacctgata gagagtaccatttcggccaagccgtcagatttgtgtgcaatt ccggctacaaaattgagggggacgaagagatgcactgct ccgacgatggcttctggagcaaggaaaaacccaagtgcg tggagattagctgcaagagccccgacgtgatcaacggga gccccatctcccaaaaaatcatctacaaggagaacgagag atttcagtataagtgcaacatgggctatgagtacagcgagc ggggggacgccgtgtgtaccgaatccgggtggagaccc ctgcctagctgc | 29 |
| | FH(SCR7) | CYFPYLENGYNQNHGRKF VQGKSIDVACHPGYALPK AQTTVTCMENGWSPTPRC | 43 | tgttacttcccctacctggagaacggctacaatcagaacca cggcagaaagttcgtgcaaggcaagagcatcgacgtggc ctgccaccccggctacgccctgcccaaggctcagaccac | 61 |
| | | | | cgtgacctgcatggagaatggctggagccccacccctag atgc | |
| | CR2(SCR1 SCR4)+(G ₄S)₂+linker +FH(SCR1 SCR4)+lin ker+(G₄S)₂ +linker+F H(SCR1-SCR4)+lin ker+(G₄S)₂ +linker+F H(SCR7) | CGSPPPILNGRISYYSTPIA VGTVIRYSCSGTFRLIGEK SLLCITKDKVDGTWDKPA PKCEYFNKYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVCGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCEGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCEGGGGSGGGGS LRKCYFPYLENGYNQNHG RKFVQGKSIDVACHPGYA LPKAQTTVTCMENGWSPT PRC | 44 | tgcggcagcccccctcccatcctgaacggcagaatcagct actacagcacccccatcgccgtgggcaccgtgatcagata cagctgcagcggcaccttcagactgatcggcgagaagag cctgctgtgcatcaccaaggacaaggtggacggcacctg ggacaagcccgcccccaagtgcgagtacttcaacaagta cagcagctgccccgagcccatcgtgcctggcggctacaa gatcagaggcagcaccccctacagacacggcgacagcg tgaccttcgcctgcaagaccaacttcagcatgaacggcaa caagagcgtgtggtgccaagccaacaacatgtggggccc cacaagactgcccacctgcgtgagcgtgttccccctggag tgccccgccctgcccatgatccacaacggccaccacaca agcgagaacgtgggcagcatcgcccccggcctgagcgt gacctacagctgcgagagcggctacctgctggtgggcga gaagatcatcaactgcctgagcagcggcaagtggagcgc cgtgccccccacctgcgaggaggctagatgcaagagcct gggcagattccccaacggcaaggtgaaggagcctcctat cctgagagtgggcgtgaccgccaacttcttctgcgacgag ggctacagactgcaaggcccccctagcagcagatgtgtg atcgctggccaaggcgtggcctggaccaaaatgcccgtct gcgggggggggggcagcgggggcgggggcagcgag gattgcaacgagctgccccctagacggaacacagagatc ctgaccggcagctggagcgatcaaacatatcccgagggc acccaagccatctacaaatgcagacctggctacagaagcc tgggcaatgtgatcatggtctgccggaaaggggagtgggt cgctctgaaccccctgcggaagtgtcagaagagaccttgc ggccaccccggcgacacacccttcgggaccttcacactg accggcggcaacgtgtttgagtacggcgtcaaggccgtgt acacctgtaacgaggggtatcagctgctgggcgagatcaa ctacagagagtgcgataccgacggctggaccaatgacat ccccatctgcgaggtggtgaagtgtctgcccgtgaccgcc cccgaaaacggcaagatcgtgagcagcgccatggagcc cgaccgggagtaccattttgggcaagctgtgagattcgtct gcaatagcggctacaagatcgaaggcgacgaggagatg cactgtagcgacgacggcttctggtccaaagagaaaccca aatgcgtggagatctcctgcaaaagccccgatgtgatcaa cggcagccccatctcccaaaaaattatctacaaggaaaac gaacggtttcagtacaaatgcaacatgggctacgaatactc cgagcggggggatgccgtgtgcaccgagagcggctggc ggcctctgcctagctgtgaagggggcggcgggagcggc gggggcggcagcgaggactgcaatgaactgcctcctaga cggaacaccgaaatcctgaccgggagctggagcgacca aacataccccgaggggacacaagccatctacaagtgcag acctgggtatagatccctcgggaacgtgatcatggtctgtc ggaagggcgagtgggtcgctctgaaccctctcagaaagt gtcagaaacggccctgcgggcaccccggcgataccccct tcggcacattcaccctgaccggggggaacgtgttcgagta cggcgtgaaggccgtctacacctgcaacgagggctacca actcctgggcgaaatcaattacagagaatgcgacacagat ggctggaccaacgacatccctatctgcgaggtcgtgaaat gtctccccgtgacagcccctgagaacggcaagattgtcag cagcgctatggaacctgatagagagtaccatttcggccaa gccgtcagatttgtgtgcaattccggctacaaaattgaggg ggacgaagagatgcactgctccgacgatggcttctggag caaggaaaaacccaagtgcgtggagattagctgcaagag ccccgacgtgatcaacgggagccccatctcccaaaaaatc atctacaaggagaacgagagatttcagtataagtgcaacat gggctatgagtacagcgagcggggggacgccgtgtgta ccgaatccgggtggagacccctgcctagctgcgagggcg | 62 |
| | | | | ggggcgggagcggcgggggcggctccctgagaaagtg ttacttcccctacctggagaacggctacaatcagaaccacg gcagaaagttcgtgcaaggcaagagcatcgacgtggcct gccaccccggctacgccctgcccaaggctcagaccaccg tgacctgcatggagaatggctggagccccacccctagatg c | |
| | CD5-sp+linker+ CR2(SCR1 SCR4)+(G ₄S)₂+linker +FH(SCR1 SCR4)+lin ker+(G₄S)₂ +linker+F H(SCR1-SCR4)+lin ker+(G₄S)₂ +linker+F H(SCR7)+ linker | MPMGSLQPLATLYLLGML VASCLGISCGSPPPILNGRI SYYSTPIAVGTVIRYSCSGT FRLIGEKSLLCITKDKVDG TWDKPAPKCEYFNKYSSC PEPIVPGGYKIRGSTPYRH GDSVTFACKTNFSMNGNK SVWCQANNMWGPTRLPT CVSVFPLECPALPMIHNGH HTSENVGSIAPGLSVTYSC ESGYLLVGEKIINCLSSGK WSAVPPTCEEARCKSLGR FPNGKVKEPPILRVGVTAN FFCDEGYRLQGPPSSRCVI AGQGVAWTKMPVCGGGG SGGGGSEDCNELPPRRNT EILTGSWSDQTYPEGTQAI YKCRPGYRSLGNVIMVCR KGEWVALNPLRKCQKRPC GHPGDTPFGTFTLTGGNVF EYGVKAVYTCNEGYQLLG EINYRECDTDGWTNDIPIC EVVKCLPVTAPENGKIVSS AMEPDREYHFGQAVRFVC NSGYKIEGDEEMHCSDDG FWSKEKPKCVEISCKSPDV INGSPISQKIIYKENERFQY KCNMGYEYSERGDAVCTE SGWRPLPSCEGGGGSGGG GSEDCNELPPRRNTEILTG SWSDQTYPEGTQAIYKCR PGYRSLGNVIMVCRKGEW VALNPLRKCQKRPCGHPG DTPFGTFTLTGGNVFEYG VKAVYTCNEGYQLLGEIN YRECDTDGWTNDIPICEV VKCLPVTAPENGKIVSSA MEPDREYHFGQAVRFVCN SGYKIEGDEEMHCSDDGF WSKEKPKCVEISCKSPDVI NGSPISQKIIYKENERFQY KCNMGYEYSERGDAVCTE SGWRPLPSCEGGGGSGGG GSLRKCYFPYLENGYNQN HGRKFVQGKSIDVACHPG YALPKAQTTVTCMENGW SPTPRCIRVKTY | 45 | atgcccatgggcagcctgcagcccctggccaccctgtacc tgctgggcatgctggtggctagctgcctgggcatcagctg cggcagcccccctcccatcctgaacggcagaatcagcta ctacagcacccccatcgccgtgggcaccgtgatcagatac agctgcagcggcaccttcagactgatcggcgagaagagc ctgctgtgcatcaccaaggacaaggtggacggcacctgg gacaagcccgcccccaagtgcgagtacttcaacaagtac agcagctgccccgagcccatcgtgcctggcggctacaag atcagaggcagcaccccctacagacacggcgacagcgt gaccttcgcctgcaagaccaacttcagcatgaacggcaac aagagcgtgtggtgccaagccaacaacatgtggggcccc acaagactgcccacctgcgtgagcgtgttccccctggagt gccccgccctgcccatgatccacaacggccaccacacaa gcgagaacgtgggcagcatcgcccccggcctgagcgtg acctacagctgcgagagcggctacctgctggtgggcgag aagatcatcaactgcctgagcagcggcaagtggagcgcc gtgccccccacctgcgaggaggctagatgcaagagcctg ggcagattccccaacggcaaggtgaaggagcctcctatc ctgagagtgggcgtgaccgccaacttcttctgcgacgagg gctacagactgcaaggcccccctagcagcagatgtgtgat cgctggccaaggcgtggcctggaccaaaatgcccgtctg cgggggggggggcagcgggggcgggggcagcgagg attgcaacgagctgccccctagacggaacacagagatcct gaccggcagctggagcgatcaaacatatcccgagggcac ccaagccatctacaaatgcagacctggctacagaagcctg ggcaatgtgatcatggtctgccggaaaggggagtgggtc gctctgaaccccctgcggaagtgtcagaagagaccttgcg gccaccccggcgacacacccttcgggaccttcacactga ccggcggcaacgtgtttgagtacggcgtcaaggccgtgta cacctgtaacgaggggtatcagctgctgggcgagatcaac tacagagagtgcgataccgacggctggaccaatgacatc cccatctgcgaggtggtgaagtgtctgcccgtgaccgccc ccgaaaacggcaagatcgtgagcagcgccatggagccc gaccgggagtaccattttgggcaagctgtgagattcgtctg caatagcggctacaagatcgaaggcgacgaggagatgc actgtagcgacgacggcttctggtccaaagagaaacccaa atgcgtggagatctcctgcaaaagccccgatgtgatcaac ggcagccccatctcccaaaaaattatctacaaggaaaacg aacggtttcagtacaaatgcaacatgggctacgaatactcc gagcggggggatgccgtgtgcaccgagagcggctggc ggcctctgcctagctgtgaagggggcggcgggagcggc gggggcggcagcgaggactgcaatgaactgcctcctaga cggaacaccgaaatcctgaccgggagctggagcgacca aacataccccgaggggacacaagccatctacaagtgcag acctgggtatagatccctcgggaacgtgatcatggtctgtc ggaagggcgagtgggtcgctctgaaccctctcagaaagt gtcagaaacggccctgcgggcaccccggcgataccccct tcggcacattcaccctgaccggggggaacgtgttcgagta cggcgtgaaggccgtctacacctgcaacgagggctacca actcctgggcgaaatcaattacagagaatgcgacacagat ggctggaccaacgacatccctatctgcgaggtcgtgaaat gtctccccgtgacagcccctgagaacggcaagattgtcag cagcgctatggaacctgatagagagtaccatttcggccaa gccgtcagatttgtgtgcaattccggctacaaaattgaggg | 63 |
| | | | | ggacgaagagatgcactgctccgacgatggcttctggag caaggaaaaacccaagtgcgtggagattagctgcaagag ccccgacgtgatcaacgggagccccatctcccaaaaaatc atctacaaggagaacgagagatttcagtataagtgcaacat gggctatgagtacagcgagcggggggacgccgtgtgta ccgaatccgggtggagacccctgcctagctgcgagggcgg gggcgggagcggcgggggcggctccctgagaaagtg ttacttcccctacctggagaacggctacaatcagaaccacg gcagaaagttcgtgcaaggcaagagcatcgacgtggcct gccaccccggctacgccctgcccaaggctcagaccaccg tgacctgcatggagaatggctggagccccacccctagatg catcagagtgaagacctac | |
| XMDC 025-(CR2-R36A K41A K67A) | CR2(SCR1 -SCR4)-(R36A K41A K67A) | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVC | 46 | tgtggcagccctcctcctattctgaatggcaggatcagcta ctacagcacccctattgccgtgggcaccgtgatcagatac agctgctctggcacattcgccctgatcggcgaagccagcc tgctgtgcatcaccaaggataaggtggacggcacctggg acaagcccgctcctaagtgcgaatatttcaacgcctacagc agctgccctgagcctattgtgcctggcggctacaagatca gaggcagcaccccatacagacacggcgacagcgtgacc tttgcctgcaagaccaacttcagcatgaacggcaacaaga gcgtgtggtgccaggccaacaatatgtggggccctacca gactgcccacctgtgtgtctgtgttccctctggaatgccccg ctctgcccatgatccacaatggccaccacaccagcgagaa cgtgggatctattgcccctggcctgtccgtgacctacagct gtgaaagcggctatctgctcgtgggcgagaagatcatcaa ctgcctgagcagcggcaagtggagcgctgttcctcctaca tgcgaagaggccagatgcaagagcctgggcagattcccc aacggcaaagtgaaagagcctcctatcctgagagtgggc gtgaccgccaacttcttctgcgacgagggctacagactgc agggccctcctagcagcagatgtgtgattgctggacaggg cgtcgcctggaccaagatgcctgtttgt | 64 |
| | FH(SCR1-SCR4) | CNELPPRRNTEILTGSWSD QTYPEGTQAIYKCRPGYR SLGNVIMVCRKGEWVALN PLRKCQKRPCGHPGDTPF GTFTLTGGNVFEYGVKAV YTCNEGYQLLGEINYREC DTDGWTNDIPICEVVKCLP VTAPENGKIVSSAMEPDRE YHFGQAVRFVCNSGYKIE GDEEMHCSDDGFWSKEK PKCVEISCKSPDVINGSPIS QKIIYKENERFQYKCNMG YEYSERGDAVCTESGWRP LPSC | 2 | tgcaacgaactgcctcctagaagaaacaccgaaatcctca caggctcctggtcagatcagacctaccctgagggtacaca ggccatctataaatgccgcccgggttaccgctccttgggca atgttattatggtctgccggaaaggggaatgggtggccttg aaccccctcagaaagtgccaaaaaagaccctgcggacat ccaggagacacacctttcgggacatttaccttgaccggtgg taacgtgttcgagtacggagtgaaggcagtctatacttgca atgagggctaccagctcctgggggaaataaattatcgcga atgtgatacagacggctggacgaacgacattcctatctgtg aggtggtcaaatgtctgcccgtcactgctcctgaaaacggc aagatagtgagttctgcaatggagccggatagggagtatc attttggtcaggccgtgcgctttgtgtgcaatagtgggtata aaatcgagggggatgaagaaatgcactgtagcgacgatg gattctggtctaaagagaaacctaagtgtgtggagattagtt gcaagagtccagacgtgattaacggtagccccatttccca aaagataatatacaaggaaaacgaaagattccagtataaat gcaacatgggctacgagtatagcgagcggggcgatgcc gtgtgtactgaaagcggctggcgccccctgccttcctgc | 19 |
| | FH(SCR8) | CSKSSIDIENGFISESQYTY ALKEKAKYQCKLGYVTA DGETSGSITCGKDGWSAQ PTC | 3 | tgttccaaaagcagcatcgacatcgaaaacggctttatatc cgagagccagtatacgtatgcgctgaaggaaaaggccaa gtatcagtgcaagctcggttatgtcacagctgatggcgaga cctccgggtctatcacatgcggcaaagacggctggtccgc tcagcctacctgc | 20 |
| | FH(SCR19-SCR20) | CGPPPPIDNGDITSFPLSVY APASSVEYQCQNLYQLEG NKRITCRNGQWSEPPKCL HPCVISREIMENYNIALRW TAKQKLYSRTGESVEFVC | 4 | tgtgggccacctcccccaattgataatggtgatataacctcc ttccctcttagtgtttacgccccagcatccagcgtggagtat caatgccaaaatctgtaccaactggaggggaacaaacgg ataacctgccgcaacggccaatggtccgagccacctaagt gcctgcatccatgtgtgatcagtcgggagatcatggagaa | 21 |
| | | KRGYRLSSRSHTLRTTCW DGKLEYPTC | | ctacaacattgccctgaggtggactgctaagcagaagctct atagccgaacaggcgagtcagtggagtttgtgtgtaaacg gggatacaggctgtccagtaggagccacaccctgcgcac tacttgctgggatgggaagctggaatatccaacttgc | |
| | CR2(SCR1 SCR4)+(G₄ S)₂+linker+ FH(SCR1-SCR4)+link er+FH(SCR 8)+linker+F H(SCR19-SCR20) | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVCGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCEIRVKTCSKSSID IENGFISESQYTYALKEKA KYQCKLGYVTADGETSGS ITCGKDGWSAQPTCIKSDS TGKCGPPPPIDNGDITSFPL SVYAPASSVEYQCQNLYQ LEGNKRITCRNGQWSEPP KCLHPCVISREIMENYNIA LRWTAKQKLYSRTGESVE FVCKRGYRLSSRSHTLRTT CWDGKLEYPTC | 47 | tgtggcagccctcctcctattctgaatggcaggatcagcta ctacagcacccctattgccgtgggcaccgtgatcagatac agctgctctggcacattcgccctgatcggcgaagccagcc tgctgtgcatcaccaaggataaggtggacggcacctggg acaagcccgctcctaagtgcgaatatttcaacgcctacagc agctgccctgagcctattgtgcctggcggctacaagatca gaggcagcaccccatacagacacggcgacagcgtgacc tttgcctgcaagaccaacttcagcatgaacggcaacaaga gcgtgtggtgccaggccaacaatatgtggggccctacca gactgcccacctgtgtgtctgtgttccctctggaatgccccg ctctgcccatgatccacaatggccaccacaccagcgagaa cgtgggatctattgcccctggcctgtccgtgacctacagct gtgaaagcggctatctgctcgtgggcgagaagatcatcaa ctgcctgagcagcggcaagtggagcgctgttcctcctaca tgcgaagaggccagatgcaagagcctgggcagattcccc aacggcaaagtgaaagagcctcctatcctgagagtgggc gtgaccgccaacttcttctgcgacgagggctacagactgc agggccctcctagcagcagatgtgtgattgctggacaggg cgtcgcctggaccaagatgcctgtttgtggcggaggtggg tcgggtggcggcggatctgaggactgcaacgaactgcct cctagaagaaacaccgaaatcctcacaggctcctggtcag atcagacctaccctgagggtacacaggccatctataaatgc cgcccgggttaccgctccttgggcaatgttattatggtctgc cggaaaggggaatgggtggccttgaaccccctcagaaag tgccaaaaaagaccctgcggacatccaggagacacacct ttcgggacatttaccttgaccggtggtaacgtgttcgagtac ggagtgaaggcagtctatacttgcaatgagggctaccagc tcctgggggaaataaattatcgcgaatgtgatacagacgg ctggacgaacgacattcctatctgtgaggtggtcaaatgtct gcccgtcactgctcctgaaaacggcaagatagtgagttctg caatggagccggatagggagtatcattttggtcaggccgt gcgctttgtgtgcaatagtgggtataaaatcgagggggatg aagaaatgcactgtagcgacgatggattctggtctaaaga gaaacctaagtgtgtggagattagttgcaagagtccagac gtgattaacggtagccccatttcccaaaagataatatacaa ggaaaacgaaagattccagtataaatgcaacatgggctac gagtatagcgagcggggcgatgccgtgtgtactgaaagc ggctggcgccccctgccttcctgcgagattagggtgaaga cctgttccaaaagcagcatcgacatcgaaaacggctttata tccgagagccagtatacgtatgcgctgaaggaaaaggcc aagtatcagtgcaagctcggttatgtcacagctgatggcga gacctccgggtctatcacatgcggcaaagacggctggtcc gctcagcctacctgcattaagagcgatagtacaggcaaat gtgggccacctcccccaattgataatggtgatataacctcct tccctcttagtgtttacgccccagcatccagcgtggagtatc aatgccaaaatctgtaccaactggaggggaacaaacggat aacctgccgcaacggccaatggtccgagccacctaagtg cctgcatccatgtgtgatcagtcgggagatcatggagaact acaacattgccctgaggtggactgctaagcagaagctctat agccgaacaggcgagtcagtggagtttgtgtgtaaacgg ggatacaggctgtccagtaggagccacaccctgcgcact acttgctgggatgggaagctggaatatccaacttgc | 65 |
| | CD5-sp+linker+ CR2(SCR1 | MPMGSLQPLATLYLLGML VASCLGISCGSPPPILNGRI SYYSTPIAVGTVIRYSCSGT FALIGEASLLCITKDKVDG | 48 | atgccaatggggtccctacaacccctggcaacactatatct gctaggtatgctcgtggcctcctgtttaggaatcagctgtgg cagccctcctcctattctgaatggcaggatcagctactaca gcacccctattgccgtgggcaccgtgatcagatacagctg | 66 |
| | SCR4)+(G₄ S)₂+linker+ FH(SCR1-SCR4)+link er+FH(SCR 8)+linker+F H(SCR19-SCR20)-+linker | TWDKPAPKCEYFNAYSSC PEPIVPGGYKIRGSTPYRH GDSVTFACKTNFSMNGNK SVWCQANNMWGPTRLPT CVSVFPLECPALPMIHNGH HTSENVGSIAPGLSVTYSC ESGYLLVGEKIINCLSSGK WSAVPPTCEEARCKSLGR FPNGKVKEPPILRVGVTAN FFCDEGYRLQGPPSSRCVI AGQGVAWTKMPVCGGGG SGGGGSEDCNELPPRRNT EILTGSWSDQTYPEGTQAI YKCRPGYRSLGNVIMVCR KGEWVALNPLRKCQKRPC GHPGDTPFGTFTLTGGNVF EYGVKAVYTCNEGYQLLG EINYRECDTDGWTNDIPIC EVVKCLPVTAPENGKIVSS AMEPDREYHFGQAVRFVC NSGYKIEGDEEMHCSDDG FWSKEKPKCVEISCKSPDV INGSPISQKIIYKENERFQY KCNMGYEYSERGDAVCTE SGWRPLPSCEIRVKTCSKS SIDIENGFISESQYTYALKE KAKYQCKLGYVTADGETS GSITCGKDGWSAQPTCIKS DSTGKCGPPPPIDNGDITSF PLSVYAPASSVEYQCQNLY QLEGNKRITCRNGQWSEP PKCLHPCVISREIMENYNI ALRWTAKQKLYSRTGESV EFVCKRGYRLSSRSHTLRT TCWDGKLEYPTCAKR | | ctctggcacattcgccctgatcggcgaagccagcctgctgt gcatcaccaaggataaggtggacggcacctgggacaag cccgctcctaagtgcgaatatttcaacgcctacagcagctg ccctgagcctattgtgcctggcggctacaagatcagaggc agcaccccatacagacacggcgacagcgtgacctttgcct gcaagaccaacttcagcatgaacggcaacaagagcgtgt ggtgccaggccaacaatatgtggggccctaccagactgc ccacctgtgtgtctgtgttccctctggaatgccccgctctgc ccatgatccacaatggccaccacaccagcgagaacgtgg gatctattgcccctggcctgtccgtgacctacagctgtgaa agcggctatctgctcgtgggcgagaagatcatcaactgcc tgagcagcggcaagtggagcgctgttcctcctacatgcga agaggccagatgcaagagcctgggcagattccccaacg gcaaagtgaaagagcctcctatcctgagagtgggcgtgac cgccaacttcttctgcgacgagggctacagactgcagggc cctcctagcagcagatgtgtgattgctggacagggcgtcg cctggaccaagatgcctgtttgtggcggaggtgggtcggg tggcggcggatctgaggactgcaacgaactgcctcctaga agaaacaccgaaatcctcacaggctcctggtcagatcaga cctaccctgagggtacacaggccatctataaatgccgccc gggttaccgctccttgggcaatgttattatggtctgccggaa aggggaatgggtggccttgaaccccctcagaaagtgcca aaaaagaccctgcggacatccaggagacacacctttcgg gacatttaccttgaccggtggtaacgtgttcgagtacggag tgaaggcagtctatacttgcaatgagggctaccagctcctgg gggaaataaattatcgcgaatgtgatacagacggctgga cgaacgacattcctatctgtgaggtggtcaaatgtctgccc gtcactgctcctgaaaacggcaagatagtgagttctgcaat ggagccggatagggagtatcattttggtcaggccgtgcgc tttgtgtgcaatagtgggtataaaatcgagggggatgaaga aatgcactgtagcgacgatggattctggtctaaagagaaac ctaagtgtgtggagattagttgcaagagtccagacgtgatt aacggtagccccatttcccaaaagataatatacaaggaaa acgaaagattccagtataaatgcaacatgggctacgagtat agcgagcggggcgatgccgtgtgtactgaaagcggctg gcgccccctgccttcctgcgagattagggtgaagacctgtt ccaaaagcagcatcgacatcgaaaacggctttatatccga gagccagtatacgtatgcgctgaaggaaaaggccaagtat cagtgcaagctcggttatgtcacagctgatggcgagacct ccgggtctatcacatgcggcaaagacggctggtccgctca gcctacctgcattaagagcgatagtacaggcaaatgtggg ccacctcccccaattgataatggtgatataacctccttccctc ttagtgtttacgccccagcatccagcgtggagtatcaatgc caaaatctgtaccaactggaggggaacaaacggataacct gccgcaacggccaatggtccgagccacctaagtgcctgc atccatgtgtgatcagtcgggagatcatggagaactacaac attgccctgaggtggactgctaagcagaagctctatagcc gaacaggcgagtcagtggagtttgtgtgtaaacggggata caggctgtccagtaggagccacaccctgcgcactacttgc tgggatgggaagctggaatatccaacttgcgccaagagg | |
| XMDC 026-(CR2-R36A K41A K67A) | CR2(SCR1 -SCR4)-(R36A K41A K67A) | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP | 46 | tgtggcagccctcctcctattctgaatggcaggatcagcta ctacagcacccctattgccgtgggcaccgtgatcagatac agctgctctggcacattcgccctgatcggcgaagccagcc tgctgtgcatcaccaaggataaggtggacggcacctggg acaagcccgctcctaagtgcgaatatttcaacgcctacagc agctgccctgagcctattgtgcctggcggctacaagatca gaggcagcaccccatacagacacggcgacagcgtgacc tttgcctgcaagaccaacttcagcatgaacggcaacaaga gcgtgtggtgccaggccaacaatatgtggggccctacca gactgcccacctgtgtgtctgtgttccctctggaatgccccg | 64 |
| | | TCEEARCKSLGRFPNGKV KEPPILRVGVTANFFCDEG YRLQGPPSSRCVIAGQGVA WTKMPVC | | ctctgcccatgatccacaatggccaccacaccagcgagaa cgtgggatctattgcccctggcctgtccgtgacctacagct gtgaaagcggctatctgctcgtgggcgagaagatcatcaa ctgcctgagcagcggcaagtggagcgctgttcctcctaca tgcgaagaggccagatgcaagagcctgggcagattcccc aacggcaaagtgaaagagcctcctatcctgagagtgggc gtgaccgccaacttcttctgcgacgagggctacagactgc agggccctcctagcagcagatgtgtgattgctggacaggg cgtcgcctggaccaagatgcctgtttgt | |
| | FH(SCR1-SCR4) | CNELPPRRNTEILTGSWSD QTYPEGTQAIYKCRPGYR SLGNVIMVCRKGEWVALN PLRKCQKRPCGHPGDTPF GTFTLTGGNVFEYGVKAV YTCNEGYQLLGEINYREC DTDGWTNDIPICEVVKCLP VTAPENGKIVSSAMEPDRE YHFGQAVRFVCNSGYKIE GDEEMHCSDDGFWSKEK PKCVEISCKSPDVINGSPIS QKIIYKENERFQYKCNMG YEYSERGDAVCTESGWRP LPSC | 2 | tgcaacgaactgcctcctagaagaaacaccgaaatcctca caggctcctggtcagatcagacctaccctgagggtacaca ggccatctataaatgccgcccgggttaccgctccttgggca atgttattatggtctgccggaaaggggaatgggtggccttg aaccccctcagaaagtgccaaaaaagaccctgcggacat ccaggagacacacctttcgggacatttaccttgaccggtgg taacgtgttcgagtacggagtgaaggcagtctatacttgca atgagggctaccagctcctgggggaaataaattatcgcga atgtgatacagacggctggacgaacgacattcctatctgtg aggtggtcaaatgtctgcccgtcactgctcctgaaaacggc aagatagtgagttctgcaatggagccggatagggagtatc attttggtcaggccgtgcgctttgtgtgcaatagtgggtata aaatcgagggggatgaagaaatgcactgtagcgacgatg gattctggtctaaagagaaacctaagtgtgtggagattagtt gcaagagtccagacgtgattaacggtagccccatttccca aaagataatatacaaggaaaacgaaagattccagtataaat gcaacatgggctacgagtatagcgagcggggcgatgcc gtgtgtactgaaagcggctggcgccccctgccttcctgc | 19 |
| | FH(SCR18-SCR20) | CVNPPTVQNAYIVSRQMS KYPSGERVRYQCRSPYEM FGDEEVMCLNGNWTEPPQ CKDSTGKCGPPPPIDNGDI TSFPLSVYAPASSVEYQCQ NLYQLEGNKRITCRNGQW SEPPKCLHPCVISREIMEN YNIALRWTAKQKLYSRTG ESVEFVCKRGYRLSSRSHT LRTTCWDGKLEYPTC | 7 | tgcgtgaaccctccaacagtccaaaatgcctatatagtctct aggcagatgagtaaatacccaagtggagagagagtgaga taccagtgccggtctccttacgagatgttcggcgacgagg aagtgatgtgcctgaacggcaattggacagagccaccac aatgtaaggatagtacaggcaaatgtgggccacctccccc aattgataatggtgatataacctccttccctcttagtgtttacg ccccagcatccagcgtggagtatcaatgccaaaatctgtac caactggaggggaacaaacggataacctgccgcaacgg ccaatggtccgagccacctaagtgcctgcatccatgtgtga tcagtcgggagatcatggagaactacaacattgccctgag gtggactgctaagcagaagctctatagccgaacaggcga gtcagtggagtttgtgtgtaaacggggatacaggctgtcca gtaggagccacaccctgcgcactacttgctgggatgggaa gctggaatatccaacttgc | 24 |
| | CR2(SCR1 SCR4)+(G₄ S)₂+linker+ FH(SCR1-SCR4)+link er+FH(SCR 18-SCR20) | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVCGGGGSGGGGS EDCNELPPRRNTEILTGSW SDQTYPEGTQAIYKCRPG YRSLGNVIMVCRKGEWV ALNPLRKCQKRPCGHPGD [?] | 49 | tgtggcagccctcctcctattctgaatggcaggatcagcta ctacagcacccctattgccgtgggcaccgtgatcagatac agctgctctggcacattcgccctgatcggcgaagccagcc tgctgtgcatcaccaaggataaggtggacggcacctggg acaagcccgctcctaagtgcgaatatttcaacgcctacagc agctgccctgagcctattgtgcctggcggctacaagatca gaggcagcaccccatacagacacggcgacagcgtgacc tttgcctgcaagaccaacttcagcatgaacggcaacaaga gcgtgtggtgccaggccaacaatatgtggggccctacca gactgcccacctgtgtgtctgtgttccctctggaatgccccg ctctgcccatgatccacaatggccaccacaccagcgagaa cgtgggatctattgcccctggcctgtccgtgacctacagct gtgaaagcggctatctgctcgtgggcgagaagatcatcaa ctgcctgagcagcggcaagtggagcgctgttcctcctaca tgcgaagaggccagatgcaagagcctgggcagattcccc aacggcaaagtgaaagagcctcctatcctgagagtgggc gtgaccgccaacttcttctgcgacgagggctacagactgc agggccctcctagcagcagatgtgtgattgctggacaggg cgtcgcctggaccaagatgcctgtttgtggcggaggtggg | 67 |
| | | KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCERDTSCVNPPTV QNAYIVSRQMSKYPSGER VRYQCRSPYEMFGDEEVM CLNGNWTEPPQCKDSTGK CGPPPPIDNGDITSFPLSVY APASSVEYQCQNLYQLEG NKRITCRNGQWSEPPKCL HPCVISREIMENYNIALRW TAKQKLYSRTGESVEFVC KRGYRLSSRSHTLRTTCW DGKLEYPTC | | tcgggtggcggcggatctgaggactgcaacgaactgcct cctagaagaaacaccgaaatcctcacaggctcctggtcag atcagacctaccctgagggtacacaggccatctataaatgc cgcccgggttaccgctccttgggcaatgttattatggtctgc cggaaaggggaatgggtggccttgaaccccctcagaaag tgccaaaaaagaccctgcggacatccaggagacacacct ttcgggacatttaccttgaccggtggtaacgtgttcgagtac ggagtgaaggcagtctatacttgcaatgagggctaccagc tcctgggggaaataaattatcgcgaatgtgatacagacgg ctggacgaacgacattcctatctgtgaggtggtcaaatgtct gcccgtcactgctcctgaaaacggcaagatagtgagttctg caatggagccggatagggagtatcattttggtcaggccgt gcgctttgtgtgcaatagtgggtataaaatcgagggggatg aagaaatgcactgtagcgacgatggattctggtctaaaga gaaacctaagtgtgtggagattagttgcaagagtccagac gtgattaacggtagccccatttcccaaaagataatatacaa ggaaaacgaaagattccagtataaatgcaacatgggctac gagtatagcgagcggggcgatgccgtgtgtactgaaagc ggctggcgccccctgccttcctgcgagcgggatacaagct gcgtgaaccctccaacagtccaaaatgcctatatagtctcta ggcagatgagtaaatacccaagtggagagagagtgagat accagtgccggtctccttacgagatgttcggcgacgagga agtgatgtgcctgaacggcaattggacagagccaccaca atgtaaggatagtacaggcaaatgtgggccacctccccca attgataatggtgatataacctccttccctcttagtgtttacgc cccagcatccagcgtggagtatcaatgccaaaatctgtacc aactggaggggaacaaacggataacctgccgcaacggc caatggtccgagccacctaagtgcctgcatccatgtgtgat cagtcgggagatcatggagaactacaacattgccctgagg tggactgctaagcagaagctctatagccgaacaggcgagt cagtggagtttgtgtgtaaacggggatacaggctgtccagt aggagccacaccctgcgcactacttgctgggatgggaag ctggaatatccaacttgc | |
| | CD5-sp+linker+ CR2(SCR1 SCR4)+(G₄ S)₂+linker+ FH(SCR1-SCR4)+link er+FH(SCR 18-SCR20)+lin ker | MPMGSLQPLATLYLLGML VASCLGISCGSPPPILNGRI SYYSTPIAVGTVIRYSCSGT FALIGEASLLCITKDKVDG TWDKPAPKCEYFNAYSSC PEPIVPGGYKIRGSTPYRH GDSVTFACKTNFSMNGNK SVWCQANNMWGPTRLPT CVSVFPLECPALPMIHNGH HTSENVGSIAPGLSVTYSC ESGYLLVGEKIINCLSSGK WSAVPPTCEEARCKSLGR FPNGKVKEPPILRVGVTAN FFCDEGYRLQGPPSSRCVI AGQGVAWTKMPVCGGGG SGGGGSEDCNELPPRRNT EILTGSWSDQTYPEGTQAI YKCRPGYRSLGNVIMVCR KGEWVALNPLRKCQKRPC GHPGDTPFGTFTLTGGNVF EYGVKAVYTCNEGYQLLG EINYRECDTDGWTNDIPIC EVVKCLPVTAPENGKIVSS AMEPDREYHFGQAVRFVC NSGYKIEGDEEMHCSDDG FWSKEKPKCVEISCKSPDV INGSPISQKIIYKENERFQY | 50 | atgccaatggggtccctacaacccctggcaacactatatct gctaggtatgctcgtggcctcctgtttaggaatcagctgtgg cagccctcctcctattctgaatggcaggatcagctactaca gcacccctattgccgtgggcaccgtgatcagatacagctg ctctggcacattcgccctgatcggcgaagccagcctgctgt gcatcaccaaggataaggtggacggcacctgggacaag cccgctcctaagtgcgaatatttcaacgcctacagcagctg ccctgagcctattgtgcctggcggctacaagatcagaggc agcaccccatacagacacggcgacagcgtgacctttgcct gcaagaccaacttcagcatgaacggcaacaagagcgtgt ggtgccaggccaacaatatgtggggccctaccagactgc ccacctgtgtgtctgtgttccctctggaatgccccgctctgc ccatgatccacaatggccaccacaccagcgagaacgtgg gatctattgcccctggcctgtccgtgacctacagctgtgaa agcggctatctgctcgtgggcgagaagatcatcaactgcc tgagcagcggcaagtggagcgctgttcctcctacatgcga agaggccagatgcaagagcctgggcagattccccaacg gcaaagtgaaagagcctcctatcctgagagtgggcgtgac cgccaacttcttctgcgacgagggctacagactgcagggc cctcctagcagcagatgtgtgattgctggacagggcgtcg cctggaccaagatgcctgtttgtggcggaggtgggtcggg tggcggcggatctgaggactgcaacgaactgcctcctaga agaaacaccgaaatcctcacaggctcctggtcagatcaga cctaccctgagggtacacaggccatctataaatgccgccc gggttaccgctccttgggcaatgttattatggtctgccggaa aggggaatgggtggccttgaaccccctcagaaagtgcca aaaaagaccctgcggacatccaggagacacacctttcgg | 68 |
| | | KCNMGYEYSERGDAVCTE SGWRPLPSCERDTSCVNPP TVQNAYIVSRQMSKYPSG ERVRYQCRSPYEMFGDEE VMCLNGNWTEPPQCKDS TGKCGPPPPIDNGDITSFPL SVYAPASSVEYQCQNLYQ LEGNKRITCRNGQWSEPP KCLHPCVISREIMENYNIA LRWTAKQKLYSRTGESVE FVCKRGYRLSSRSHTLRTT CWDGKLEYPTCAKR | | gacatttaccttgaccggtggtaacgtgttcgagtacggag tgaaggcagtctatacttgcaatgagggctaccagctcctg ggggaaataaattatcgcgaatgtgatacagacggctgga cgaacgacattcctatctgtgaggtggtcaaatgtctgccc gtcactgctcctgaaaacggcaagatagtgagttctgcaat ggagccggatagggagtatcattttggtcaggccgtgcgc tttgtgtgcaatagtgggtataaaatcgagggggatgaaga aatgcactgtagcgacgatggattctggtctaaagagaaac ctaagtgtgtggagattagttgcaagagtccagacgtgatt aacggtagccccatttcccaaaagataatatacaaggaaa acgaaagattccagtataaatgcaacatgggctacgagtat agcgagcggggcgatgccgtgtgtactgaaagcggctg gcgccccctgccttcctgcgagcgggatacaagctgcgtg aaccctccaacagtccaaaatgcctatatagtctctaggca gatgagtaaatacccaagtggagagagagtgagatacca gtgccggtctccttacgagatgttcggcgacgaggaagtg atgtgcctgaacggcaattggacagagccaccacaatgta aggatagtacaggcaaatgtgggccacctcccccaattga taatggtgatataacctccttccctcttagtgtttacgcccca gcatccagcgtggagtatcaatgccaaaatctgtaccaact ggaggggaacaaacggataacctgccgcaacggccaat ggtccgagccacctaagtgcctgcatccatgtgtgatcagt cgggagatcatggagaactacaacattgccctgaggtgga ctgctaagcagaagctctatagccgaacaggcgagtcagt ggagtttgtgtgtaaacggggatacaggctgtccagtagg agccacaccctgcgcactacttgctgggatgggaagctgg aatatccaacttgcgccaagagg | |
| XMDC 029-(CR2-R36A K41A K67A) | CR2(SCR1 -SCR4)-(R36A K41A K67A) | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVC | 46 | tgcggcagcccccctcccatcctgaacggcagaatcagct actacagcacccccatcgccgtgggcaccgtgatcagata cagctgcagcggcaccttcgccctgatcggcgaggctag cctgctgtgcatcaccaaggacaaggtggacggcacctg ggacaagcccgcccccaagtgcgagtacttcaacgccta cagcagctgccccgagcccatcgtgcctggcggctacaa gatcagaggcagcaccccctacagacacggcgacagcg tgaccttcgcctgcaagaccaacttcagcatgaacggcaa caagagcgtgtggtgccaagccaacaacatgtggggccc cacaagactgcccacctgcgtgagcgtgttccccctggag tgccccgccctgcccatgatccacaacggccaccacaca agcgagaacgtgggcagcatcgcccccggcctgagcgt gacctacagctgcgagagcggctacctgctggtgggcga gaagatcatcaactgcctgagcagcggcaagtggagcgc cgtgccccccacctgcgaggaggctagatgcaagagcct gggcagattccccaacggcaaggtgaaggagcctcctat cctgagagtgggcgtgaccgccaacttcttctgcgacgag ggctacagactgcaaggcccccctagcagcagatgtgtg atcgctggccaaggcgtggcctggaccaaaatgcccgtct gc | 69 |
| | FH(SCR1-SCR4) | CNELPPRRNTEILTGSWSD QTYPEGTQAIYKCRPGYR SLGNVIMVCRKGEWVALN PLRKCQKRPCGHPGDTPF GTFTLTGGNVFEYGVKAV YTCNEGYQLLGEINYREC DTDGWTNDIPICEVVKCLP VTAPENGKIVSSAMEPDRE YHFGQAVRFVCNSGYKIE GDEEMHCSDDGFWSKEK PKCVEISCKSPDVINGSPIS QKIIYKENERFQYKCNMG YEYSERGDAVCTESGWRP | 2 | tgcaacgagctgccccctagacggaacacagagatcctg accggcagctggagcgatcaaacatatcccgagggcacc caagccatctacaaatgcagacctggctacagaagcctgg gcaatgtgatcatggtctgccggaaaggggagtgggtcg ctctgaaccccctgcggaagtgtcagaagagaccttgcgg ccaccccggcgacacacccttcgggaccttcacactgacc ggcggcaacgtgtttgagtacggcgtcaaggccgtgtaca cctgtaacgaggggtatcagctgctgggcgagatcaacta cagagagtgcgataccgacggctggaccaatgacatccc catctgcgaggtggtgaagtgtctgcccgtgaccgccccc gaaaacggcaagatcgtgagcagcgccatggagcccga ccgggagtaccattttgggcaagctgtgagattcgtctgca atagcggctacaagatcgaaggcgacgaggagatgcact | 28 |
| | | LPSC | | gtagcgacgacggcttctggtccaaagagaaacccaaat gcgtggagatctcctgcaaaagccccgatgtgatcaacgg cagccccatctcccaaaaaattatctacaaggaaaacgaa cggtttcagtacaaatgcaacatgggctacgaatactccga gcggggggatgccgtgtgcaccgagagcggctggcgg cctctgcctagctgt | |
| | | | | tgcaatgaactgcctcctagacggaacaccgaaatcctga ccgggagctggagcgaccaaacataccccgaggggaca caagccatctacaagtgcagacctgggtatagatccctcg ggaacgtgatcatggtctgtcggaagggcgagtgggtcg ctctgaaccctctcagaaagtgtcagaaacggccctgcgg gcaccccggcgatacccccttcggcacattcaccctgacc ggggggaacgtgttcgagtacggcgtgaaggccgtctac acctgcaacgagggctaccaactcctgggcgaaatcaatt acagagaatgcgacacagatggctggaccaacgacatcc ctatctgcgaggtcgtgaaatgtctccccgtgacagcccct gagaacggcaagattgtcagcagcgctatggaacctgata gagagtaccatttcggccaagccgtcagatttgtgtgcaatt ccggctacaaaattgagggggacgaagagatgcactgct ccgacgatggcttctggagcaaggaaaaacccaagtgcg tggagattagctgcaagagccccgacgtgatcaacggga gccccatctcccaaaaaatcatctacaaggagaacgagag atttcagtataagtgcaacatgggctatgagtacagcgagc ggggggacgccgtgtgtaccgaatccgggtggagaccc ctgcctagctgc | 29 |
| | CR2(SCR1 SCR4)+(G ₄S)₂+linker +FH(SCR1 - SCR4)+lin ker+(G₄S)₂ +linker+F H(SCR1-SCR4) | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVCGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCEGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV | 51 | tgcggcagcccccctcccatcctgaacggcagaatcagct actacagcacccccatcgccgtgggcaccgtgatcagata cagctgcagcggcaccttcgccctgatcggcgaggctag cctgctgtgcatcaccaaggacaaggtggacggcacctg ggacaagcccgcccccaagtgcgagtacttcaacgccta cagcagctgccccgagcccatcgtgcctggcggctacaa gatcagaggcagcaccccctacagacacggcgacagcg tgaccttcgcctgcaagaccaacttcagcatgaacggcaa caagagcgtgtggtgccaagccaacaacatgtggggccc cacaagactgcccacctgcgtgagcgtgttccccctggag tgccccgccctgcccatgatccacaacggccaccacaca agcgagaacgtgggcagcatcgcccccggcctgagcgt gacctacagctgcgagagcggctacctgctggtgggcga gaagatcatcaactgcctgagcagcggcaagtggagcgc cgtgccccccacctgcgaggaggctagatgcaagagcct gggcagattccccaacggcaaggtgaaggagcctcctat cctgagagtgggcgtgaccgccaacttcttctgcgacgag ggctacagactgcaaggcccccctagcagcagatgtgtg atcgctggccaaggcgtggcctggaccaaaatgcccgtct gcgggggggggggcagcgggggcgggggcagcgag gattgcaacgagctgccccctagacggaacacagagatc ctgaccggcagctggagcgatcaaacatatcccgagggc acccaagccatctacaaatgcagacctggctacagaagcc tgggcaatgtgatcatggtctgccggaaaggggagtgggt cgctctgaaccccctgcggaagtgtcagaagagaccttgc ggccaccccggcgacacacccttcgggaccttcacactg accggcggcaacgtgtttgagtacggcgtcaaggccgtgt acacctgtaacgaggggtatcagctgctgggcgagatcaa ctacagagagtgcgataccgacggctggaccaatgacat ccccatctgcgaggtggtgaagtgtctgcccgtgaccgcc cccgaaaacggcaagatcgtgagcagcgccatggagcc cgaccgggagtaccattttgggcaagctgtgagattcgtct gcaatagcggctacaagatcgaaggcgacgaggagatg cactgtagcgacgacggcttctggtccaaagagaaaccca aatgcgtggagatctcctgcaaaagccccgatgtgatcaa | 70 |
| | | KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSC | | cggcagccccatctcccaaaaaattatctacaaggaaaac gaacggtttcagtacaaatgcaacatgggctacgaatactc cgagcggggggatgccgtgtgcaccgagagcggctggc ggcctctgcctagctgtgaagggggcggcgggagcggc gggggcggcagcgaggactgcaatgaactgcctcctaga cggaacaccgaaatcctgaccgggagctggagcgacca aacataccccgaggggacacaagccatctacaagtgcag acctgggtatagatccctcgggaacgtgatcatggtctgtc ggaagggcgagtgggtcgctctgaaccctctcagaaagt gtcagaaacggccctgcgggcaccccggcgataccccct tcggcacattcaccctgaccggggggaacgtgttcgagta cggcgtgaaggccgtctacacctgcaacgagggctacca actcctgggcgaaatcaattacagagaatgcgacacagat ggctggaccaacgacatccctatctgcgaggtcgtgaaat gtctccccgtgacagcccctgagaacggcaagattgtcag cagcgctatggaacctgatagagagtaccatttcggccaa gccgtcagatttgtgtgcaattccggctacaaaattgaggg ggacgaagagatgcactgctccgacgatggcttctggag caaggaaaaacccaagtgcgtggagattagctgcaagag ccccgacgtgatcaacgggagccccatctcccaaaaaatc atctacaaggagaacgagagatttcagtataagtgcaacat gggctatgagtacagcgagcggggggacgccgtgtgta ccgaatccgggtggagacccctgcctagctgc | |
| | CD5-sp+linker+ CR2(SCR1 SCR4)+(G ₄S)₂+linker +FH(SCR1 SCR4)+lin ker+(G₄S)₂ +linker+F H(SCR1-SCR4)+lin ker | MPMGSLQPLATLYLLGML VASCLGISCGSPPPILNGRI SYYSTPIAVGTVIRYSCSGT FALIGEASLLCITKDKVDG TWDKPAPKCEYFNAYSSC PEPIVPGGYKIRGSTPYRH GDSVTFACKTNFSMNGNK SVWCQANNMWGPTRLPT CVSVFPLECPALPMIHNGH HTSENVGSIAPGLSVTYSC ESGYLLVGEKIINCLSSGK WSAVPPTCEEARCKSLGR FPNGKVKEPPILRVGVTAN FFCDEGYRLQGPPSSRCVI AGQGVAWTKMPVCGGGG SGGGGSEDCNELPPRRNT EILTGSWSDQTYPEGTQAI YKCRPGYRSLGNVIMVCR KGEWVALNPLRKCQKRPC GHPGDTPFGTFTLTGGNVF EYGVKAVYTCNEGYQLLG EINYRECDTDGWTNDIPIC EVVKCLPVTAPENGKIVSS AMEPDREYHFGQAVRFVC NSGYKIEGDEEMHCSDDG FWSKEKPKCVEISCKSPDV INGSPISQKIIYKENERFQY KCNMGYEYSERGDAVCTE SGWRPLPSCEGGGGSGGG GSEDCNELPPRRNTEILTG SWSDQTYPEGTQAIYKCR PGYRSLGNVIMVCRKGEW VALNPLRKCQKRPCGHPG DTPFGTFTLTGGNVFEYG VKAVYTCNEGYQLLGEIN YRECDTDGWTNDIPICEV VKCLPVTAPENGKIVSSA | 52 | atgcccatgggcagcctgcagcccctggccaccctgtacc tgctgggcatgctggtggctagctgcctgggcatcagctg cggcagcccccctcccatcctgaacggcagaatcagcta ctacagcacccccatcgccgtgggcaccgtgatcagatac agctgcagcggcaccttcgccctgatcggcgaggctagc ctgctgtgcatcaccaaggacaaggtggacggcacctgg gacaagcccgcccccaagtgcgagtacttcaacgcctac agcagctgccccgagcccatcgtgcctggcggctacaag atcagaggcagcaccccctacagacacggcgacagcgt gaccttcgcctgcaagaccaacttcagcatgaacggcaac aagagcgtgtggtgccaagccaacaacatgtggggcccc acaagactgcccacctgcgtgagcgtgttccccctggagt gccccgccctgcccatgatccacaacggccaccacacaa gcgagaacgtgggcagcatcgcccccggcctgagcgtg acctacagctgcgagagcggctacctgctggtgggcgag aagatcatcaactgcctgagcagcggcaagtggagcgcc gtgccccccacctgcgaggaggctagatgcaagagcctg ggcagattccccaacggcaaggtgaaggagcctcctatc ctgagagtgggcgtgaccgccaacttcttctgcgacgagg gctacagactgcaaggcccccctagcagcagatgtgtgat cgctggccaaggcgtggcctggaccaaaatgcccgtctg cgggggggggggcagcgggggcgggggcagcgagg attgcaacgagctgccccctagacggaacacagagatcct gaccggcagctggagcgatcaaacatatcccgagggcac ccaagccatctacaaatgcagacctggctacagaagcctg ggcaatgtgatcatggtctgccggaaaggggagtgggtc gctctgaaccccctgcggaagtgtcagaagagaccttgcg gccaccccggcgacacacccttcgggaccttcacactga ccggcggcaacgtgtttgagtacggcgtcaaggccgtgta cacctgtaacgaggggtatcagctgctgggcgagatcaac tacagagagtgcgataccgacggctggaccaatgacatc cccatctgcgaggtggtgaagtgtctgcccgtgaccgccc ccgaaaacggcaagatcgtgagcagcgccatggagccc gaccgggagtaccattttgggcaagctgtgagattcgtctg caatagcggctacaagatcgaaggcgacgaggagatgc actgtagcgacgacggcttctggtccaaagagaaacccaa atgcgtggagatctcctgcaaaagccccgatgtgatcaac | 71 |
| | | MEPDREYHFGQAVRFVCN SGYKIEGDEEMHCSDDGF WSKEKPKCVEISCKSPDVI NGSPISQKIIYKENERFQY KCNMGYEYSERGDAVCTE SGWRPLPSCE | | ggcagccccatctcccaaaaaattatctacaaggaaaacg aacggtttcagtacaaatgcaacatgggctacgaatactcc gagcggggggatgccgtgtgcaccgagagcggctggc ggcctctgcctagctgtgaagggggcggcgggagcggc gggggcggcagcgaggactgcaatgaactgcctcctaga cggaacaccgaaatcctgaccgggagctggagcgacca aacataccccgaggggacacaagccatctacaagtgcag acctgggtatagatccctcgggaacgtgatcatggtctgtc ggaagggcgagtgggtcgctctgaaccctctcagaaagt gtcagaaacggccctgcgggcaccccggcgataccccct tcggcacattcaccctgaccggggggaacgtgttcgagta cggcgtgaaggccgtctacacctgcaacgagggctacca actcctgggcgaaatcaattacagagaatgcgacacagat ggctggaccaacgacatccctatctgcgaggtcgtgaaat gtctccccgtgacagcccctgagaacggcaagattgtcag cagcgctatggaacctgatagagagtaccatttcggccaa gccgtcagatttgtgtgcaattccggctacaaaattgaggg ggacgaagagatgcactgctccgacgatggcttctggag caaggaaaaacccaagtgcgtggagattagctgcaagag ccccgacgtgatcaacgggagccccatctcccaaaaaatc atctacaaggagaacgagagatttcagtataagtgcaacat gggctatgagtacagcgagcggggggacgccgtgtgta ccgaatccgggtggagacccctgcctagctgcgag | |
| XMDC 061-(CR2-R36A K41A K67A) | CR2(SCR1 -SCR4)-(R36A K41A K67A) | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVC | 46 | tgtggcagccctcctcctattctgaatggcaggatcagcta ctacagcacccctattgccgtgggcaccgtgatcagatac agctgctctggcacattcgccctgatcggcgaagccagcc tgctgtgcatcaccaaggataaggtggacggcacctggg acaagcccgctcctaagtgcgaatatttcaacgcctacagc agctgccctgagcctattgtgcctggcggctacaagatca gaggcagcaccccatacagacacggcgacagcgtgacc tttgcctgcaagaccaacttcagcatgaacggcaacaaga gcgtgtggtgccaggccaacaatatgtggggccctacca gactgcccacctgtgtgtctgtgttccctctggaatgccccg ctctgcccatgatccacaatggccaccacaccagcgagaa cgtgggatctattgcccctggcctgtccgtgacctacagct gtgaaagcggctatctgctcgtgggcgagaagatcatcaa ctgcctgagcagcggcaagtggagcgctgttcctcctaca tgcgaagaggccagatgcaagagcctgggcagattcccc aacggcaaagtgaaagagcctcctatcctgagagtgggc gtgaccgccaacttcttctgcgacgagggctacagactgc agggccctcctagcagcagatgtgtgattgctggacaggg cgtcgcctggaccaagatgcctgtttgt | 64 |
| | FH(SCR1-SCR4) | CNELPPRRNTEILTGSWSD QTYPEGTQAIYKCRPGYR SLGNVIMVCRKGEWVALN PLRKCQKRPCGHPGDTPF GTFTLTGGNVFEYGVKAV YTCNEGYQLLGEINYREC DTDGWTNDIPICEVVKCLP VTAPENGKIVSSAMEPDRE YHFGQAVRFVCNSGYKIE GDEEMHCSDDGFWSKEK PKCVEISCKSPDVINGSPIS QKIIYKENERFQYKCNMG YEYSERGDAVCTESGWRP LPSC | 2 | tgcaacgaactgcctcctagaagaaacaccgaaatcctca caggctcctggtcagatcagacctaccctgagggtacaca ggccatctataaatgccgcccgggttaccgctccttgggca atgttattatggtctgccggaaaggggaatgggtggccttg aaccccctcagaaagtgccaaaaaagaccctgcggacat ccaggagacacacctttcgggacatttaccttgaccggtgg taacgtgttcgagtacggagtgaaggcagtctatacttgca atgagggctaccagctcctgggggaaataaattatcgcga atgtgatacagacggctggacgaacgacattcctatctgtg aggtggtcaaatgtctgcccgtcactgctcctgaaaacggc aagatagtgagttctgcaatggagccggatagggagtatc attttggtcaggccgtgcgctttgtgtgcaatagtgggtata aaatcgagggggatgaagaaatgcactgtagcgacgatg gattctggtctaaagagaaacctaagtgtgtggagattagtt gcaagagtccagacgtgattaacggtagccccatttccca aaagataatatacaaggaaaacgaaagattccagtataaat gcaacatgggctacgagtatagcgagcggggcgatgcc gtgtgtactgaaagcggctggcgccccctgccttcctgc | 19 |
| | FH(SCR18) | CVNPPTVQNAYIVSRQMS KYPSGERVRYQCRSPYEM FGDEEVMCLNGNWTEPPQ C | 39 | tgcgtgaaccctccaacagtccaaaatgcctatatagtctct aggcagatgagtaaatacccaagtggagagagagtgaga taccagtgccggtctccttacgagatgttcggcgacgagg aagtgatgtgcctgaacggcaattggacagagccaccac aatgt | 57 |
| | FH(SCR20) | CVISREIMENYNIALRWTA KQKLYSRTGESVEFVCKR GYRLSSRSHTLRTTCWDG KLEYPTC | 40 | tgtgtgatcagtcgggagatcatggagaactacaacattgc cctgaggtggactgctaagcagaagctctatagccgaaca ggcgagtcagtggagtttgtgtgtaaacggggatacaggc tgtccagtaggagccacaccctgcgcactacttgctgggat gggaagctggaatatccaacttgc | 58 |
| | CR2(SCR1 SCR4)+(G ₄S)₂+linker +FH(SCR1 - SCR4)+lin ker+FH(S CR18)+lin ker+FH(S CR20) | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVCGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCERDTSCVNPPTV QNAYIVSRQMSKYPSGER VRYQCRSPYEMFGDEEVM CLNGNWTEPPQCKDSTGK CVISREIMENYNIALRWTA KQKLYSRTGESVEFVCKR GYRLSSRSHTLRTTCWDG KLEYPTC | 53 | tgtggcagccctcctcctattctgaatggcaggatcagcta ctacagcacccctattgccgtgggcaccgtgatcagatac agctgctctggcacattcgccctgatcggcgaagccagcc tgctgtgcatcaccaaggataaggtggacggcacctggg acaagcccgctcctaagtgcgaatatttcaacgcctacagc agctgccctgagcctattgtgcctggcggctacaagatca gaggcagcaccccatacagacacggcgacagcgtgacc tttgcctgcaagaccaacttcagcatgaacggcaacaaga gcgtgtggtgccaggccaacaatatgtggggccctacca gactgcccacctgtgtgtctgtgttccctctggaatgccccg ctctgcccatgatccacaatggccaccacaccagcgagaa cgtgggatctattgcccctggcctgtccgtgacctacagct gtgaaagcggctatctgctcgtgggcgagaagatcatcaa ctgcctgagcagcggcaagtggagcgctgttcctcctaca tgcgaagaggccagatgcaagagcctgggcagattcccc aacggcaaagtgaaagagcctcctatcctgagagtgggc gtgaccgccaacttcttctgcgacgagggctacagactgc agggccctcctagcagcagatgtgtgattgctggacaggg cgtcgcctggaccaagatgcctgtttgtggcggaggtggg tcgggtggcggcggatctgaggactgcaacgaactgcct cctagaagaaacaccgaaatcctcacaggctcctggtcag atcagacctaccctgagggtacacaggccatctataaatgc cgcccgggttaccgctccttgggcaatgttattatggtctgc cggaaaggggaatgggtggccttgaaccccctcagaaag tgccaaaaaagaccctgcggacatccaggagacacacct ttcgggacatttaccttgaccggtggtaacgtgttcgagtac ggagtgaaggcagtctatacttgcaatgagggctaccagc tcctgggggaaataaattatcgcgaatgtgatacagacgg ctggacgaacgacattcctatctgtgaggtggtcaaatgtct gcccgtcactgctcctgaaaacggcaagatagtgagttctg caatggagccggatagggagtatcattttggtcaggccgt gcgctttgtgtgcaatagtgggtataaaatcgagggggatg aagaaatgcactgtagcgacgatggattctggtctaaaga gaaacctaagtgtgtggagattagttgcaagagtccagac gtgattaacggtagccccatttcccaaaagataatatacaa ggaaaacgaaagattccagtataaatgcaacatgggctac gagtatagcgagcggggcgatgccgtgtgtactgaaagc ggctggcgccccctgccttcctgcgagcgggatacaagct gcgtgaaccctccaacagtccaaaatgcctatatagtctcta ggcagatgagtaaatacccaagtggagagagagtgagat accagtgccggtctccttacgagatgttcggcgacgagga agtgatgtgcctgaacggcaattggacagagccaccaca atgtaaggatagtacaggcaaatgtgtgatcagtcgggag atcatggagaactacaacattgccctgaggtggactgctaa gcagaagctctatagccgaacaggcgagtcagtggagttt gtgtgtaaacggggatacaggctgtccagtaggagccac accctgcgcactacttgctgggatgggaagctggaatatc caacttgc | 72 |
| | CD5-sp+linker+ | MPMGSLQPLATLYLLGML VASCLGISCGSPPPILNGRI | 54 | atgccaatggggtccctacaacccctggcaacactatatct gctaggtatgctcgtggcctcctgtttaggaatcagctgtgg | 73 |
| | CR2(SCR1 SCR4)+(G ₄S)₂+linker +FH(SCR1 SCR4)+lin ker+FH(S CR18)+lin ker+FH(S CR20)+lin ker | SYYSTPIAVGTVIRYSCSGT FALIGEASLLCITKDKVDG TWDKPAPKCEYFNAYSSC PEPIVPGGYKIRGSTPYRH GDSVTFACKTNFSMNGNK SVWCQANNMWGPTRLPT CVSVFPLECPALPMIHNGH HTSENVGSIAPGLSVTYSC ESGYLLVGEKIINCLSSGK WSAVPPTCEEARCKSLGR FPNGKVKEPPILRVGVTAN FFCDEGYRLQGPPSSRCVI AGQGVAWTKMPVCGGGG SGGGGSEDCNELPPRRNT EILTGSWSDQTYPEGTQAI YKCRPGYRSLGNVIMVCR KGEWVALNPLRKCQKRPC GHPGDTPFGTFTLTGGNVF EYGVKAVYTCNEGYQLLG EINYRECDTDGWTNDIPIC EVVKCLPVTAPENGKIVSS AMEPDREYHFGQAVRFVC NSGYKIEGDEEMHCSDDG FWSKEKPKCVEISCKSPDV INGSPISQKIIYKENERFQY KCNMGYEYSERGDAVCTE SGWRPLPSCERDTSCVNPP TVQNAYIVSRQMSKYPSG ERVRYQCRSPYEMFGDEE VMCLNGNWTEPPQCKDS TGKCVISREIMENYNIALR WTAKQKLYSRTGESVEFV CKRGYRLSSRSHTLRTTC WDGKLEYPTCAKR | | cagccctcctcctattctgaatggcaggatcagctactaca gcacccctattgccgtgggcaccgtgatcagatacagctg ctctggcacattcgccctgatcggcgaagccagcctgctgt gcatcaccaaggataaggtggacggcacctgggacaag cccgctcctaagtgcgaatatttcaacgcctacagcagctg ccctgagcctattgtgcctggcggctacaagatcagaggc agcaccccatacagacacggcgacagcgtgacctttgcct gcaagaccaacttcagcatgaacggcaacaagagcgtgt ggtgccaggccaacaatatgtggggccctaccagactgc ccacctgtgtgtctgtgttccctctggaatgccccgctctgc ccatgatccacaatggccaccacaccagcgagaacgtgg gatctattgcccctggcctgtccgtgacctacagctgtgaa agcggctatctgctcgtgggcgagaagatcatcaactgcc tgagcagcggcaagtggagcgctgttcctcctacatgcga agaggccagatgcaagagcctgggcagattccccaacg gcaaagtgaaagagcctcctatcctgagagtgggcgtgac cgccaacttcttctgcgacgagggctacagactgcagggc cctcctagcagcagatgtgtgattgctggacagggcgtcg cctggaccaagatgcctgtttgtggcggaggtgggtcggg tggcggcggatctgaggactgcaacgaactgcctcctaga agaaacaccgaaatcctcacaggctcctggtcagatcaga cctaccctgagggtacacaggccatctataaatgccgccc gggttaccgctccttgggcaatgttattatggtctgccggaa aggggaatgggtggccttgaaccccctcagaaagtgcca aaaaagaccctgcggacatccaggagacacacctttcgg gacatttaccttgaccggtggtaacgtgttcgagtacggag tgaaggcagtctatacttgcaatgagggctaccagctcctg ggggaaataaattatcgcgaatgtgatacagacggctgga cgaacgacattcctatctgtgaggtggtcaaatgtctgccc gtcactgctcctgaaaacggcaagatagtgagttctgcaat ggagccggatagggagtatcattttggtcaggccgtgcgc tttgtgtgcaatagtgggtataaaatcgagggggatgaaga aatgcactgtagcgacgatggattctggtctaaagagaaac ctaagtgtgtggagattagttgcaagagtccagacgtgatt aacggtagccccatttcccaaaagataatatacaaggaaa acgaaagattccagtataaatgcaacatgggctacgagtat agcgagcggggcgatgccgtgtgtactgaaagcggctg gcgccccctgccttcctgcgagcgggatacaagctgcgtg aaccctccaacagtccaaaatgcctatatagtctctaggca gatgagtaaatacccaagtggagagagagtgagatacca gtgccggtctccttacgagatgttcggcgacgaggaagtg atgtgcctgaacggcaattggacagagccaccacaatgta aggatagtacaggcaaatgtgtgatcagtcgggagatcat ggagaactacaacattgccctgaggtggactgctaagcag aagctctatagccgaacaggcgagtcagtggagtttgtgtg taaacggggatacaggctgtccagtaggagccacaccct gcgcactacttgctgggatgggaagctggaatatccaactt gcgccaagagg | |
| XMDC 062-(CR2-R36A K41A K67A) | CR2(SCR1 -SCR4)-(R36A K41A K67A) | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG | 46 | tgcggcagcccccctcccatcctgaacggcagaatcagct actacagcacccccatcgccgtgggcaccgtgatcagata cagctgcagcggcaccttcgccctgatcggcgaggctag cctgctgtgcatcaccaaggacaaggtggacggcacctg ggacaagcccgcccccaagtgcgagtacttcaacgccta cagcagctgccccgagcccatcgtgcctggcggctacaa gatcagaggcagcaccccctacagacacggcgacagcg tgaccttcgcctgcaagaccaacttcagcatgaacggcaa caagagcgtgtggtgccaagccaacaacatgtggggccc cacaagactgcccacctgcgtgagcgtgttccccctggag tgccccgccctgcccatgatccacaacggccaccacaca agcgagaacgtgggcagcatcgcccccggcctgagcgt | 69 |
| | | YRLQGPPSSRCVIAGQGVA WTKMPVC | | gacctacagctgcgagagcggctacctgctggtgggcga gaagatcatcaactgcctgagcagcggcaagtggagcgc cgtgccccccacctgcgaggaggctagatgcaagagcct gggcagattccccaacggcaaggtgaaggagcctcctat cctgagagtgggcgtgaccgccaacttcttctgcgacgag ggctacagactgcaaggcccccctagcagcagatgtgtg atcgctggccaaggcgtggcctggaccaaaatgcccgtct gc | |
| | FH(SCR1-SCR4) | CNELPPRRNTEILTGSWSD QTYPEGTQAIYKCRPGYR SLGNVIMVCRKGEWVALN PLRKCQKRPCGHPGDTPF GTFTLTGGNVFEYGVKAV YTCNEGYQLLGEINYREC DTDGWTNDIPICEVVKCLP VTAPENGKIVSSAMEPDRE YHFGQAVRFVCNSGYKIE GDEEMHCSDDGFWSKEK PKCVEISCKSPDVINGSPIS QKIIYKENERFQYKCNMG YEYSERGDAVCTESGWRP LPSC | 2 | tgcaacgagctgccccctagacggaacacagagatcctg accggcagctggagcgatcaaacatatcccgagggcacc caagccatctacaaatgcagacctggctacagaagcctgg gcaatgtgatcatggtctgccggaaaggggagtgggtcg ctctgaaccccctgcggaagtgtcagaagagaccttgcgg ccaccccggcgacacacccttcgggaccttcacactgacc ggcggcaacgtgtttgagtacggcgtcaaggccgtgtaca cctgtaacgaggggtatcagctgctgggcgagatcaacta cagagagtgcgataccgacggctggaccaatgacatccc catctgcgaggtggtgaagtgtctgcccgtgaccgccccc gaaaacggcaagatcgtgagcagcgccatggagcccga ccgggagtaccattttgggcaagctgtgagattcgtctgca atagcggctacaagatcgaaggcgacgaggagatgcact gtagcgacgacggcttctggtccaaagagaaacccaaat gcgtggagatctcctgcaaaagccccgatgtgatcaacgg cagccccatctcccaaaaaattatctacaaggaaaacgaa cggtttcagtacaaatgcaacatgggctacgaatactccga gcggggggatgccgtgtgcaccgagagcggctggcgg cctctgcctagctgt | 28 |
| | | | | tgcaatgaactgcctcctagacggaacaccgaaatcctga ccgggagctggagcgaccaaacataccccgaggggaca caagccatctacaagtgcagacctgggtatagatccctcg ggaacgtgatcatggtctgtcggaagggcgagtgggtcg ctctgaaccctctcagaaagtgtcagaaacggccctgcgg gcaccccggcgatacccccttcggcacattcaccctgacc ggggggaacgtgttcgagtacggcgtgaaggccgtctac acctgcaacgagggctaccaactcctgggcgaaatcaatt acagagaatgcgacacagatggctggaccaacgacatcc ctatctgcgaggtcgtgaaatgtctccccgtgacagcccct gagaacggcaagattgtcagcagcgctatggaacctgata gagagtaccatttcggccaagccgtcagatttgtgtgcaatt ccggctacaaaattgagggggacgaagagatgcactgct ccgacgatggcttctggagcaaggaaaaacccaagtgcg tggagattagctgcaagagccccgacgtgatcaacggga gccccatctcccaaaaaatcatctacaaggagaacgagag atttcagtataagtgcaacatgggctatgagtacagcgagc ggggggacgccgtgtgtaccgaatccgggtggagaccc ctgcctagctgc | 29 |
| | FH(SCR7) | CYFPYLENGYNQNHGRKF VQGKSIDVACHPGYALPK AQTTVTCMENGWSPTPRC | 43 | tgttacttcccctacctggagaacggctacaatcagaacca cggcagaaagttcgtgcaaggcaagagcatcgacgtggc ctgccaccccggctacgccctgcccaaggctcagaccac cgtgacctgcatggagaatggctggagccccacccctag atgc | 61 |
| | CR2(SCR1 SCR4)+(G ₄S)₂+linker +FH(SCR1 - SCR4)+lin | CGSPPPILNGRISYYSTPIA VGTVIRYSCSGTFALIGEA SLLCITKDKVDGTWDKPA PKCEYFNAYSSCPEPIVPG GYKIRGSTPYRHGDSVTFA CKTNFSMNGNKSVWCQA NNMWGPTRLPTCVSVFPL | 55 | tgcggcagcccccctcccatcctgaacggcagaatcagct actacagcacccccatcgccgtgggcaccgtgatcagata cagctgcagcggcaccttcgccctgatcggcgaggctag cctgctgtgcatcaccaaggacaaggtggacggcacctg ggacaagcccgcccccaagtgcgagtacttcaacgccta cagcagctgccccgagcccatcgtgcctggcggctacaa gatcagaggcagcaccccctacagacacggcgacagcg | 74 |
| | ker+(G₄S)₂ +linker+F H(SCR1-SCR4)+lin ker+(G₄S)₂ +linker+F H(SCR7) | ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVCGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCEGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCEGGGGSGGGGS LRKCYFPYLENGYNQNHG RKFVQGKSIDVACHPGYA LPKAQTTVTCMENGWSPT PRC | | tgaccttcgcctgcaagaccaacttcagcatgaacggcaa caagagcgtgtggtgccaagccaacaacatgtggggccc cacaagactgcccacctgcgtgagcgtgttccccctggag tgccccgccctgcccatgatccacaacggccaccacaca agcgagaacgtgggcagcatcgcccccggcctgagcgt gacctacagctgcgagagcggctacctgctggtgggcga gaagatcatcaactgcctgagcagcggcaagtggagcgc cgtgccccccacctgcgaggaggctagatgcaagagcct gggcagattccccaacggcaaggtgaaggagcctcctat cctgagagtgggcgtgaccgccaacttcttctgcgacgag ggctacagactgcaaggcccccctagcagcagatgtgtg atcgctggccaaggcgtggcctggaccaaaatgcccgtct gcgggggggggggcagcgggggcgggggcagcgag gattgcaacgagctgccccctagacggaacacagagatc ctgaccggcagctggagcgatcaaacatatcccgagggc acccaagccatctacaaatgcagacctggctacagaagcc tgggcaatgtgatcatggtctgccggaaaggggagtgggt cgctctgaaccccctgcggaagtgtcagaagagaccttgc ggccaccccggcgacacacccttcgggaccttcacactg accggcggcaacgtgtttgagtacggcgtcaaggccgtgt acacctgtaacgaggggtatcagctgctgggcgagatcaa ctacagagagtgcgataccgacggctggaccaatgacat ccccatctgcgaggtggtgaagtgtctgcccgtgaccgcc cccgaaaacggcaagatcgtgagcagcgccatggagcc cgaccgggagtaccattttgggcaagctgtgagattcgtct gcaatagcggctacaagatcgaaggcgacgaggagatg cactgtagcgacgacggcttctggtccaaagagaaaccca aatgcgtggagatctcctgcaaaagccccgatgtgatcaa cggcagccccatctcccaaaaaattatctacaaggaaaac gaacggtttcagtacaaatgcaacatgggctacgaatactc cgagcggggggatgccgtgtgcaccgagagcggctggc ggcctctgcctagctgtgaagggggcggcgggagcggc gggggcggcagcgaggactgcaatgaactgcctcctaga cggaacaccgaaatcctgaccgggagctggagcgacca aacataccccgaggggacacaagccatctacaagtgcag acctgggtatagatccctcgggaacgtgatcatggtctgtc ggaagggcgagtgggtcgctctgaaccctctcagaaagt gtcagaaacggccctgcgggcaccccggcgataccccct tcggcacattcaccctgaccggggggaacgtgttcgagta cggcgtgaaggccgtctacacctgcaacgagggctacca actcctgggcgaaatcaattacagagaatgcgacacagat ggctggaccaacgacatccctatctgcgaggtcgtgaaat gtctccccgtgacagcccctgagaacggcaagattgtcag cagcgctatggaacctgatagagagtaccatttcggccaa gccgtcagatttgtgtgcaattccggctacaaaattgaggg ggacgaagagatgcactgctccgacgatggcttctggag caaggaaaaacccaagtgcgtggagattagctgcaagag ccccgacgtgatcaacgggagccccatctcccaaaaaatc atctacaaggagaacgagagatttcagtataagtgcaacat gggctatgagtacagcgagcggggggacgccgtgtgta ccgaatccgggtggagacccctgcctagctgcgagggcg ggggcgggagcggcgggggcggctccctgagaaagtg ttacttcccctacctggagaacggctacaatcagaaccacg gcagaaagttcgtgcaaggcaagagcatcgacgtggcct gccaccccggctacgccctgcccaaggctcagaccaccg tgacctgcatggagaatggctggagccccacccctagatg c | |
| | CD5-sp+linker+ CR2(SCR1 | MPMGSLQPLATLYLLGML VASCLGISCGSPPPILNGRI SYYSTPIAVGTVIRYSCSGT | 56 | atgcccatgggcagcctgcagcccctggccaccctgtacc tgctgggcatgctggtggctagctgcctgggcatcagctg cggcagcccccctcccatcctgaacggcagaatcagcta | 75 |
| | SCR4)+(G ₄S)₂+linker +FH(SCR1 - SCR4)+lin ker+(G₄S)₂ +linker+F H(SCR1-SCR4)+lin ker+(G₄S)₂ +linker+F H(SCR7)+ linker | FALIGEASLLCITKDKVDG TWDKPAPKCEYFNAYSSC PEPIVPGGYKIRGSTPYRH GDSVTFACKTNFSMNGNK SVWCQANNMWGPTRLPT CVSVFPLECPALPMIHNGH HTSENVGSIAPGLSVTYSC ESGYLLVGEKIINCLSSGK WSAVPPTCEEARCKSLGR FPNGKVKEPPILRVGVTAN FFCDEGYRLQGPPSSRCVI AGQGVAWTKMPVCGGGG SGGGGSEDCNELPPRRNT EILTGSWSDQTYPEGTQAI YKCRPGYRSLGNVIMVCR KGEWVALNPLRKCQKRPC GHPGDTPFGTFTLTGGNVF EYGVKAVYTCNEGYQLLG EINYRECDTDGWTNDIPIC EVVKCLPVTAPENGKIVSS AMEPDREYHFGQAVRFVC NSGYKIEGDEEMHCSDDG FWSKEKPKCVEISCKSPDV INGSPISQKIIYKENERFQY KCNMGYEYSERGDAVCTE SGWRPLPSCEGGGGSGGG GSEDCNELPPRRNTEILTG SWSDQTYPEGTQAIYKCR PGYRSLGNVIMVCRKGEW VALNPLRKCQKRPCGHPG DTPFGTFTLTGGNVFEYG VKAVYTCNEGYQLLGEIN YRECDTDGWTNDIPICEV VKCLPVTAPENGKIVSSA MEPDREYHFGQAVRFVCN SGYKIEGDEEMHCSDDGF WSKEKPKCVEISCKSPDVI NGSPISQKIIYKENERFQY KCNMGYEYSERGDAVCTE SGWRPLPSCEGGGGSGGG GSLRKCYFPYLENGYNQN HGRKFVQGKSIDVACHPG YALPKAQTTVTCMENGW SPTPRCIRVKT | | ctacagcacccccatcgccgtgggcaccgtgatcagatac agctgcagcggcaccttcgccctgatcggcgaggctagc ctgctgtgcatcaccaaggacaaggtggacggcacctgg gacaagcccgcccccaagtgcgagtacttcaacgcctac agcagctgccccgagcccatcgtgcctggcggctacaag atcagaggcagcaccccctacagacacggcgacagcgt gaccttcgcctgcaagaccaacttcagcatgaacggcaac aagagcgtgtggtgccaagccaacaacatgtggggcccc acaagactgcccacctgcgtgagcgtgttccccctggagt gccccgccctgcccatgatccacaacggccaccacacaa gcgagaacgtgggcagcatcgcccccggcctgagcgtg acctacagctgcgagagcggctacctgctggtgggcgag aagatcatcaactgcctgagcagcggcaagtggagcgcc gtgccccccacctgcgaggaggctagatgcaagagcctg ggcagattccccaacggcaaggtgaaggagcctcctatc ctgagagtgggcgtgaccgccaacttcttctgcgacgagg gctacagactgcaaggcccccctagcagcagatgtgtgat cgctggccaaggcgtggcctggaccaaaatgcccgtctg cgggggggggggcagcgggggcgggggcagcgagg attgcaacgagctgccccctagacggaacacagagatcct gaccggcagctggagcgatcaaacatatcccgagggcac ccaagccatctacaaatgcagacctggctacagaagcctg ggcaatgtgatcatggtctgccggaaaggggagtgggtc gctctgaaccccctgcggaagtgtcagaagagaccttgcg gccaccccggcgacacacccttcgggaccttcacactga ccggcggcaacgtgtttgagtacggcgtcaaggccgtgta cacctgtaacgaggggtatcagctgctgggcgagatcaac tacagagagtgcgataccgacggctggaccaatgacatc cccatctgcgaggtggtgaagtgtctgcccgtgaccgccc ccgaaaacggcaagatcgtgagcagcgccatggagccc gaccgggagtaccattttgggcaagctgtgagattcgtctg caatagcggctacaagatcgaaggcgacgaggagatgc actgtagcgacgacggcttctggtccaaagagaaaccca aatgcgtggagatctcctgcaaaagccccgatgtgatcaac ggcagccccatctcccaaaaaattatctacaaggaaaacg aacggtttcagtacaaatgcaacatgggctacgaatactcc gagcggggggatgccgtgtgcaccgagagcggctggc ggcctctgcctagctgtgaagggggcggcgggagcggc gggggcggcagcgaggactgcaatgaactgcctcctaga cggaacaccgaaatcctgaccgggagctggagcgacca aacataccccgaggggacacaagccatctacaagtgcag acctgggtatagatccctcgggaacgtgatcatggtctgtc ggaagggcgagtgggtcgctctgaaccctctcagaaagt gtcagaaacggccctgcgggcaccccggcgataccccct tcggcacattcaccctgaccggggggaacgtgttcgagta cggcgtgaaggccgtctacacctgcaacgagggctacca actcctgggcgaaatcaattacagagaatgcgacacagat ggctggaccaacgacatccctatctgcgaggtcgtgaaat gtctccccgtgacagcccctgagaacggcaagattgtcag cagcgctatggaacctgatagagagtaccatttcggccaa gccgtcagatttgtgtgcaattccggctacaaaattgaggg ggacgaagagatgcactgctccgacgatggcttctggag caaggaaaaacccaagtgcgtggagattagctgcaagag ccccgacgtgatcaacgggagccccatctcccaaaaaatc atctacaaggagaacgagagatttcagtataagtgcaacat gggctatgagtacagcgagcggggggacgccgtgtgta ccgaatccgggtggagacccctgcctagctgcgagggc gggggcgggagcggcgggggcggctccctgagaaagtg ttacttcccctacctggagaacggctacaatcagaaccacg gcagaaagttcgtgcaaggcaagagcatcgacgtggcct | |
| | | | | gccaccccggctacgccctgcccaaggctcagaccaccg tgacctgcatggagaatggctggagccccacccctagatg catcagagtgaagacc | |
| CR2-FH(1-5+FH1-5 | CR2(SCR1 -SCR4) | CGSPPPILNGRISYYSTPIA VGTVIRYSCSGTFRLIGEK SLLCITKDKVDGTWDKPA PKCEYFNKYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVC | 1 | tgcggcagcccccctcccatcctcaacggcagaatcagct actacagcacccccatcgccgtgggcaccgtgatcagata cagctgcagcggcaccttcagactgatcggcgagaagag cctgctgtgcatcaccaaggacaaggtggacggcacctg ggacaagcccgcccccaagtgcgagtacttcaacaagta cagcagctgccccgagcccatcgtgcccggcggctataa gatcagaggcagcaccccctacagacacggcgacagcg tgaccttcgcctgcaagaccaacttcagcatgaacggcaa caagagcgtgtggtgccaagccaacaacatgtggggccc cacaagactgcccacctgcgtgagcgtgttccccctggag tgccccgccctgcccatgatccacaacggccaccacaca agcgagaacgtgggcagcatcgcccccggcctgagcgt gacctacagctgcgagagcggctacctgctggtgggcga gaagatcatcaactgcctgagcagcggcaagtggagcgc cgtgccccccacctgcgaggaggctagatgcaagagcct gggcagattccccaacggcaaggtgaaggagccccccat cctgagagtgggcgtgaccgccaacttcttctgcgacgag ggctacagactgcaaggcccccctagcagcagatgcgtg atcgccggccaaggcgtggcttggacaaaaatgcctgtgt gt | 76 |
| | FH(SCR1-SCR5 | CNELPPRRNTEILTGSWSD QTYPEGTQAIYKCRPGYR SLGNVIMVCRKGEWVALN PLRKCQKRPCGHPGDTPF GTFTLTGGNVFEYGVKAV YTCNEGYQLLGEINYREC DTDGWTNDIPICEVVKCLP VTAPENGKIVSSAMEPDRE YHFGQAVRFVCNSGYKIE GDEEMHCSDDGFWSKEK PKCVEISCKSPDVINGSPIS QKIIYKENERFQYKCNMG YEYSERGDAVCTESGWRP LPSCEEKSCDNPYIPNGDY SPLRIKHRTGDEITYQCRN GFYPATRGNTAKCTSTGWI PAPRC | 14 | tgcaatgagctccccccccggagaaacaccgagatcctg accggcagctggagcgatcagacctatcccgagggcacc caagccatttataagtgcagacccggctacagaagcctgg ggaatgtgatcatggtctgcagaaagggcgagtgggtgg ccctgaatcccctccggaagtgccaaaagcggccctgtg ggcaccccggcgacacacccttcggcacctttaccctgac cggcggcaatgtcttcgagtatggcgtcaaggccgtgtata cctgcaacgaaggctatcagctgctgggcgagatcaacta tcgggagtgtgacaccgacggctggacaaacgacatccc catctgcgaggtggtgaagtgcctgcccgtgaccgccccc gaaaacggcaaaatcgtcagcagcgctatggaacccgac cgggaataccacttcggccaagctgtcagattcgtgtgtaa tagcgggtataagattgagggcgatgaggaaatgcactgc agcgacgacggcttctggagcaaggagaagcccaaatg cgtggagatcagctgtaaaagccccgacgtgatcaacgg cagccccatcagccaaaaaatcatttacaaggagaacgaa agatttcagtacaagtgtaatatgggctacgagtatagcga gcggggcgacgctgtgtgcaccgagagcggctggagac ctctcccttcctgcgaggagaagagctgcgacaaccccta catccccaacggcgactactcccccctgagaatcaaacat agaaccggggacgagatcacctatcagtgcagaaatggc ttctaccctgccacaagagggaacaccgccaagtgcacat ccaccgggtggattcccgctcctagatgt | 77 |
| | | | | tgcaacgagctgccccctcggagaaacacagagatcctg accgggagctggagcgatcagacctaccccgaggggac acaagccatctacaagtgcagacctgggtatagaagcctg ggcaacgtgattatggtgtgcagaaagggggaatgggtg gccctgaatcccctgagaaagtgtcagaagagaccctgc ggccaccctggcgatacaccctttggcaccttcaccctgac cgggggcaacgtgttcgagtacggcgtgaaggccgtgta cacatgcaacgagggctatcaactgctgggggagatcaat tacagagagtgcgataccgatgggtggaccaacgacattc ctatctgcgaagtcgtgaagtgcctccctgtcaccgccccc gagaacggcaagatcgtgagcagcgctatggagcctgat agagagtaccactttgggcaagccgtgagattcgtctgca acagcgggtacaagatcgagggcgacgaggagatgcac tgttccgatgacgggttctggagcaaagagaagcccaagt | 78 |
| | | | | gtgtggagatcagctgcaaaagccccgatgtgatcaatgg cagccccattagccaaaagattatctataaggaaaacgag agattccaatacaagtgcaacatggggtacgagtactccg aaagaggcgacgccgtctgcaccgaatccggctggaga cctctccctagctgtgaggagaagagctgtgataaccccta tattcccaacggcgattattcccctctgagaatcaagcacc ggaccggcgacgagattacctatcagtgtagaaacggctt ctaccccgctacaagaggcaataccgctaagtgcacctcc accggctggattcccgcccctagatgc | |
| | CR2(SCR1 SCR4)+(G₄ S)₂+linker+ FH(SCR1-SCR5)+link er+(G₄S)₂+ FH(SCR1-SCR5) | CGSPPPILNGRISYYSTPIA VGTVIRYSCSGTFRLIGEK SLLCITKDKVDGTWDKPA PKCEYFNKYSSCPEPIVPG GYKIRGSTPYRHGDSVTFA CKTNFSMNGNKSVWCQA NNMWGPTRLPTCVSVFPL ECPALPMIHNGHHTSENV GSIAPGLSVTYSCESGYLL VGEKIINCLSSGKWSAVPP TCEEARCKSLGRFPNGKV KEPPILRVGVTANFFCDEG YRLQGPPSSRCVIAGQGVA WTKMPVCGGGGSGGGGS EDCNELPPRRNTEILTGSW SDQTYPEGTQAIYKCRPG YRSLGNVIMVCRKGEWV ALNPLRKCQKRPCGHPGD TPFGTFTLTGGNVFEYGV KAVYTCNEGYQLLGEINY RECDTDGWTNDIPICEVV KCLPVTAPENGKIVSSAME PDREYHFGQAVRFVCNSG YKIEGDEEMHCSDDGFWS KEKPKCVEISCKSPDVING SPISQKIIYKENERFQYKCN MGYEYSERGDAVCTESG WRPLPSCEEKSCDNPYIPN GDYSPLRIKHRTGDEITYQ CRNGFYPATRGNTAKCTST GWIPAPRCTLKGGGGSGG GGSEDCNELPPRRNTEILT GSWSDQTYPEGTQAIYKC RPGYRSLGNVIMVCRKGE WVALNPLRKCQKRPCGHP GDTPFGTFTLTGGNVFEY GVKAVYTCNEGYQLLGEI NYRECDTDGWTNDIPICE VVKCLPVTAPENGKIVSSA MEPDREYHFGQAVRFVCN SGYKIEGDEEMHCSDDGF WSKEKPKCVEISCKSPDVI NGSPISQKIIYKENERFQY KCNMGYEYSERGDAVCTE SGWRPLPSCEEKSCDNPYI PNGDYSPLRIKHRTGDEIT YQCRNGFYPATRGNTAKC TSTGWIPAPRC | 79 | tgcggcagcccccctcccatcctcaacggcagaatcagct actacagcacccccatcgccgtgggcaccgtgatcagata cagctgcagcggcaccttcagactgatcggcgagaagag cctgctgtgcatcaccaaggacaaggtggacggcacctg ggacaagcccgcccccaagtgcgagtacttcaacaagta cagcagctgccccgagcccatcgtgcccggcggctataa gatcagaggcagcaccccctacagacacggcgacagcg tgaccttcgcctgcaagaccaacttcagcatgaacggcaa caagagcgtgtggtgccaagccaacaacatgtggggccc cacaagactgcccacctgcgtgagcgtgttccccctggag tgccccgccctgcccatgatccacaacggccaccacaca agcgagaacgtgggcagcatcgcccccggcctgagcgt gacctacagctgcgagagcggctacctgctggtgggcga gaagatcatcaactgcctgagcagcggcaagtggagcgc cgtgccccccacctgcgaggaggctagatgcaagagcct gggcagattccccaacggcaaggtgaaggagccccccat cctgagagtgggcgtgaccgccaacttcttctgcgacgag ggctacagactgcaaggcccccctagcagcagatgcgtg atcgccggccaaggcgtggcttggacaaaaatgcctgtgt gtggcgggggcgggagcggcgggggcggcagcgaag actgcaatgagctccccccccggagaaacaccgagatcc tgaccggcagctggagcgatcagacctatcccgagggca cccaagccatttataagtgcagacccggctacagaagcct ggggaatgtgatcatggtctgcagaaagggcgagtgggt ggccctgaatcccctccggaagtgccaaaagcggccctg tgggcaccccggcgacacacccttcggcacctttaccctg accggcggcaatgtcttcgagtatggcgtcaaggccgtgt atacctgcaacgaaggctatcagctgctgggcgagatcaa ctatcgggagtgtgacaccgacggctggacaaacgacat ccccatctgcgaggtggtgaagtgcctgcccgtgaccgcc cccgaaaacggcaaaatcgtcagcagcgctatggaaccc gaccgggaataccacttcggccaagctgtcagattcgtgt gtaatagcgggtataagattgagggcgatgaggaaatgca ctgcagcgacgacggcttctggagcaaggagaagccca aatgcgtggagatcagctgtaaaagccccgacgtgatcaa cggcagccccatcagccaaaaaatcatttacaaggagaac gaaagatttcagtacaagtgtaatatgggctacgagtatag cgagcggggcgacgctgtgtgcaccgagagcggctgga gacctctcccttcctgcgaggagaagagctgcgacaaccc ctacatccccaacggcgactactcccccctgagaatcaaa catagaaccggggacgagatcacctatcagtgcagaaat ggcttctaccctgccacaagagggaacaccgccaagtgc acatccaccgggtggattcccgctcctagatgtacactcaa gggcgggggcggcagcggcgggggcggcagcgagg actgcaacgagctgccccctcggagaaacacagagatcc tgaccgggagctggagcgatcagacctaccccgagggg acacaagccatctacaagtgcagacctgggtatagaagcc tgggcaacgtgattatggtgtgcagaaagggggaatgggt ggccctgaatcccctgagaaagtgtcagaagagaccctg cggccaccctggcgatacaccctttggcaccttcaccctga ccgggggcaacgtgttcgagtacggcgtgaaggccgtgt | 80 |
| | | | | acacatgcaacgagggctatcaactgctgggggagatca attacagagagtgcgataccgatgggtggaccaacgacat tcctatctgcgaagtcgtgaagtgcctccctgtcaccgccc ccgagaacggcaagatcgtgagcagcgctatggagcctg atagagagtaccactttgggcaagccgtgagattcgtctgc aacagcgggtacaagatcgagggcgacgaggagatgca ctgttccgatgacgggttctggagcaaagagaagcccaag tgtgtggagatcagctgcaaaagccccgatgtgatcaatg gcagccccattagccaaaagattatctataaggaaaacga gagattccaatacaagtgcaacatggggtacgagtactcc gaaagaggcgacgccgtctgcaccgaatccggctggag acctctccctagctgtgaggagaagagctgtgataacccct atattcccaacggcgattattcccctctgagaatcaagcacc ggaccggcgacgagattacctatcagtgtagaaacggctt ctaccccgctacaagaggcaataccgctaagtgcacctcc accggctggattcccgcccctagatgc | |
| | CD5-sp+linker+ CR2(SCR1 SCR4)+(G₄ S)₂+linker+ FH(SCR1-SCR5)+link er+(G₄S)₂+ FH(SCR1-SCR5)+link er | MPMGSLQPLATLYLLGML VASCLGISCGSPPPILNGRI SYYSTPIAVGTVIRYSCSGT FRLIGEKSLLCITKDKVDG TWDKPAPKCEYFNKYSSC PEPIVPGGYKIRGSTPYRH GDSVTFACKTNFSMNGNK SVWCQANNMWGPTRLPT CVSVFPLECPALPMIHNGH HTSENVGSIAPGLSVTYSC ESGYLLVGEKIINCLSSGK WSAVPPTCEEARCKSLGR FPNGKVKEPPILRVGVTAN FFCDEGYRLQGPPSSRCVI AGQGVAWTKMPVCGGGG SGGGGSEDCNELPPRRNT EILTGSWSDQTYPEGTQAI YKCRPGYRSLGNVIMVCR KGEWVALNPLRKCQKRPC GHPGDTPFGTFTLTGGNVF EYGVKAVYTCNEGYQLLG EINYRECDTDGWTNDIPIC EVVKCLPVTAPENGKIVSS AMEPDREYHFGQAVRFVC NSGYKIEGDEEMHCSDDG FWSKEKPKCVEISCKSPDV INGSPISQKIIYKENERFQY KCNMGYEYSERGDAVCTE SGWRPLPSCEEKSCDNPYI PNGDYSPLRIKHRTGDEIT YQCRNGFYPATRGNTAKC TSTGWIPAPRCTLKGGGGS GGGGSEDCNELPPRRNTEI LTGSWSDQTYPEGTQAIY KCRPGYRSLGNVIMVCRK GEWVALNPLRKCQKRPCG HPGDTPFGTFTLTGGNVFE YGVKAVYTCNEGYQLLGE INYRECDTDGWTNDIPICE VVKCLPVTAPENGKIVSSA MEPDREYHFGQAVRFVCN SGYKIEGDEEMHCSDDGF WSKEKPKCVEISCKSPDVI NGSPISQKIIYKENERFQY | 81 | atgcccatgggcagcctgcagcccctggccaccctgtacc tgctgggcatgctggtggctagctgcctgggcatcagctg cggcagcccccctcccatcctcaacggcagaatcagctac tacagcacccccatcgccgtgggcaccgtgatcagataca gctgcagcggcaccttcagactgatcggcgagaagagcc tgctgtgcatcaccaaggacaaggtggacggcacctggg acaagcccgcccccaagtgcgagtacttcaacaagtaca gcagctgccccgagcccatcgtgcccggcggctataaga tcagaggcagcaccccctacagacacggcgacagcgtg accttcgcctgcaagaccaacttcagcatgaacggcaaca agagcgtgtggtgccaagccaacaacatgtggggcccca caagactgcccacctgcgtgagcgtgttccccctggagtg ccccgccctgcccatgatccacaacggccaccacacaag cgagaacgtgggcagcatcgcccccggcctgagcgtga cctacagctgcgagagcggctacctgctggtgggcgaga agatcatcaactgcctgagcagcggcaagtggagcgccg tgccccccacctgcgaggaggctagatgcaagagcctgg gcagattccccaacggcaaggtgaaggagccccccatcc tgagagtgggcgtgaccgccaacttcttctgcgacgaggg ctacagactgcaaggcccccctagcagcagatgcgtgatc gccggccaaggcgtggcttggacaaaaatgcctgtgtgtg gcgggggcgggagcggcgggggcggcagcgaagact gcaatgagctccccccccggagaaacaccgagatcctga ccggcagctggagcgatcagacctatcccgagggcaccc aagccatttataagtgcagacccggctacagaagcctggg gaatgtgatcatggtctgcagaaagggcgagtgggtggc cctgaatcccctccggaagtgccaaaagcggccctgtgg gcaccccggcgacacacccttcggcacctttaccctgacc ggcggcaatgtcttcgagtatggcgtcaaggccgtgtatac ctgcaacgaaggctatcagctgctgggcgagatcaactat cgggagtgtgacaccgacggctggacaaacgacatcccc atctgcgaggtggtgaagtgcctgcccgtgaccgccccc gaaaacggcaaaatcgtcagcagcgctatggaacccgac cgggaataccacttcggccaagctgtcagattcgtgtgtaa tagcgggtataagattgagggcgatgaggaaatgcactgc agcgacgacggcttctggagcaaggagaagcccaaatg cgtggagatcagctgtaaaagccccgacgtgatcaacgg cagccccatcagccaaaaaatcatttacaaggagaacgaa agatttcagtacaagtgtaatatgggctacgagtatagcga gcggggcgacgctgtgtgcaccgagagcggctggagac ctctcccttcctgcgaggagaagagctgcgacaaccccta catccccaacggcgactactcccccctgagaatcaaacat agaaccggggacgagatcacctatcagtgcagaaatggc ttctaccctgccacaagagggaacaccgccaagtgcacat | 82 |
| | | KCNMGYEYSERGDAVCTE SGWRPLPSCEEKSCDNPYI PNGDYSPLRIKHRTGDEIT YQCRNGFYPATRGNTAKC TSTGWIPAPRCTLK | | ccaccgggtggattcccgctcctagatgtacactcaaggg cgggggcggcagcggcgggggcggcagcgaggactg caacgagctgccccctcggagaaacacagagatcctgac cgggagctggagcgatcagacctaccccgaggggacac aagccatctacaagtgcagacctgggtatagaagcctggg caacgtgattatggtgtgcagaaagggggaatgggtggc cctgaatcccctgagaaagtgtcagaagagaccctgcgg ccaccctggcgatacaccctttggcaccttcaccctgaccgg gggcaacgtgttcgagtacggcgtgaaggccgtgtaca catgcaacgagggctatcaactgctgggggagatcaatta cagagagtgcgataccgatgggtggaccaacgacattcct atctgcgaagtcgtgaagtgcctccctgtcaccgcccccg agaacggcaagatcgtgagcagcgctatggagcctgata gagagtaccactttgggcaagccgtgagattcgtctgcaa cagcgggtacaagatcgagggcgacgaggagatgcact gttccgatgacgggttctggagcaaagagaagcccaagt gtgtggagatcagctgcaaaagccccgatgtgatcaatgg cagccccattagccaaaagattatctataaggaaaacgag agattccaatacaagtgcaacatggggtacgagtactccg aaagaggcgacgccgtctgcaccgaatccggctggaga cctctccctagctgtgaggagaagagctgtgataaccccta tattcccaacggcgattattcccctctgagaatcaagcacc ggaccggcgacgagattacctatcagtgtagaaacggctt ctaccccgctacaagaggcaataccgctaagtgcacctcc accggctggattcccgcccctagatgcaccctcaag | |

### Example 8: Preparation and Identification of Other Recombinant AAV Viruses

Recombinant AAV viruses were prepared using a triple-plasmid packaging system. The helper plasmid (phelper), AAV Cap and Rep proteins expression plasmid, and the target vector expression plasmid (ssAAV-XMDC061, ssAAV-XMDC062, ssAAV-XMDC025-(CR2-R36A K41A K67A), ssAAV-XMDC026-(CR2-R36A K41A K67A), ssAAV-XMDC029-(CR2-R36A K41A K67A), ssAAV-XMDC061-(CR2-R36A K41A K67A), ssAAV-XMDC062-(CR2-R36A K41A K67A), ssAAV-CR2-FH(SCR1-5+1-5)) were mixed at a mass ratio of 2:1:1, and formed transfection complexes with PEI transfection reagent. These complexes were used to transfect HEK293T cells for the packaging of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), and AAV-CR2-FH(SCR1-5+1-5) viruses. The supernatant was collected twice on the 3rd and 7th days after transfection, respectively, to obtain AAV virus particles containing the target genes. Different gradients of iodixanol (15%, 25%, 40% and 60%) were used for density gradient centrifugation (Beckman's ultracentrifuge) to obtain purified AAV viruses. For the obtained purified AAV viruses, the quality of AAV was identified by transmission electron microscopy, and the titers of AAV viruses were quantified by qPCR.

### Example 9: PEG-400-induced Dry AMD Mouse Model and Administration of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, AAV-CR2-FH(SCR1-5+1-5) by Injection

The specific method is as follows:

Eighty-eight 4 to 5-week-old male C57BL/6J mice of SPF grade (purchased from Beijing Vital River Laboratory Animal Technology Co., Ltd.), weighing approximately 14-18 g, were raised according to their rhythm in an environment with alternating 12-hour light-dark cycle.

Since a certain period of time is required for the expression of the target gene to reach a stable level after AAV injection, bilateral intravitreal injection of the drug on Day 1, and bilateral subretinal injection of PEG-400 on Day 22 were conducted to establish the model, and the eyeballs were enucleated on Day 27 for frozen sectioning, followed by staining to observe the structures of the retina and RPE. The treatment is shown in Table 4.

**Table 4. Administration Scheme**

| Group | Administration (Injection on D1) | Concentration (vg/mL) | Volume (µL) | Dosage (vg/eye) | Administration Route | Numbers of Animals |
|---|---|---|---|---|---|---|
| Control | PBS | - | 2 | - | Intravitreal injection | 8 |
| Model | PBS | - | 2 | - | Intravitreal injection | 8 |
| XMDC061 | AAV-XMDC061 | 2.8×10¹² | 2 | 5.6×10⁹ | Intravitreal injection | 8 |
| XMDC062 | AAV-XMDC062 | 2.8×10¹² | 2 | 5.6×10⁹ | Intravitreal injection | 8 |
| XMDC025-(CR2-R36A K41A K67A) | AAV-XMDC025-(CR2-R36A K41A K67A) | 2.8×10¹² | 2 | 5.6×10⁹ | Intravitreal injection | 8 |
| XMDC026-(CR2-R36A K41A K67A) | AAV-XMDC026-(CR2-R36A K41A K67A) | 2.8×10¹² | 2 | 5.6×10⁹ | Intravitreal injection | 8 |
| XMDC029-(CR2-R36A K41A K67A) | AAV-XMDC029-(CR2-R36A K41A K67A) | 2.8×10¹² | 2 | 5.6×10⁹ | Intravitreal injection | 8 |
| XMDC061-(CR2-R36A K41A K67A) | AAV-XMDC061-(CR2-R36A K41A K67A) | 2.8×10¹² | 2 | 5.6×10⁹ | Intravitreal injection | 8 |
| XMDC062-(CR2-R36A K41A K67A) | AAV-XMDC062-(CR2-R36A K41A K67A) | 2.8×10¹² | 2 | 5.6×10⁹ | Intravitreal injection | 8 |
| XMDC029 | AAV-XMDC029 | 2.8×10¹² | 2 | 5.6×10⁹ | Intravitreal injection | 8 |
| CR2-FH(SCR1-5+1-5) | AAV-CR2-FH(SCR1-5+1-5) | 2.8×10¹² | 2 | 5.6×10⁹ | Intravitreal injection | 8 |

The specific operational steps for injection administration were the same as in Example 3.

### Example 10: Evaluation of the Effects of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, AAV-CR2-FH(SCR1-5+1-5) on the Thickness and Nuclear Density of the Outer Nuclear Layer of the Retina in PEG-400-induced Dry AMD Model Mice By Hematoxylin-eosin (HE) Staining

The eyeballs of the mice in Example 9 were enucleated, and one eye from each mouse was taken for frozen sectioning and HE staining. After staining, photographs were taken, and Image J was used to measure and analyze the differences in the thickness and nuclear density of the outer nuclear layer (ONL) of the retinal sections. The specific operation steps were the same as in Example 4.

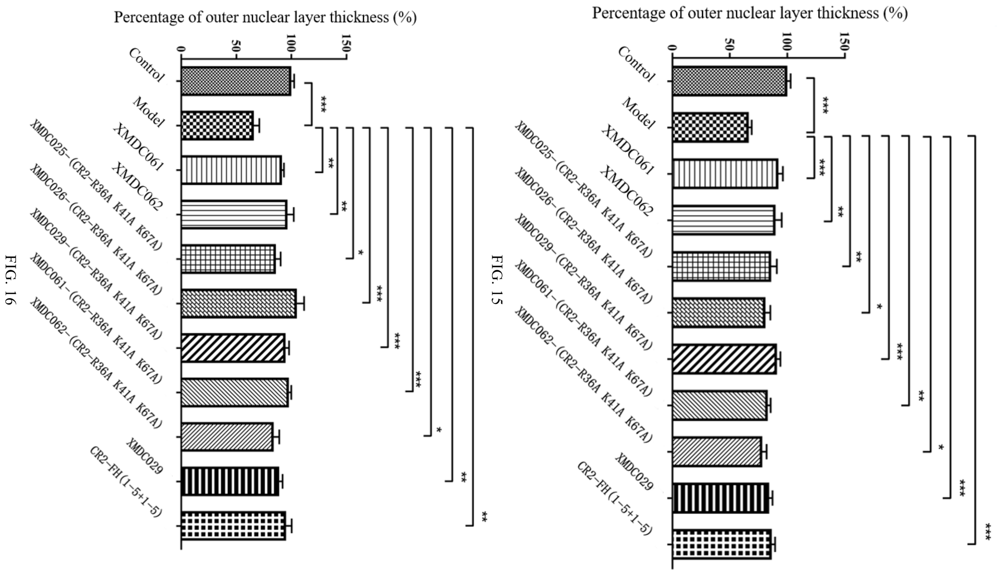

The results are shown in Figures 15, 16, and 17. Compared with the control group, the thickness and nuclear density of the outer nuclear layer of the retina in the model group mice were significantly reduced, and the retinal structure was obviously damaged. Compared with the model group, injections of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, AAV-CR2-FH(SCR1-5+1-5) could all significantly increase the thickness and nuclear density of the outer nuclear layer and improve retinal structural damage.

### Example 11: Evaluation of the Effects of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5) on Retinal Photoreceptor Cells in PEG-400-induced Dry AMD Model Mice by Immunofluorescence Staining

The frozen sections in Example 9 were immunofluorescence stained, photographed under a fluorescence microscope, and the specific operation steps were the same as in Example 5.

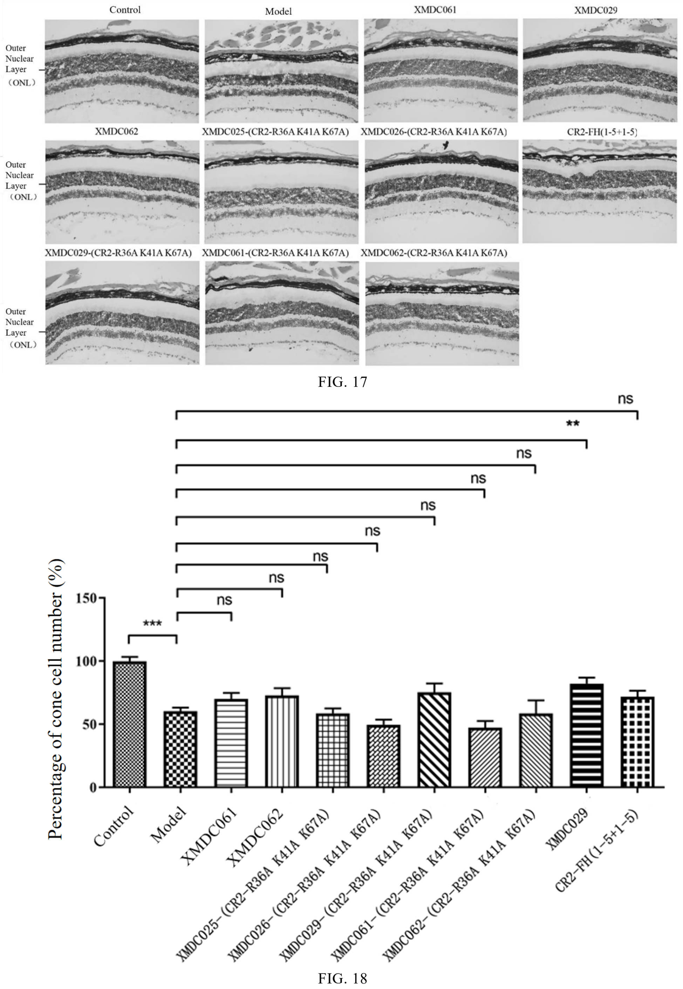

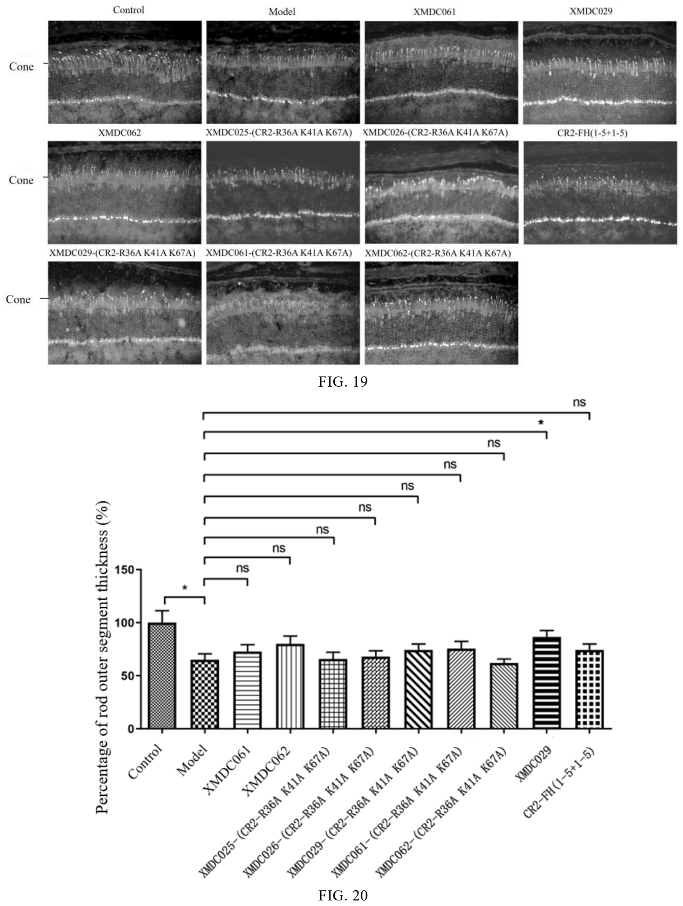

The results are shown in Figures 18, 19, 20, and 21. Compared with the control group, the model group showed reduced number of retinal cone cells, narrowed thickness of the outer segments of rod cells, and significantly damaged cone and rod cells. Compared with the model group, injection of AAV-XMDC029 could significantly improve the damage caused by model establishment and effectively protect the thickness of the outer segments of cone and rod cells.

### Example 12: Evaluation of the Effects of AAV-XMDC061, AAV-XMDC062, AAV-XMDC025- (CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5) on RPE Cells in PEG-400-induced Dry AMD Model Mice by F-actin Staining

The other eyes removed from the mice in Example 9 were stained with F-actin, observed and photographed under a fluorescence microscope, and the specific operation steps were the same as in Example 6.

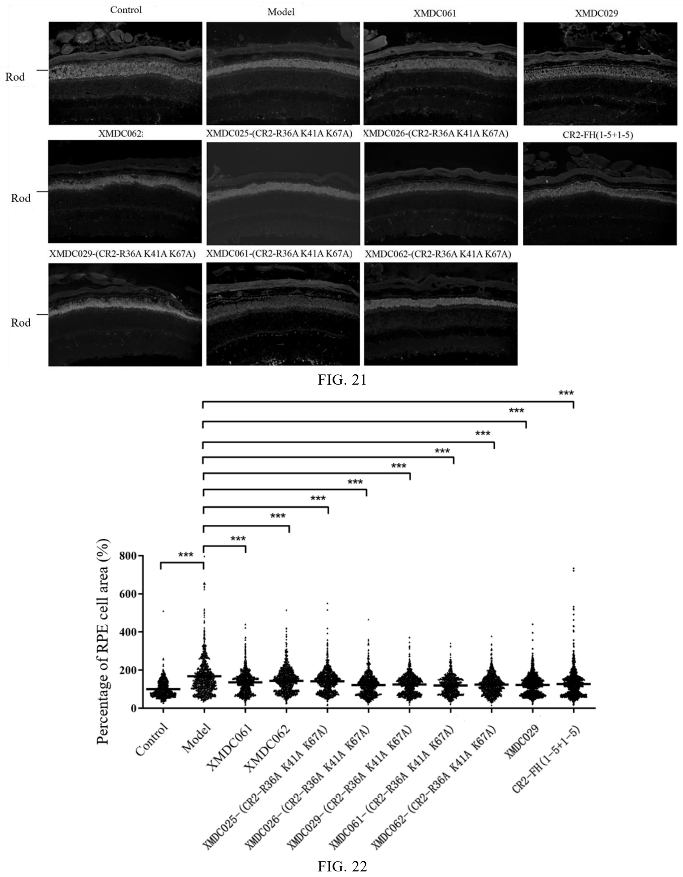

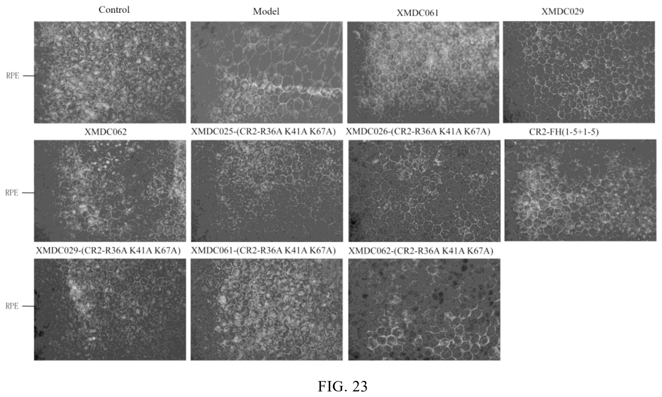

The results are shown in Figures 22 and 23. Compared with the control group, the RPE cells in the model group were significantly enlarged and obviously damaged. Compared with the model group, the injections of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5) could all significantly reduce the area of RPE cells, and the damage to RPE cells was significantly improved.

Combining the results of HE staining, immunofluorescence staining, and F-actin staining, injections of AAV-XMDC061, AAV-XMDC062, AAV-XMDC025-(CR2-R36A K41A K67A), AAV-XMDC026-(CR2-R36A K41A K67A), AAV-XMDC029-(CR2-R36A K41A K67A), AAV-XMDC061-(CR2-R36A K41A K67A), AAV-XMDC062-(CR2-R36A K41A K67A), AAV-XMDC029, and AAV-CR2-FH(SCR1-5+1-5) all have a significant improvement effect on the structural damage of the retinal outer nuclear layer and RPE cell damage in the PEG-400-induced dry AMD mouse model. Among them, AAV-XMDC029 can also effectively improve the damage to the thickness of the outer segments of cone and rod cells.

### Statistical Analysis

Data processing and statistical analysis were performed using GraphPad Prism 8.0 software. Statistical levels were set at 5% or p ≤ 0.05, the mean and standard errors (Mean ± SEM) of various analysis indexes were calculated, and p ≤ 0.05 means that the difference is statistically significant.

The present disclosure is not limited to the above embodiments. Any changes, modifications, substitutions, combinations, and simplifications made without departing from the spirit and principles of the present disclosure belong to the equivalent technical solutions of the present disclosure, and are all included in the protection scope of the present disclosure.

## Claims

1. A complement receptor 2 (CR2)-factor H (FH) fusion protein, comprising: a) a CR2 moiety comprising a CR2 fragment, and b) a FH moiety comprising a FH fragment, the CR2 moiety and the FH moiety are optionally linked by a linker sequence, wherein the CR2 moiety comprises the first four N-terminal SCR domains of CR2 or a variant thereof, the FH moiety comprises the first four N-terminal short consensus repeat (SCR) domains of FH.

2. The CR2-FH fusion protein of claim 1, wherein the FH moiety comprises two or more FH fragments comprising the first four N-terminal SCR domains of FH, wherein the two or more FH fragments are optionally linked by a linker sequence.

3. The CR2-FH fusion protein of claim 1, wherein the FH moiety comprises the first four N-terminal SCR domains of FH, the N-terminal eighth SCR domain of FH, and the N-terminal nineteenth to twentieth SCR domains of FH.

4. The CR2-FH fusion protein of claim 1, wherein the FH moiety comprises the first four N-terminal SCR domains of FH, and the N-terminal eighteenth to twentieth SCR domains of FH.

5. The CR2-FH fusion protein of claim 1, wherein the FH moiety comprises the first four N-terminal SCR domains of FH, the N-terminal eighteenth SCR domain of FH, and the N-terminal twentieth SCR domain of FH.

6. The CR2-FH fusion protein of claim 1, wherein the FH moiety comprises the first four N-terminal SCR domains of two FHs and the N-terminal seventh SCR domain of FH.

7. The CR2-FH fusion protein of any one of claims 1 to 6, wherein the linker sequence comprises a sequence represented by (G₄S)ₙ and/or an endogenous linking sequence, wherein n is an integer greater than 0.

8. The CR2-FH fusion protein of any one of claims 1 to 6, wherein the domains are linked by a linker sequence, and the linker sequence comprises a sequence represented by (G₄S)ₙ and/or an endogenous linking sequence, wherein n is an integer greater than 0.

9. The CR2-FH fusion protein of any one of claims 1 to 8, wherein the first four N-terminal short consensus repeat (SCR) domains of FH comprise an amino acid sequence as shown in SEQ ID NO: 2.

10. The CR2-FH fusion protein of claim 3, wherein the N-terminal eighth SCR domain of FH comprises an amino acid sequence as shown in SEQ ID NO: 3.

11. The CR2-FH fusion protein of claim 3, wherein the N-terminal nineteenth to twentieth SCR domains of FH comprise an amino acid sequence as shown in SEQ ID NO: 4.

12. The CR2-FH fusion protein of claim 4, wherein the N-terminal eighteenth to twentieth SCR domains of FH comprise an amino acid sequence as shown in SEQ ID NO: 7.

13. The CR2-FH fusion protein of claim 5, wherein the N-terminal eighteenth SCR domain of FH comprises an amino acid sequence as shown in SEQ ID NO: 39.

14. The CR2-FH fusion protein of claim 5, wherein the N-terminal twentieth SCR domain of FH comprises an amino acid sequence as shown in SEQ ID NO: 40.

15. The CR2-FH fusion protein of claim 6, wherein the N-terminal seventh SCR domain of FH comprises an amino acid sequence as shown in SEQ ID NO: 43.

16. The CR2-FH fusion protein of any one of claims 1 to 15, wherein the CR2 moiety or the variant thereof comprises an amino acid sequence as shown in SEQ ID NO: 1 or SEQ ID NO: 46.

17. The CR2-FH fusion protein of any one of claims 1 to 16, wherein the CR2-FH fusion protein comprises an amino acid sequence as shown in SEQ ID NO: 5, SEQ ID NO: 8, SEQ ID NO: 10, SEQ ID NO: 41, SEQ ID NO: 44, SEQ ID NO: 47, SEQ ID NO: 49, SEQ ID NO: 51, SEQ ID NO: 53, or SEQ ID NO: 55.

18. The CR2-FH fusion protein of any one of claims 1 to 17, wherein the CR2-FH fusion protein comprises a signal peptide sequence, preferably, the signal peptide sequence is located at the N-terminus, more preferably, the amino acid sequence of the signal peptide is as shown in SEQ ID NO: 17.

19. The CR2-FH fusion protein of claim 18, wherein the CR2-FH fusion protein comprises an amino acid sequence as shown in SEQ ID NO: 6, SEQ ID NO: 9, SEQ ID NO: 11, SEQ ID NO: 42, SEQ ID NO: 45, SEQ ID NO: 48, SEQ ID NO: 50, SEQ ID NO: 52, SEQ ID NO: 54, or SEQ ID NO: 56.

20. A polynucleotide encoding the CR2-FH fusion protein of any one of claims 1 to 19.

21. The polynucleotide of claim 20, wherein the polynucleotide sequence encoding the CR2-FH fusion protein comprises a nucleotide sequence as shown in SEQ ID NO: 22, 23, 25, 26, 30, 31, 59, 60, 62, 63, 65, 66, 67, 68, 70, 71, 72, 73, 74 or 75.

22. A vector encoding the polynucleotide of claim 20 or 21.

23. The vector of claim 22, wherein the vector is at least one selected from adeno-associated virus (AAV) vectors, adenoviral vectors, RNA viral vectors, lentiviral vectors, and vaccinia viral vectors.

24. A host cell comprising the polynucleotide of claim 20 or 21 or the vector of claim 23.

25. An AAV particle comprising the AAV vector of claim 23.

26. A pharmaceutical composition comprising at least one of the CR2-FH fusion protein of any one of claims 1 to 19, the polynucleotide of claim 20 or 21, the vector of claim 22 or 23, the host cell of claim 24, and the AAV particle of claim 25,
and a pharmaceutically acceptable carrier.

27. The composition of claim 26, wherein the composition is suitable for intraocular, intravenous, intraarterial, subcutaneous, endotracheal or inhalation administration.

28. Use of the CR2-FH fusion protein of any one of claims 1 to 19, the polynucleotide of claim 20 or 21, the vector of claim 22 or 23, the host cell of claim 24, the AAV particles of claim 25, or the pharmaceutical composition of claim 26 in the preparation of a medicament for treating an alternative complement pathway-related disease in a subject.

29. The use of claim 28, wherein the alternative complement pathway-related disease is an inflammatory disease or an autoimmune disease.

30. The use of claim 28, wherein the alternative complement pathway-related disease is age-related macular degeneration, preferably dry age-related macular degeneration.

31. The use of claim 28, wherein the alternative complement pathway-related disease is a symptom of microangiopathic hemolytic anemia, thrombocytopenia, or acute renal failure.

32. The use of claim 28, wherein the alternative complement pathway-related disease is selected from the group consisting of macular degeneration, ischemia reperfusion, organ transplant rejection, drusen-related diseases, pregnancy-related diseases, adverse drug reactions, and post-cardiopulmonary bypass complications.

33. The use of claim 28, wherein the alternative complement pathway-related disease is selected from the group consisting of age-related macular degeneration (AMD), rheumatoid arthritis, C3 glomerulonephritis, membrane proliferative glomerulonephritis II (MPGN II), Factor H-related hemolytic uremic syndrome (HUS), paroxysmal nocturnal hemoglobinuria (PNH), systemic lupus erythematosus (SLE), lupus nephritis, stroke, myocardial infarction, acute respiratory distress syndrome (ARDS), sepsis, burns, inflammation associated with cardiopulmonary bypass and hemodialysis, plasmapheresis, platelet isolation, leukapheresis, extracorporeal membrane oxygenation (ECMO), heparin-induced extracorporeal LDL precipitation (HELP), and radiological contrast induced allergic reactions.
